(19) Europäisches Patentamt
European Patent Office
Office européen des brevets

(11) Publication number: **0 463 514 B1**

(12) **EUROPEAN PATENT SPECIFICATION**

(45) Date of publication of patent specification: **05.04.95**

(51) Int. Cl.[6]: **C07C 323/59**, A61K 31/195, A61K 31/22, A61K 31/225

(21) Application number: **91109808.5**

(22) Date of filing: **14.06.91**

(54) **3.3'-Dithiobis (propionic acids) and esters thereof.**

(30) Priority: **28.06.90 SE 9002274**
**28.06.90 SE 9002275**

(43) Date of publication of application:
**02.01.92 Bulletin 92/01**

(45) Publication of the grant of the patent:
**05.04.95 Bulletin 95/14**

(84) Designated Contracting States:
**AT BE CH DE DK ES FR GB GR IT LI LU NL SE**

(56) References cited:
**EP-A- 0 300 100**
**FR-A- 2 503 151**
**JP-A-62 195 356**
**US-A- 4 708 965**
**US-A- 4 827 016**
**DE-A- 2 326 444**
**FR-M- 8 205**
**US-A- 3 952 115**
**US-A- 4 724 239**

(73) Proprietor: **Astra Aktiebolag**
**S-151 85 Södertälje (SE)**

(72) Inventor: **Andersson, Carl-Magnus Alexander**
**Filippavägen 6B**
**S-222 41 Lund (SE)**
Inventor: **Bergstrand, Sten Häkan Axel**
**Villavägen 2**
**S-230 50 Bjärred (SE)**
Inventor: **Hallberg, Anders Rudolf**
**Björklundavägen 4A**
**S-756 46 Uppsala (SE)**
Inventor: **Särnstrand, Bengt Olof**
**Leifs väg 28**
**S-237 00 Bjärred (SE)**
Inventor: **Tunek, Per Anders Sigvard**
**Spexarevägen 5 C**
**S-223 71 Lund (SE)**

(74) Representative: **Danielsson, Sten Ove et al**
**AB ASTRA**
**Patent and Trademark Department**
**S-151 85 Södertälje (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 463 514 B1

## Description

### Field of the Invention

The present invention relates to new cystine derivatives with immunomodulating activity, processes for their preparation, pharmaceutical compositions containing them and methods for their pharmacological use.

The object of the invention is to provide a cystine derivative with immunomodulating activity. Such a substance will be useful in the treatment of various diseases.

### Background of the Invention

N-Acetyl-L-cysteine is a compound widely used for treating chronic obstructive airway diseases/chronic bronchitis (for further references see Multicentre Study Group. Long-term oral acetylcysteine in chronic bronchitis. A double-blind controlled study. Eur. J. Respir. Dis. 1980, 61 (suppl. 111), 93-108; Boman, G., Bäcker, U., Larsson, S., Melander, B., and Wåhlander, L. Oral acetylcysteine reduces exacerbation rate in chronic bronchitis. Report of a trial organized by the Swedish Society for Pulmonary Disease. Eur. J. Respir. Dis. 1983, 64, 405-415; and British Thoracic Society Research Committee. Oral N-acetylcysteine and exacerbation rates in patients with chronic bronchitis and severe airway obstruction. Thorax 1985, 40, 832-835). The mechanism of action of the compound is not disclosed; its effect has been attributed to mucolytic properties (see Multicentre Study Group. Long-term oral acetylcysteine in chronic bronchitis. A double-blind controlled study. Eur. J. Respir. Dis. 1980, 61 (suppl. 111), 93-108; Boman, G., Bäcker, U., Larsson, S., Melander, B., and Wåhlander, L. Oral acetylcysteine reduces exacerbation rate in chronic bronchitis. Report of a trial organized by the Swedish Society for Pulmonary Disease. Eur. J. Respir. Dis. 1983, 64, 405-415; and British Thoracic Society Research Committee. Oral N-acetylcysteine and exacerbation rates in patients with chronic bronchitis and severe airway obstruction. Thorax 1985, 40, 832-835), antioxidant properties (see Aruoma, O.I., Halliwell, B., Hoey, B.M., and Butler, J. Free Radical Biol. Med. 1989, 6, 593-597), and also immunomodulating properties (see Bergstrand, H., Björnson, A., Eklund, A., Hernbrand, R., Eklund, A., Larsson, K., Linden M., and Nilsson, A. Stimuli-induced superoxide radical generation in vitro by human alveolar macrophages from smokers: Modulation by N-Acetylcysteine treatment in vivo. J. Free Radicals Biol. & Med. 2, 1986, 119-127).

Also known is N,N'-diacetyl-L-cystine. This compound has previously shortly been described in the patent literature as well as in the scientific literature (US 4827016; EP 300100; US 4724239; US 4708965; DE 2326444; Wilson, I.D., and Nicholson, J.K. Analysis of thiols and disulfides in Sulphur-containing drugs and related organic compounds. Chemistry, Biochemistry and Toxicology (ed L.A. Damani) Vol. 2A. Analytical, biochemical and toxicological aspects of sulphur xenobiochemistry. Ellis Horwood Series in Biochemical Pharmacology (Halstred Press: a division of John Wiley & Sons) Chichester 1989, p. 45; and Sjödin, K., Nilsson, E., Hallberg, A., and Tunek, A. Metabolism of N-Acetyl-L-cysteine. Some structural requirements for the deacetylation and consequences for the oral bioavailability. Biochem. Pharmacol. 1989, 38, 3981-3985). In US 4827016 the compound is claimed to be effective for topical treatment of dermal inflammations which are induced and propagated by leukotrienes. However, nothing has been reported or generally known regarding its pharmacological and/or therapeutic properties with respect to immunological systems and inflammatory diseases of the lung such as chronic bronchitis.

Previously, immunostimulating properties have been reported for simple disulfides such as hydroxyethyldisulfide (HEDS, see: St. Georgiev, V. New synthetic immunomodulating agents. Trends in Pharmacological Science 1988, 446-51).

Disclosure of the Invention

According to the present invention it has been found that a compound of the general formula

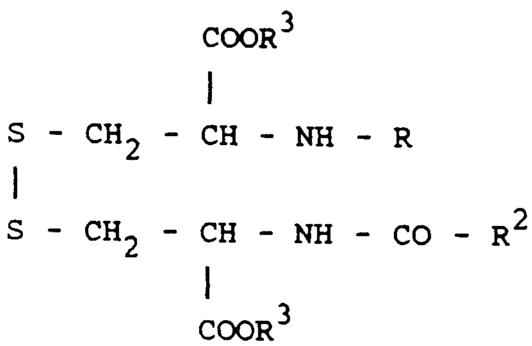

I

wherein R is hydrogen or a moiety -CO-$R^1$ wherein $R^1$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, $R^2$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, and $R^3$ is hydrogen, methyl, ethyl, propyl, isopropyl, butyl or isobutyl, provided that $R^1$ and $R^2$ are not simultaneously methyl and further provided that when $R^3$ is hydrogen $R^1$ and $R^2$ are not simultaneously n-propyl, n-heptyl, n-nonyl or n-undecyl and further provided that when R and $R^3$ are all hydrogen, then $R^2$ is not n-nonyl, or a physiologically acceptable salt and/or a stereochemical isomer thereof, is an immunomodulating, particularly immunostimulating, agent.

Therefore, the compounds of the invention may be used for treatment of diseases where a defect in the immune system and/or an ineffective host defence is at hand or can be suspected.

Examples of such diseases are chronic bronchitis and other inflammatory diseases of the airways such as asthma and rhinitis but also certain forms of autoimmune diseases like diabetes and rheumatoid arthritis and/or various malignant diseases. HIV-infection or full blown AIDS may be treated with the compounds. Also atherosclerotic disease may be treated with the compounds.

Effective amounts of the compounds of the invention for use in the treatment of the above mentioned diseases are in the range 0.5-500 mg, preferably 5-50 mg, daily dose.

Preferred compounds of the formula I above are those wherein R is hydrogen or a moiety -CO-$R^1$ wherein $R^1$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, $R^2$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, and $R^3$ is hydrogen, methyl or ethyl, provided that $R^1$ and $R^2$ are not simultaneously methyl and further provided that when $R^3$ is hydrogen $R^1$ and $R^2$ are not simultaneously n-propyl or n-heptyl. Preferred compounds of the formula I when $R^3$ is hydrogen are those wherein $R^1$ and $R^2$ are simultaneously isopropyl and tert. butyl, respectively.

Preferred compounds of the formula I above when $R^3$ = hydrogen are those where $R^1$ and $R^2$ contain 3-7 carbon atoms.

Particularly, preferred compounds of the formula I above when $R^3 \neq$ hydrogen are those where $R^1$ and $R^2$ are isopropyl and $R^3$ is methyl; $R^1$ and $R^2$ are n-pentyl and $R^3$ is methyl; $R^1$ and $R^2$ are n-heptyl and $R^3$ is methyl or ethyl.

The stereoisomeric form of the compounds of the invention particularly preferred is the L-isomer, i.e. compounds derived from L-cystine.

A physiologically acceptable salt of the compounds of formula I when $R^3$ is hydrogen is e.g. a salt of sodium, ammonium, calcium or magnesium, together with the non-toxic acid addition salts thereof. Also included are salts derived from arginine, lysine, histidine, ethanolamine, diethanolamine, ethylenediamine and choline, or other suitable organic amines.

A physiologically acceptable salt of the compounds of the formula I when $R^3 \neq$ hydrogen and R = hydrogen is the hydrochloride, hydrobromide, hydrosulphate, oxalate, tartrate etc. The salts may also be in the form of solvates, e.g. hydrates.

Pharmaceutical formulations

The described active substances can be included in different dosage forms e.g. tablets, coated tablets, gelatin capsules, solutions and aerosols.

For the preparation of tablets, coated tablets and gelatin capsules the active substance can be combined with pharmaceutically acceptable materials, e.g. lactose, starch, dicalcium phosphate, microcrystalline cellulose, polyvinylpyrrolidone, gelatin, cellulose derivatives, colloidal silicone dioxide, talc and stearic acid or its salts.

For the preparation of oral solutions suitable excipients are water, saccharose, glucose, sorbitol, fructose and xylitol.

The dosage forms can besides mentioned excipients contain preservatives, stabilizers, viscosity regulating agents, emulsifiers, sweetening agents, colouring agents, flavouring agents, tonicity regulating agents, buffers or antioxidants. They can also contain other therapeutically valuable substances.

Methods of Preparation

The compounds of the invention may be obtained by any of the following methods:

A. The oxidation of an N-acylcysteine derivative of the formula

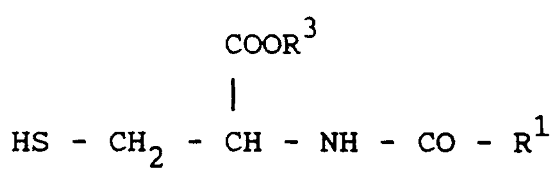

wherein $R^1$ and $R^3$ are as defined above or when $R^3$ is hydrogen optionally salt thereof, to the formation of a compound of the formula

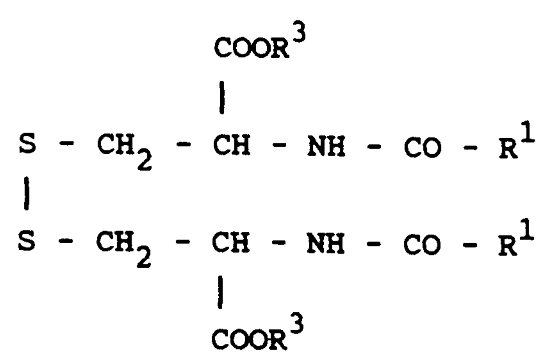

As oxidant may be used:

Hydrogen peroxide, air under alkaline conditions, alkylhydroperoxides, peracids, a halogen, nitrous or nitric oxide, oxidizing metals such as thallium (III), trialkylsulfonium salts, or selenium or tellurium oxides. The oxidation can also be performed electrochemically.

A halogen is for instance chlorine, bromine or iodine. As salts in method A and the following may be used: sodium, potassium, calcium, ammonium etc.

B. The reaction, in the presence of a suitable base, of a compound having the formula

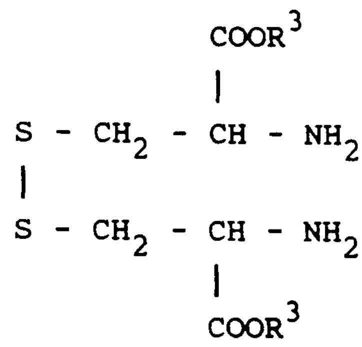

or a salt thereof wherein $R^3$ is as defined above, with a compound of the formula

$R^1$-COX

wherein $R^1$ is defined as above and COX is a reactive group capable of reacting with an amino group under formation of an amide moiety, to the formation of a compound of the formula

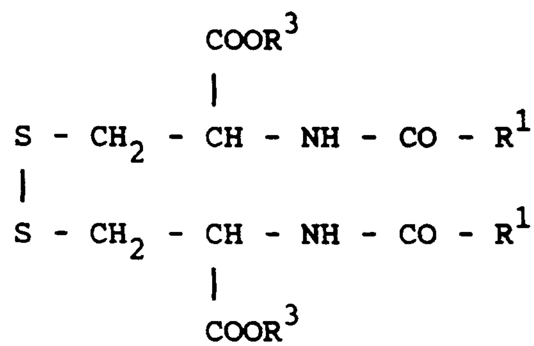

The acylating agent $R^1$COX can for instance be an acid halide, anhydride, an amide, or an activated acid or ester.

A salt in method B and in any following methods where a salt can be used can be an hydrochloride, hydrobromide, hydrosulphate, oxalate, tartrate etc. A salt in method B can also be any of the alkali salts mentioned in method A.

C. The reaction of a 2-(N-acylamino)-3-halopropionic acid derivative of the formula

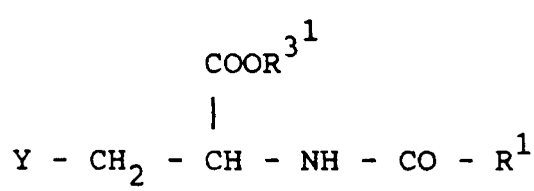

wherein $R^1$ is as defined above,

$$R^{3^{1}}$$

is $R^3$ as defined above or an acid or base labile organic group and Y is a halogen atom, with sulphur or

disulphide dianion in the presence of a base, to the formation of a compound of the formula

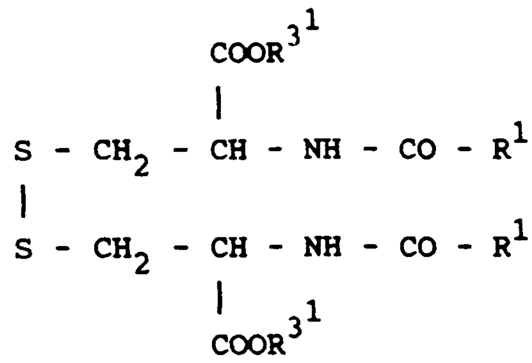

whereafter, if a compound wherein $R^3$ = H is derived, removal of the protecting group

$$R^{3^1}$$

The protecting group,

$$R^{3^1},$$

may be an acid or base labile organic group such as an alkyl, benzyl, aryl, vinyl or an allyl group.

D. The oxidation of a mixture of N-acylcysteine derivatives of the formulas

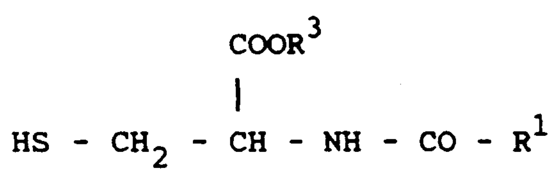

and

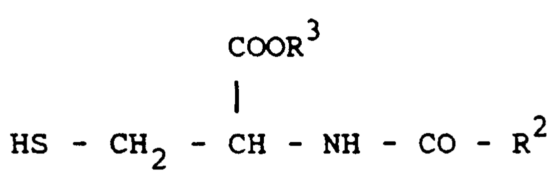

wherein $R^1$, $R^2$ and $R^3$ are as defined above or when $R^3$ is hydrogen optionally alkali salts thereof, to the formation of a compound of the formula

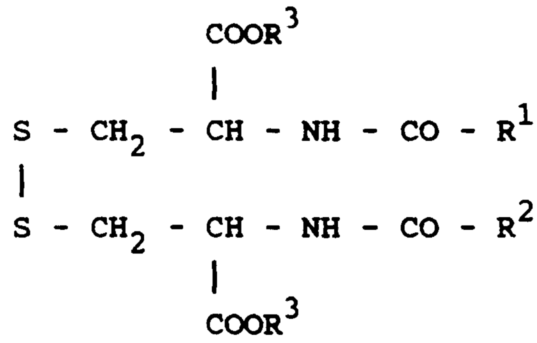

The oxidising agents of method A are applicable.

E. The oxidation of a mixture of cysteine or a cysteine ester and an N-acylcysteine derivative of the formulas

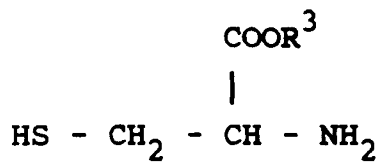

or a salt thereof

and

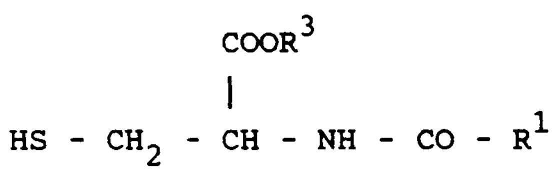

or alkali salts thereof,

wherein $R^1$ and $R^3$ are as defined above, to the formation of a compound of the formula

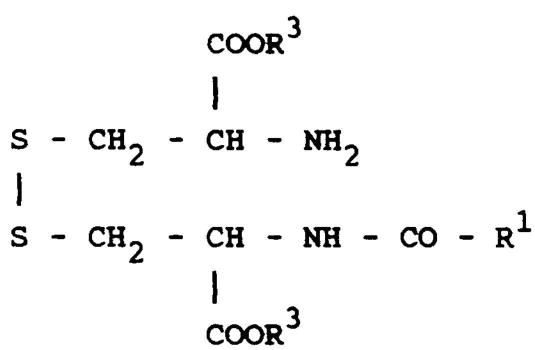

or a salt thereof.

The oxidising agents of method A may be used.

F. The reaction, in the presence of a suitable base, of an excess of cystine or a cystine diester having the formula

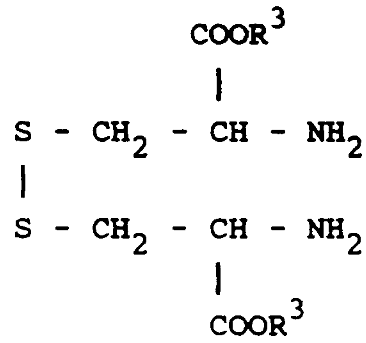

or a salt thereof wherein $R^3$ is as defined above, with a compound of the formula

$R^2$ - COX

wherein $R^2$ and COX are as defined above, to the formation of a compound of the formula

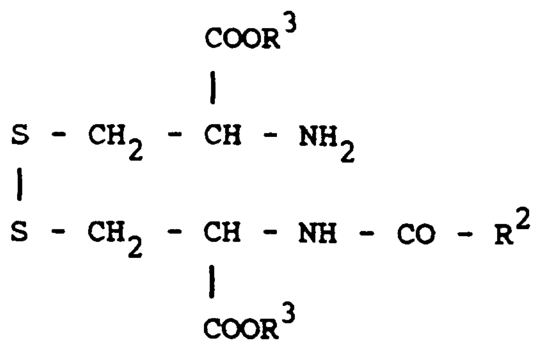

G. The reaction, in the presence of a suitable base, of a compound of the formula

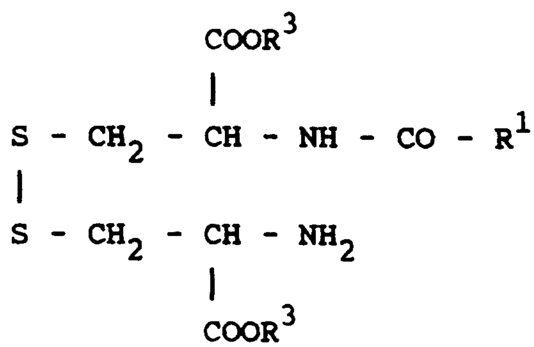

or a salt thereof wherein $R^1$ and $R^3$ are as defined above, with a compound of the formula

$R^2$ - COX

wherein $R^2$ and COX are as defined above, to the formation of a compound of the formula

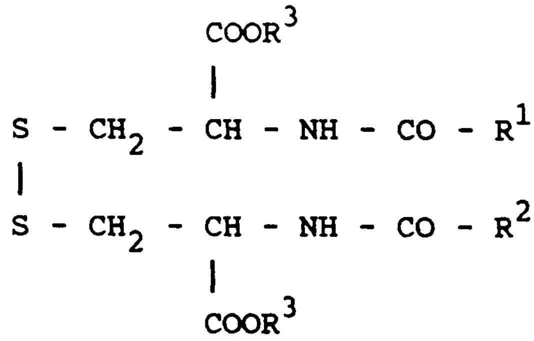

H. The reaction of an N-acylcysteine derivative of the formula

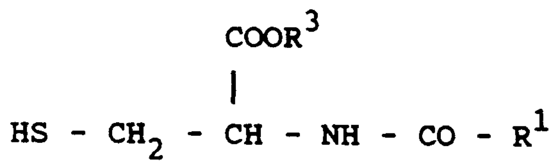

or a salt thereof with an activating reagent such as diethylazodicarboxylate to give an adduct of e.g. the formula

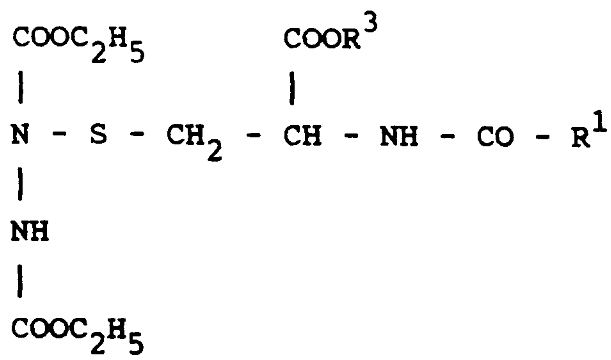

followed by reaction with a second, different or the same N-acylcysteine or N-acylcysteine ester to give a compound of the formula

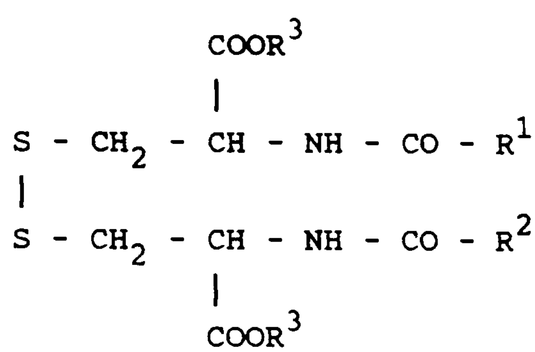

I. The reaction of a compound of the formula

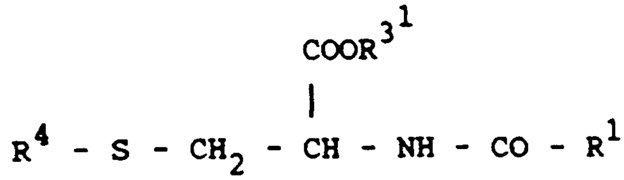

wherein $R^4$ is -Cl,

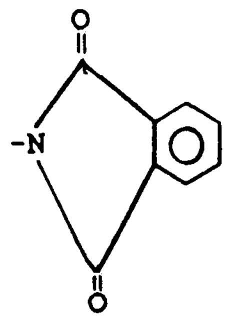

,

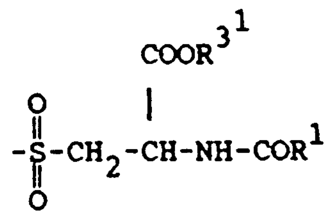

and $R^1$ and

$R^{3^{1}}$

are as defined above, with a compound of the formula

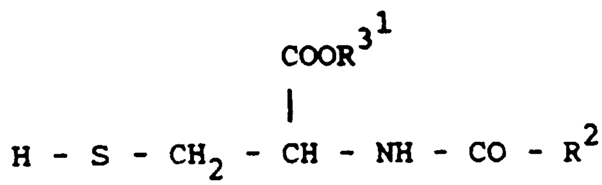

wherein $R^2$ and

$R^{3^{1}}$

are as defined above to the formation of a compound of the formula

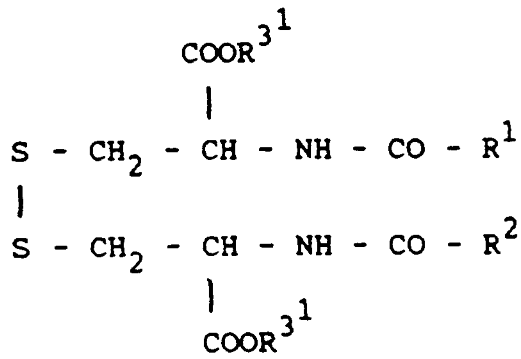

whereafter, if a compound wherein $R^3$ = H is desired, removal of the protecting group

$R^{3^1}$.

J. The esterification of a compound of the formula

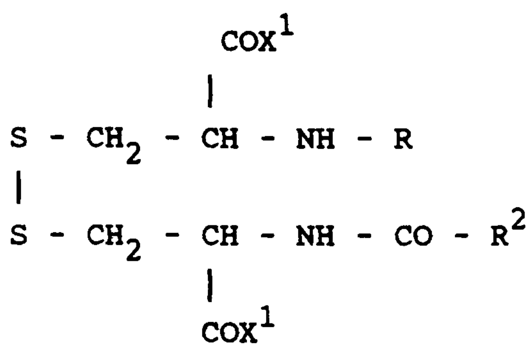

wherein R and $R^2$ are as defined above and $X^1$ is OH or a halogen atom, with a compound of the formula

$R^{3E}$ - OH

wherein $R^{3E}$ is methyl, ethyl, propyl, isopropyl, butyl or isobutyl, to the formation of a compound of the formula

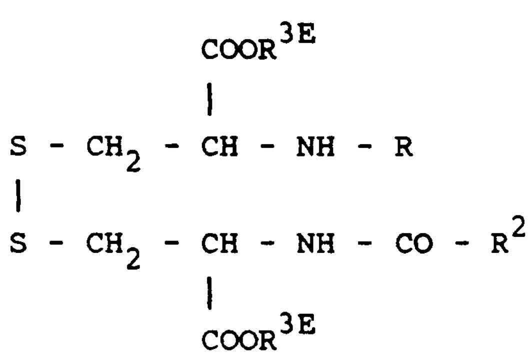

K. The alkylation of a compound of the formula

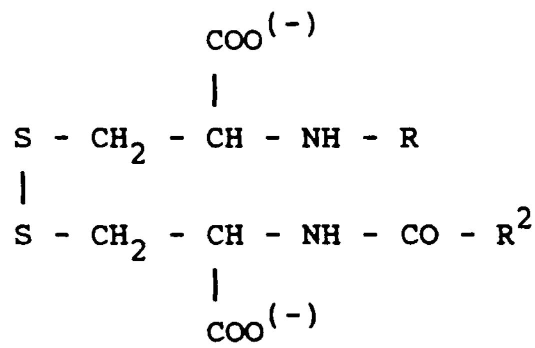

wherein R and $R^2$ are as defined above, with a compound of the formula

$R^{3E}$ - Z

wherein $R^{3E}$ is as defined above and Z is a halide, alkylsulphate, tosylate or another nucleofuge to the formation of a compound of the formula

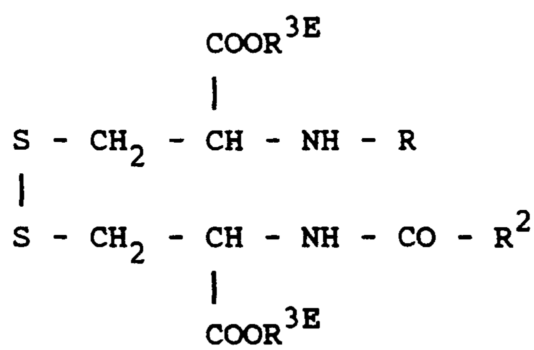

L. The reaction, in the presence of a suitable base, of a carboxyl protected cystine derivative of the formula

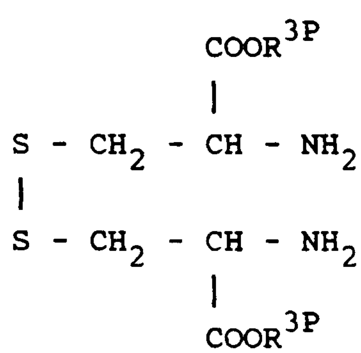

or a hydrochloride salt thereof, wherein $R^{3P}$ is an acid or base labile organic group, with a compound of the formula

$R^1$- COX

wherein $R^1$ is defined as above and COX is a reactive group capable of reacting with an amino group

under formation of an amide moiety, and thereafter removal of the protecting group $R^{3P}$ to the formation of a compound of the formula

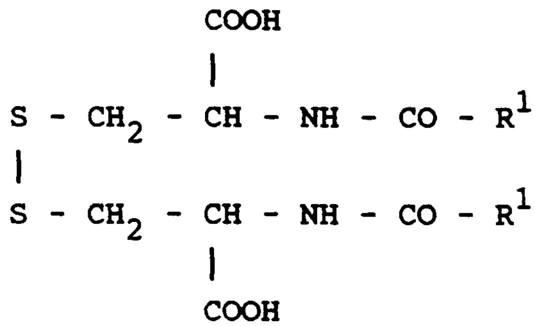

The acylating agent $R^1COX$ is as defined under B. The protecting group, $R^{3P}$, may be an acid or base labile organic group such as an alkyl, benzyl, aryl, vinyl or allyl group.

M. The reaction, under alkaline conditions, of a carboxyl protected cystine derivative of the formula

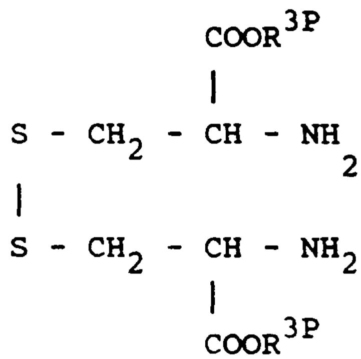

or a hydrochloride salt thereof, wherein $R^{3P}$ is as defined above, with a compound of the formula

$R^2$ - COX

wherein $R^2$ and COX are as defined above and thereafter removal of the protecting group $R^{3P}$ to the formation of a compound of the formula

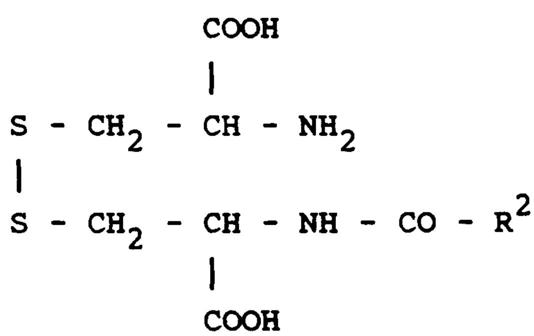

N. The reaction of a carboxyl protected cystine derivative of the formula

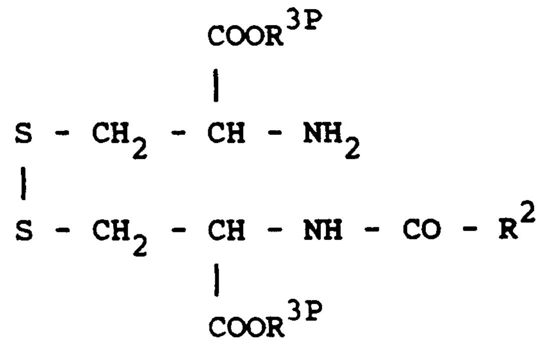

or a hydrochloride salt thereof, wherein $R^2$ and $R^{3P}$ are as defined above, with a compound of the formula

$R^1$ - COX

wherein $R^1$ and COX are as defined above and thereafter removal of the protecting group $R^{3P}$ to the formation of a compound of the formula

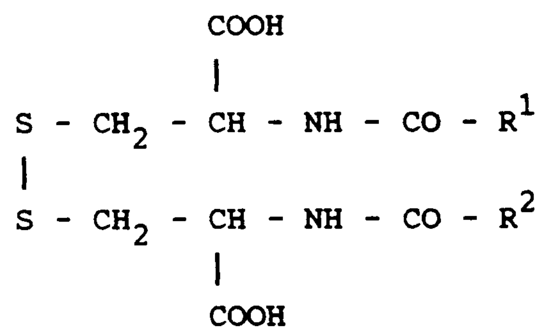

O. The equilibration, in alcohol or aqueous solution, of a mixture of a cystine derivative and a cysteine derivative having the formulas

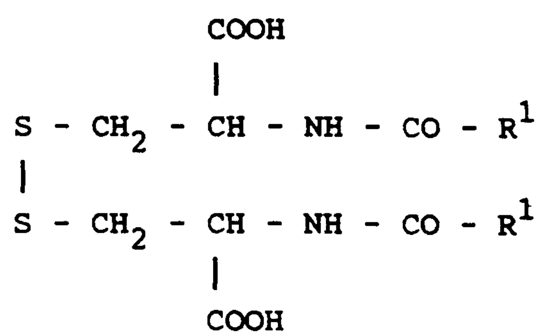

and

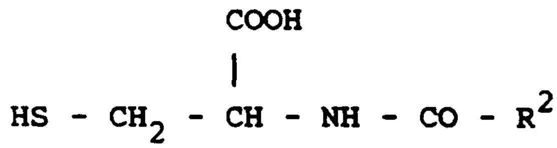

or alkali salts thereof, wherein $R^1$ and $R^2$ are as defined above, to the formation of a compound of the formula

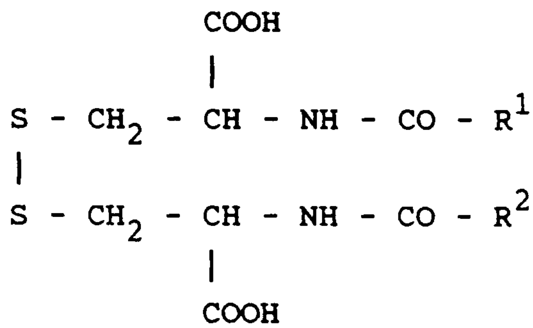

An optional step in all methods A-O is transferring of the obtained compound into a physiologically acceptable salt.

**Working examples**

Example 1. **(R,R)-N,N'-dipentanoyl-3,3'-dithiobis(2-aminopropionic acid)dimethylester:** A suspension of L-cystinedimethylester dihydrochloride (1.0 g, 3 mmol) in THF (20 mL) (white slurry) was stirred and cooled to 0 °C. To the reaction mixture was added 4 equiv. (2.0 mL) of N-ethyldiisopropylamine and 2.2 equiv. (0.8 mL, 6.6 mmol) of pentanoylchloride. The mixture was stirred for 4 h on the ice-bath and the white precipitate of N-ethyldiisopropylammonium chloride was removed by filtration. The solvent was removed by evaporation and the crude product was redissolved in dichlormethane. After washing with water the organic phase was dried over sodium sulphate. Filtration and evaporation of the solvent gave the crude title product, which was recrystallised from ethyl acetate.
Physical data:
$^1$H-NMR (300 MHz, $CDCl_3$): δ 6.42 (2H, d, NH), 4.88 (2H, dt, NCH), 3.77 (6H, s, $OCH_3$), 3.21 (4H, m, $SCH_2$), 2.27 (4H, t, $COCH_2$), 1.63 (4H, m, $CH_3CH_2CH_2$), 1.36 (4H, m, $CH_3CH_2$), 0.92 (6H, t, $CH_3$). $\alpha_D^{25}$ = -146 ° (C = 0,490, MeOH). Mp = 110-112 °C. Anal. Calcd for $C_{18}H_{32}O_6N_2S_2$: C, 49.52; H, 7.39; N, 6.42; S, 14.69. Found: C, 49.40; H, 7.40; N, 6.40; S, 14.70.

Example 2. **(R,R)-N,N'-dipropionyl-3,3'-dithiobis(2-aminopropionic acid)dimethylester:** The compound was prepared according to the procedure described in Example 1 using propionylchloride as acylating reagent.
Physical data:
$^1$H-NMR (300 MHz, $CDCl_3$): δ 6.44 (2H, d, NH), 4.89 (2H, dt, NCH), 3.78 (6H, s, $OCH_3$), 3.22 (4H, m, $SCH_2$), 2.31 (4H, q, $CH_3CH_2CH_2$), 1.18 (6H, t, $CH_3$).

Example 3. **(R,R)-N,N'-dipentanoyl-3,3'-dithiobis(2-aminopropionic acid)diethylester:** The compound was prepared according to the procedure described in Example 1 starting from L-cystinediethylester dihydrochloride and using pentanoylchloride as acylating reagent.
Physical data:
$^1$H-NMR (300 MHz, $CDCl_3$): δ 6.43 (2H, d, NH), 4.85 (2H, dt, NCH), 4.23 (4H, m, $OCH_2$), 3.22 (4H, m, $SCH_2$), 2.27 (4H, t, $COCH_2$), 1.63 (6H, m, $CH_3CH_2CH_2$), 1.37 (4H, m, $CH_3CH_2$), 1.30 (6H, t, $OCH_2CH_3$, 0.92 (6H, t, $CH_3$). $\alpha_D^{28}$ = -130 ° (C = 0.514, MeOH). Mp = 109 °C. Anal. Calcd for $C_{20}H_{36}O_6N_2S_2$: C, 51.70; H, 7.81; N, 6.03; S, 13.80. Found: C, 51.85; H, 7.75; N, 6.10; S, 13.60.

Example 4. **(R,R)-N,N'-dipropionyl-3,3'-dithiobis(2-aminopropionic acid)diethylester:** The compound was prepared according to the procedure described in Example 1 starting from L-cystine diethylester dihydrochloride and using propionylchloride as acylating reagent.
Physical data:

$^1$H-NMR (300 MHz, $CDCl_3$): δ 6.44 (2H, d, NH), 4.86 (2H, dt, NCH), 4.23 (4H, m, $OCH_2$), 3.22 (4H, m, $SCH_2$), 2.31 (4H, q, $COCH_2$), 1.30 (6H, t, $CO_2CH_2CH_3$, 1.18 (6H, t, $CH_3$).

Example 5. **(R,R)-N,N'-dihexanonyl-3,3'-dithiobis(2-aminopropionic acid)diethylester:** The compound was prepared according to the procedure described in Example 1 starting from L-cystinediethylester dihydrochloride and using hexanoylchloride as acylating reagent.

Physical data:

$^1$H-NMR (300 MHz, $CDCl_3$): δ 6.44 (2H, d, NH), 4.86 (2H, dt, NCH), 4.23 (4H, m, $OCH_2$), 3.21 (4H, m, $SCH_2$), 2.26 (4H, t, $COCH_2$), 1.65 (4H, m, $COCH_2CH_2$), 1.31 (4H, m, $CH_3CH_2CH_2$, 1.31 (4H, m, $CH_3CH_2$), 1.31 (6H, t, $OCH_2CH_3$), 0.90 (6H, t, $CH_3$).

Example 6. **(R,R)-N,N'-di(2-methylpropionyl)-3,3'-dithiobis(2-aminopropionic acid)diethylester:** The compound was prepared according to the procedure described in Example 1 starting from L-cystinediethylester dihydrochloride and using 2-methylpropanoylchloride as acylating reagent.

Physical data:

$^1$H-NMR (300 MHz, $CDCl_3$): δ 6.42 (2H, d, NH), 4.84 (2H, dt, NCH), 4.23 (4H, m, $OCH_2$), 3.22 (4H, m, $SCH_2$), 2.46 (2H, m, $(CH_3)_2CH$), 1.30 (6H, t, $OCH_2CH_3$), 1.18 (12H, d, $(CH_3)_2$). $\alpha_D^{25}$ = -127° (C = 0,512, MeOH). Mp = 140-141 °C. Anal. Calcd for $C_{18}H_{32}O_6N_2S_2$: C, 49.52; H, 7.39; N, 6.42; S, 14.69. Found: C, 49.15; H, 7.40; N, 6.30; S, 14.75.

Example 7. **(R,R)-N,N'-di(1-oxo-dodecanyl)-3,3'-dithiobis(2-aminopropionic acid) dimethylester:** The compound was prepared according to the procedure described in Example 1, starting from (R,R)-3,3'-dithiobis(2-aminopropionic acid) dimethylester dihydrochloride and using dodecanoic acid chloride as the acylating agent.

Total yield: 40%

Physical data:

Mp = 98-99 °C. $[\alpha]_D^{25}$ = -93° (c = 0.531, MeOH). $^1$H-NMR (300 MHz $CDCl_3$) δ 6.42 (2H, d, NH) 4.88 (2H, dt, NCH) 3.77 (6H, s, $OCH_3$) 3.21 (4H, m, $SCH_2$) 2.26 (4H, t, $OCH_2$), 1.64 (4H, m, $OCH_2CH_2$), 1.26 (32H, m, $(CH_2)_8$), 0.88 (6H, t, $CH_2CH_3$). Anal. Calcd for $C_{32}H_{60}N_2O_6S_2$: C, 60.72; H, 9.56; N, 4.43; S, 10.13.

Found: C, 60.4; H, 9.3; N, 4.5; S 10.1

Example 8. **(S,S)-N,N'-di(2-methylpropionyl)-3,3'-dithiobis(2-aminopropionic acid)dimethylester:** N,N'-diisobutyryl-D-cystine (1.86 g, 4.9 mmol) was dissolved in 10 ml of methanol containing one drop of hydrochloric acid. Trimethylorthoformate (0.6 ml, 5.5 mmol) was added and the reaction mixture was stirred at room temperature for 4 days. After evaporation of the solvent, the product was purified by column chromatography (solvent heptane:ethyl acetate 1:5).

Physical data: Mp 145.5-147.5 °C. $^1$H-NMR (300 MHz $CDCl_3$) δ 6.39 (2H, d, NH) 4.86 (2H, dt, NCH) 3.78 (6H, s, $OCH_3$) 3.21 (4H, m, $SCH_2$) 2.47 (2H, h, $CH(CH_3)_2$) 1.18 (12H, d, $CH(CH_3)_2$. $[\alpha]_D^{25}$ = + 135,2 (C = 0.42, MeOH)

Example 9. **(R,R)-N,N'-dihexanoyl-3,3'-dithiobis(2-aminopropionic acid)dimethylester:** The compound was prepared according to the procedure described in Example 1 using hexanoylchloride as acylating reagent.

Physical data:

$^1$H-NMR (300 MHz, $CDCl_3$): δ 6.41 (2H, d, NH), 4.88 (2H, dt, NCH), 3.76 (6H, s, $OCH_3$), 3.21 (4H, m, $SCH_2$), 2.26 (4H, t, $COCH_2$), 1.65 (4H, q, $CH_3CH_2CH_2CH_2$), 1.33 (4H, m, $CH_3CH_2$, 1.33 (4H, m, $CH_3CH_2$), 0.89 (6H, t, $CH_3$). $\alpha_D^{25}$ = -135° (C = 0,486,MeOH). Mp = 90-92 °C. Anal. Calcd for $C_{20}H_{36}N_2O_6S_2$: C, 51.70; H, 7.81; N, 6.03; S, 13.80.

Found: C, 51.90; H, 7.95; N, 6.10; S, 13.75.

Example 10. **(R,R)-N,N'-di(1-oxo-octyl)-3,3'-dithiobis-(2-aminopropionic acid)dimethylester:** The compound was prepared according to the procedure described in Example 1 using octanoylchloride as acylating reagent.

Physical data:

$^1$H-NMR (300 MHz, $CDCl_3$): δ 6.41 (2H, d, NH), 4.88 (2H, dt, NCH), 3.77 (6H, s, $OCH_3$), 3.21 (4H, m, $SCH_2$), 2.26 (4H, t, $COCH_2$), 1.65 (4H, t, $COCH_2CH_2$), 1.29 (16H, m, $CH_3(CH_2)$1-4), 0.88 (6H, t, $CH_2CH_3$). $\alpha_D^{25}$ = -119° (C = 0,490, MeOH). Mp = 90-91° Anal. Calcd for $C_{24}H_{44}N_2O_6S_2$: C, 55.36; H, 8.52; N, 5.38; S, 12.31. Found: C, 54.80; H, 8.45; N, 5.35; S, 11.80.

Example 11. **(R,R)-N,N'-di(1-oxo-octyl)-3,3'-dithiobis(2-aminopropionic acid)diethylester:** The compound was prepared according to the procedure described in Example 1 starting from L-cystinediethylester dihydrochloride and using octanoylchloride as acylating reagent.

Physical data:

$^1$H-NMR (300 MHz, $CDCl_3$): δ 6.43 (2H, d, NH), 4.85 (2H, dt, NCH), 4.23 (4H, m, $CH_3CH_2O$), $_3$.21 (4H, m, $SCH_2$), 2.26 (4H, t, $COCH_2$), 1.65 (4H, t, $COCH_2CH_2$), 1.30 (6H, m, $OCH_2CH_3$), 1.30 (16H, m, $CH_3(CH_2)$1-4),

0.88 (6H, t, $CH_2CH_3$).

Example 12. **(R,R)-N,N'-di(2,2-dimethylpropionyl)-3,3'-dithiobis(2-aminopropionic acid)-diethylester:** The compound was prepared according to the procedure described in Example 1 starting from L-cystinediethylester dihydrochloride and using 2,2-dimethylpropanoylchloride as acylating reagent.

Physical data:

$^1$H-NMR (300 MHz, $CDCl_3$): δ 6.52 (2H, d, NH), 4.80 (2H, dt, NCH), 4.22 (4H, m, $CH_3CH_2O$), 3.22 (4H, m, $SCH_2$), 1.30 (6H, t, $OCH_2CH_3$), 1.23 (18H, s, $C(CH_3)_3$).

Example 13. **(R,R)-N,N'-di(2,2-dimethylpropionyl)-3,3'-dithiobis(2-aminopropionic acid)-dimethylester:** The compound was prepared according to the procedure described in Example 1 using 2,2-dimethylpropanoylchloride as acylating reagent.

Physical data:

$^1$H-NMR (300 MHz, $CDCl_3$): δ 6.51 (2H, d, NH), 4.83 (2H, dt, NCH), 3.78 (6H, s, $OCH_3$), 3.21 (4H, m, $SCH_2$), 1.23 (18H, s, $C(CH_3)_3$).

Example 14. **(R,R)-N,N'-di(2-methylpropionyl)-3,3'-dithiobis(2-aminopropionic acid)-dimethylester:** The compound was prepared according to the procedure described in Example 1 but with 2-methylpropionyl chloride replacing pentanoyl chloride.

Physical data:

Mp 142-5°C. δ 6.40 (2H,d,NH), 4.86(2H,dt, NCH), 3.78(6H, s, $OCH_3$), 3.21 (4H,m,$SCH_2$), 2.47 (2H,h,CH-$(CH_3)_2$), 1.18(12H,d,$CH(CH_3)_2$).

Example 15. **(R,R)-N-acetyl-N'-hexanoyl-3,3'-dithiobis-(2-aminopropionic acid)dimethylester:** -(R,R)-3,3'-dithiobis(2-aminopropionic acid)dimethylester dihydrochloride (690 mg, 2 mmol) was stirred together with 1.39 ml (10 mmol) of triethylamine in 20 mL of THF in a 100 mL round bottomed flask. The turbid solution was cooled to 0°C on an ice bath before a solution of acetyl chloride (142 µL, 2 mmol) and hexanoylchloride (276 µL, 2 mmol) in 3 mL of THF was added dropwise. The turbid solution was stirred for 1 h at 0°C. A white precipitate of 1 g (100%) $Et_3NHCl$ was filtered off and the filtrate was evaporated to give an oily residue, which was partitioned between 10 mL of $H_2O$ and 10 mL of $CHCl_3$. After separating the phases, the aqueous phase was extracted with 3x10 mL of $CHCl_3$. After evaporating the combined $CHCl_3$-phases the crude product (containing the desired product and the symmetrical derivatives) was separated by Flash chromatography according to Still et al. (J. Org. Chem., 1978, 43, 2923) on silica gel 60 (E. Merck 5735, 230-400 mesh ASTM), with EtOAc/Heptane/MeOH 6:3:1 as the eluent. The separation was monitored by thin layer chromatography (plastic sheets silica gel 60 $WF_254s$, E. Merck 16483, with EtOAc/Heptane/MeOH 6:3:1 as eluent and $I_2$ as visualization agent). The fractions with acceptable purity were pooled and evaporated to give an oily residue. This residue was dissolved in acetone and the solution was again evaporated to give the title product as a colourless solid.

Yield 184 mg, 25%.

Physical data:

TLC: $R_f = 0.30$ (EtOAc/Heptane/MeOH = 6/3/1).

$^1$H-NMR (300 MHz, $CDCl_3$): δ 6.53 (1H, d, NH), 6.43 (1H, d, NH), 4.88 (2H, m, NCH), 3.78 (6H, s, $CO_2CH_3$), 3.22 (4H, m, $SCH_2$), 2.26 (2H, t, $COCH_2$), 2.07 (3H, s, $COCH_3$), 1.65 (2H, m, $CH_2$), 1.32 (4H, m, $CH_2$), 0.90 (3H, t, $CH_3$). FAB-MS (m/z): 431 $[MNa]^+$, 409 $[MH]^+$, 311 $[MH-C_7H_{11}O]^+$.

Exampel 16. **(R,R)-N,N'-di(2-methylpropionyl)-3,3'-dithiobis(2-aminopropionic acid):** N-Isobutyryl-L-cysteine (9.5 g, 50 mmol) was dissolved in 50 mL of water. Hydrogen peroxide (30%, 3.1 mL, 30 mmol) was added and the reaction mixture was stirred at room temperature for 6 hours. After evaporation of the solvent under reduced pressure, a white crystallised oil (9.8 g) was obtained. Recrystallisation from ethyl acetate furnished the title compound as a white solid which was dried in vacuo.

Yield: 4.8 g (50%). Physical data: Mp. 143-5°C; $^1$H-NMR (300 MHz, DMSO-$d_6$), δ (2H, b, $CO_2H$), 8.16 (2H, d, NH), 4.47 (2H, m, CHN), 3.15 (2H, dd, $CH_2S$, J=14Hz, 5Hz), 2.92 (2H, dd, $CH_2S$, J=14Hz, 9Hz), 2.43 (2H, h, $CH(CH_3)_2$, J=7Hz), 1.01 (12H, d, $CH_3$, J=7Hz). $[\delta]_d^{25}$ = -169.8 (MeOH, C=0.510)

Example 17. **(R,R)N-Acetyl-3,3'-dithiobis(2-aminopropionic acid):** L-cysteine (2.42 g, 20 mmol) and N-acetyl-L-cysteine (3.26 g, 20 mmol) were dissolved in 25 mL of water. The pH of this solution was 2.6 according to lithmus paper. Aqueous hydrogen peroxide (30%, 2.3 mL, 21 mmol) was added, and the reaction mixture was allowed to stand at room temperature overnight. A white precipitate was filtered off (1.26 g). This material was shown to be L-cystine by comparison of spectral data with an authentic sample. The yellowish filtrate, containing the desired compound and the two symmetrical compounds (R,R)-N,N'-diacetylcystine and (minor amounts of) cystine was separated on a preparative HPLC using a Dynamax $C_18$-column (8 µm, 60 Å, 21.4 x 250 mm) with a Dynamax $C_18$-guard column (8 µm, 21.4 x 50 mm) and with Gilson dual solvent delivery system (305 pump, pumphead 100 SC acting as a solvent delivery pump, pumphead 5 SC acting as a sample injector, 806 manometric module, 811B dynamic mixer, 115 UV

detector, 201 fraction collector, 201-202 fraction controller). The solvents used were A = 10 mM $HOAc/H_2O$ and B = MeOH, with a 95:5 ratio of A:B. The solvent flow was 10 mL/min and the separation was recorded at 230 nm. After detection of each fraction on TLC (Merck 16483, plastic sheets silica gel 60 WF 254s, eluent $^nBuOH/HOAc/H_2O$ 1:1:1, $I_2$ as visualization agent), the fractions with acceptable purity were pooled and evaporated to give an oily residue. This residue was dissolved in acetone (pa) and the solution was evaporated to give the title product as white crystals. Yield: 10%.

Physical data:

TLC: $R_f = 0.69$ ($^nBuOH/HOAc/H_2O$ = 1/1/1). $^1H$-NMR (300 MHz, $D_2O$):

$\delta$ 4.73 (1H, dd, NCH), 4.18 (1H, dd, NCH), 3.37 (2H, m, $SCH_2$), 3.10 (2H, m, $SCH_2$), 2.06 (3H, s, $CH_3$). TSP-MS (m/z): 283 $[MH]^+$, 164 $[MH-C_3H_5NO_2]^+$.

Example 18. **(R,R)-N-Acetyl-N'-(2-methylpropionyl)-3,3'-dithiobis(2-aminopropionic acid):** A mixture of N-acetyl-L-cysteine (0.652 g, 4 mmol) and N-isobutyryl-L-cysteine (0.764 g, 4 mmol) in 10 mL of MeOH was stirred while hydrogen peroxide (30%, 0.60 mL, 5 mmol) was added dropwise. The stirring was continued for 3 hours at room temperature, after which the solvent was removed on the rotary evaporator. Addition of 25 mL of acetone and repeated evaporation afforded 1.5 g of crude material as an oil which solidified on standing. The desired title compound was isolated from this mixture by preparative HPLC as described in Example 17. Yield: 31%.

Physical data:

HPLC elution with 60% A (isocratic, for solvents see Example 17). TLC: $R_f = 0.76$ ($^nBuOH/H_2/HOAc$ = 1/1/1). $^1H$-NMR (300 MHz, $D_2O$):

$\delta$ 4.73 (2H, m, NCH), 3.33 (2H, m, $SCH_2$), 3.03 (2H, m, $SCH_2$), 2.59 (1H, n, H), 2.06 (3H, s, $COH_3$), 1.13 (3H, d, $CH_3$), 1.11 (3H, s, $CH_3$). FAB-MS (m/z): 376 $[MNa]^+$, 353 $[MH]^+$.

Example 19. **(R,R)-N-(2-methylpropionyl)-3,3'-dithiobis-(2-aminopropionic acid):** The compound was prepared by the procedure given in Example 17, starting from L-cysteine and N-isobutyryl-L-cysteine. Yield: 8%.

Physical data: HPLC elution with 55% A (isocratic, see Example 17 for solvents). TLC: $R_f = 0.67$ $^nBuOH/H_2O/HOAc$ = 1/1/1). $^1H$-NMR (300 MHz, $D_2O$): $\delta$ 4.73 (1H, dd, NCH), 4.14 (1H, dd, NCH), 3.38 (2H, n, $SCH_2$), 3.08 (2H, m, $SCH_2$), 2.06 (1H, n, CH), 1.15 (3H, d, $CH_3$), 1.13 (3H, d, $CH_3$). TSP-MS (m/z): 311 $[MH]^+$.

Example 20. **(R,R)-N-Acetyl-N'-(2,2-dimethylpropionyl)-3,3'-dithiobis(2-aminopropionic acid):** The compound was obtained following the procedure given in Example 18, starting from N-acetyl-L-cysteine and N-pivaloyl-L-cysteine. Yield: 21%.

Physical data:

HPLC elution gradient: 50% A/15 min, 50 30% A/5 min, 30% A/ isocratic (for solvents see Example 17). TLC: $R_f = 0.78$ $^nBuOH/H_2O/HOAc$ = 1/1/1).

$^1H$-NMR (300 MHz, $D_2O$): $\delta$ 4.72 (2H, m, NCH), 3.35 (2H, m, $CH_2$), 3.04 (2H, m, $SCH_2$), 2.06 (3H, s, $CH_3$), 1.21 (9H, s, $CCH_3$).

FAB-MS (m/z): 411 $[MNa_2]^+$, 389 $[MNa]^+$, 367 $[MH]^+$, 349 $[MH-H_2O]^+$.

Example 21. **(R,R)-N,N'-di(2,2-dimethylpropionyl)-3,3'-dithiobis(2-aminopropionic acid):** The compound was isolated from the crude material of Example 20. Yield: 15%.

Physical data:

HPLC elution gradient: 50% A/15 min, 50 30% A/15 min, 30%. A/isocratic (see Example 17 for solvents). TLC: $R_f = 0.88$ $^nBuOH/H_2O/HOAc$ = 1/1/1).

$^1H$-NMR (300 MHz, $D_2O$): $\delta$ 4.72 (2H, dd, NCH), 3.37 (2H, dd, $SCH_2$), 3.06 (2H, dd, $SCH_2$), 1.21 (18H, s, $CCH_3$).

FAB-MS (m/z): 431 $[MNa]^+$, 409 $[MH]^+$, 391 $[MH-H_2O]^+$.

Example 22. **(R,R)-N-Acetyl-N'-pentanoyl-3,3'-dithiobis-(2-aminopropionic acid):** This material was prepared as in Example 18, starting from N-acetylcysteine and N-pentanoylcysteine. Yield: 23%.

Physical data:

HPLC elution gradient: 50% A/20 min, 50 25% A/5 min, 25%

A/isocratic (see Example 17 for solvents). TLC: $R_f = 0.84$ n-$BuOH/H_2O/HOAc$ = 1/1/1).

$^1H$-NMR (300 MHz, $D_2O$): $\delta$ 4.70 (2H, m, NCH), 3.30 (2H, n, $SCH_2$), 2.99 (2H, m, $SCH_2$), 2.30 (2H, t, $COCH_2$), 2.04 (3H, s, $COCH_3$), 1.57 (2H, m, $CH_2$), 1.30 (2H, m, $CH_2$), 0.86 (3H, t, $CH_3$).

FAB-MS (m/z): 389 $[MNa]^+$, 367 $[MH]^+$, 349 $[MH-H_2O]^+$.

Example 23. **(R,R)-N,N'-dihexanoyl-3,3'-dithiobis(2-aminopropionic acid):**

A suspension of 1 equiv. 3,3'-dithiobis(2-aminopropionic acid) dimethylester dihydrochloride in tetrahydrofuran (white slurry) was stirred and cooled to O °C. To the reaction mixture was added 4 equiv. of N-ethyldiisopropylamine and 2.2 equiv. of hexanoylchloride. The mixture was stirred for 4 hrs on an ice bath

and the white precipitate of N-ethyldiisopropylammonium chloride was filtered off. The solvent was removed by evaporation under reduced pressure and the crude product was redissolved in dichlormethane. After washing with water, the organic phase was dried over sodium sulphate. Filtration and evaporation of the solvent gave crude (R,R)-N,N'-dihexanoyl-3,3'-dithiobis(2-aminopropionic acid) dimethylester, which was recrystallized from methanol/water and triturated with diethylether.

A white slurry (0.1 M) of the above formed dimethylester and 0.5 M sodium hydroxide in 10% methanol was stirred vigorously at room temperature. After about 48 hrs the pH of the clear solution was adjusted to 2, and the formed white precipitate of crude product was filtered off and recrystallized from acetone/hexane to give the title compound as white crystals. Total yield: 28%.

Physical data:

Mp: 132-135 °C. $[\alpha]_D^{25}$: -164° (c = 0.501, MeOH). $^1$H-NMR (300 MHz, DMSO-$d_6$), $\delta$ 8.22 (2H, d, NH), 4.49 (2H, m, CHN), 3.14 (2H, dd, $CH_2S$), 2.91 (2H, dd, $CH_2S$), 2.12 (4H, t, $CH_2CO$), 1.50 (4H, p, $CH_2CH_2CO$), 1.26 (8H, m, $(CH_2)_2$), 0.87 (6H, t, $CH_3$). Anal. Calcd for $C_{18}H_{32}N_2O_6S_2$: C, 49.5; H, 7.4; N, 6.4; S, 14.7. Found: C, 49.4; H, 7.2; N, 6.2; S, 14.3.

Example 24 **(R,R)-N,N'-di(1-oxo-octyl)-3,3'-dithiobis(2-aminopropionic acid):**

The compound was prepared according to the procedure described in Example 23, using octanoic acid chloride as the acylating agent.

The initially formed (R,R)-N,N'-di(1-oxo-octyl)-3,3'-dithiobis(2-aminopropionic acid) dimethylester was recrystallized from ethyl acetate and the title compound was recrystallized from acetone/heptane and triturated with diethylether to give white crystals. Total yield: 20%

Physical data:

Mp: 105-107 °C. $[\alpha]_D^{25}$:-147° (c = 0.541, MeOH). $^1$H-NMR (300 MHz, DMSO-$d_6$), $\delta$ 8.18 (2H, d, NH), 4.48 (2H, m, CHN), 3.17 (2H, dd, $CH_2S$), 2.89 (2H, dd, $CH_2S$), 2.12 (4H, t, $CH_2CO$), 1.49 (4H, m, $CH_2CH_2CO$), 1.26 (16H, m, $(CH_2)_4$), 0.87 (6H, t, $CH_3$). Anal. Calcd for $C_{22}H_{40}N_2O_6S_2$: C, 53.6; H, 8.2; N, 5.7; S, 13.0. Found: C, 53.4; H, 8.5; N, 5.7; S, 12.6.

Example 25 **(R,R)-N,N'-di(1-oxo-dodecanyl)-3,3'-dithiobis(2-aminopropionic acid):**

The compound was prepared according to the procedure described in Example 23, using dodecanoic acid chloride as the acylating agent. The hydrolysis was carried out in 20% methanol.

The initially formed (R,R)-N,N'-di(1-oxo-dodecanyl)-3,3'-dithiobis(2-aminopropionic acid) dimethylester was recrystallized from ethyl acetate. The title compound was recrystallized from toluene/dichloromethane to give white crystals.

Total yield: 23%

Physical data:

Mp: 110-112 °C. $[\alpha]_D^{25}$: -113° (c = 0.507, MeOH). $^1$H-NMR (300 MHz, DMSO-$d_6$), $\delta$ 8.19 (2H, d, NH), 4.48 (2H, m, CHN), 3.15 (2H, dd, $CH_2S$), 2.89 (2H, dd, $CH_2S$), 2.11 (4H, t, $CH_2CO$), 1.49 (4H, m, $CH_2CH_2CO$), 1.25 (32H, m, $(CH_2)_8$), 0.87 (6H, t, $CH_3$). Anal. Calcd for $C_{30}H_{56}N_2O_6S_2$: C, 59.6; H, 9.3; N, 4.6; S, 10.6. Found: C, 59.2; H, 9.4; N, 4.6; S, 10.3.

Example 26 **(S,S)-N,N'-di(2-methylpropionyl)-3,3'-dithiobis(2-aminopropionic acid):**

Potassium carbonate (17.6 g, 127 mmol) was dissolved in 40 ml of water and 40 ml of methylene chloride under nitrogen. The solution was cooled (-10 °C) and 2-methylpropionoyl chloride (5.6 ml, 40 mmol) and D-cysteine hydrochloride monohydrate (8.7 g, 49.5 mol) was added quickly. The reaction mixture was stirred at room temperature for 4 hours, after which hydrochloric acid was added until the pH of the mixture became less than 1 according to lithmus paper. The aqueous layer was discarded and light petroleum (40-60°) was added to the organic layer whereupon N-isobutyryl-D-cysteine precipitated as white crystals.

N-isobutyryl-D-cysteine (1 g, 5.2 mmol) was dissolved in 10 ml of methanol. Hydrogen peroxide (30%, 0.3 ml, 2.6 mmol) was added and the reaction mixture was stirred at room temperature for 6 hours. After evaporation of the solvent under reduced pressure, a white crystallised oil was obtained. Recrystallisation from ethyl acetate furnished the title compound as a white solid which was dried in vacuo.

Yield: 0.55 g (55%) Physical data: Mp 135-137 °C. $^1$H-NMR (300 MHz, DMSO-$d_6$) $\delta$ 8.16 (2H, d, NH) 4.47 (2H, m, CHN) 3.15 (2H, dd, $CH_2S$, J = 14 Hz, 5 Hz) 2.93 (2H, dd, $CH_2S$, J = 14 Hz, 9 Hz) 2.43 (2H, h, $CH(CH_3)_2$ J = 7 Hz) 1.01 (12H, d, $CH_3$, J = 7 Hz).

$[\alpha]_D^{25}$ = +167.6 (c = 0.516, MeOH).

Example 27.

Formulation A

Tablet containing 10 mg of active substance per tablet:

| | |
|---|---|
| Active substance | 10 mg |
| Lactose | 100 mg |
| Potato starch | 50 mg |
| Polyvinylpyrrolidone | 5 mg |
| Microcrystalline cellulose | 15 mg |
| Magnesium stearate | 1 mg |

Formulation B

Direct compression tablet containing 5 mg of active substance per tablet:

| | |
|---|---|
| Active substance | 5 mg |
| Lactose, anhydrous | 150 mg |
| Microcrystalline cellulose | 50 mg |
| Colloidal silicon dioxide | 1 mg |
| Magnesium stearate | 2 mg |

If desired, the obtained tablets can be film coated with e.g. hydroxypropyl methylcellulose, hydroxypropyl cellulose or dimethylaminoethyl methacrylate methacrylic acid ester copolymer.

Formulation C

Solution for injection containing active substance 1 mg/ml

| | |
|---|---|
| Active substance | 1.0 mg |
| Sodium chloride | 8.8 mg |
| Water for injection | to 1 ml |

Formulation D

Oral solution containing active substance 1 mg/ml

| | |
|---|---|
| Active substance | 1.0 mg |
| Sorbitol | 150 mg |
| Glycerin | 100 mg |
| Disodium edetate | 0.5 mg |
| Preservative | q.s. |
| Flavour | q.s. |
| Water, purified | to 1 ml |

Formulation E

Powder aerosol giving 1 mg per dose
The micronized active substance can be filled into a powder inhaler device e.g. Turbuhaler[R] giving 1 mg/dose.

**Effects of the compounds of the invention in a model of delayed type hypersensitivity in the mouse.**

The property of the compounds of the invention to stimulate immune responses are illustrated by their efficacy in a model in the mouse of the delayed type hypersensitivity (DTH) reaction.

Both male and female Balb/c mice obtained from Bomhotsgaard (Denmark) and Charlie Rivers (England), were used at the weight of 18-20 gram. 4-ethoxymethylene-2-phenyloxazolone (OXA) was purchased from BDH (England) and served as an antigen in this test.

The mice were sensitized, Day 0, by epicutaneous application of 150 ul absolute ethanol-acetone (3:1) solution containing 3% OXA on the shaved thorax and abdomen. Treatment with the compound under examination, DiNAC (as a positive control) or vehicle (phosphate buffer, pH 7.0) was initiated by oral feeding immediately after sensitization and contiuned once to Day 6. Seven days (Day 6) after the sensitization both ears of all mice were challenged on both sides by topical application of 20 ul 1% OXA dissolved in olive oil. Ear thickness was measured prior to and 24 or 48 hours after challenge using an Oditest spring calliper. Challenges and measurements were performed under light pentobarbital anaesthesia. The intensity of the DTH reactions was expressed according to the formula: $T_{t24/48}$-$T_{t0}$ um units, where t0 and t24/48 represent the ear thickness before and 24 or 48 hours after challenge, respectively, in individual test (T). The results were expressed as the mean+/-S.E.M. The level of significance between means of the groups was obtained by Student's two-tailed t-test. Table 1 shows representative results from 24 and 48 hours measurements expressed as % increase in ear thickness relative to that of the non-challenged reference ear. A figure of 100 thus indicates a doubled ear thickness.

Table 1

| Immunostimulatory action | | | | | | |
|---|---|---|---|---|---|---|
| **R** | **$R^2$** | **$R^3$** | **% Increase, 24h** | | **% Increase, 48h** | |
| | | | **0.03** | **3.0** | **0.03** | **3.0** |
| | | | **μmol/kg** | | **μmol/kg** | |
| $C(CH_3)_2$ | $C(CH_3)_2$ | $CH^3$ | 110*** | 92** | 17* | 27*** |
| $n\text{-}C_5H_{11}$ | $n\text{-}C_5H_{11}$ | $CH_3$ | 73** | 86** | 51*** | 45*** |
| $n\text{-}C_7H_{15}$ | $n\text{-}C_7H_{15}$ | $CH_3$ | 58*** | 52*** | 28** | 20* |
| $n\text{-}C_7H_{15}$ | $n\text{-}C_7H_{15}$ | $C_2H_5$ | 49*** | 7 | 0 | 0 |
| $COC(CH_3)_3$ | $C(CH_3)_3$ | H | 14* | | 26* | 27* |
| $COCH(CH_3)_2$ | $CH(CH_3)_2$ | H | 17 | 18 | 27** | 62*** |
| $COCH_3$ | $C(CH_3)_3$ | H | 24* | 26* | 14 | 17* |
| H | $CH(CH_3)_2$ | H | 37** | 31*** | | |

*, **, ***: $P < 0.05, 0.01, 0.001$

**Claims**

**Claims for the following Contracting States : AT, BE, CH, DE, DK, FR, GB, IT, LI, LU, NL, SE**

1. Compounds of the general formula

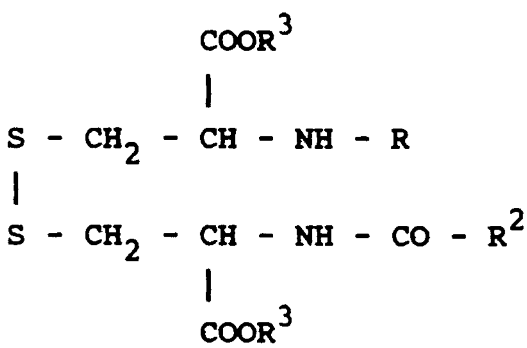

I

wherein R is hydrogen or a moiety -CO-$R^1$ wherein $R^1$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, $R^2$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl and $R^3$ is hydrogen, methyl, ethyl, propyl, isopropyl, butyl or isobutyl, provided that $R^1$ and $R^2$ are not simultaneously methyl and further provided that when $R^3$ is hydrogen $R^1$ and $R^2$ are not simultaneously n-propyl, n-heptyl, n-nonyl or n-undecyl and further provided that when R and $R^3$ are all hydrogen, then $R^2$ is not n-nonyl or a physiologically acceptable salt and/or a stereochemical isomer thereof.

2. A compound according to claim 1 wherein R is hydrogen or a moiety -CO-$R^1$ wherein $R^1$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, $R^2$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl and $R^3$ is hydrogen, methyl or ethyl, provided that $R^1$ and $R^2$ are not simultaneously methyl and further provided that when $R^3$ is hydrogen $R^1$ and $R^2$ are not simultaneously n-propyl, n-heptyl, n-nonyl or n-undecyl and further provided that when R and $R^3$ are all hydrogen then $R^2$ is not n-nonyl.

3. A compound according to claim 1 wherein $R^1$ and $R^2$ are isopropyl and $R^3$ is methyl.

4. A compound according to claim 1 wherein $R^1$ and $R^2$ are n-pentyl and $R^3$ is methyl.

5. A compound according to claim 1 wherein $R^1$ and $R^2$ are n-heptyl and $R^3$ is methyl or ethyl.

6. A compound according to claim 1 wherein $R^1$ and $R^2$ are simultaneously isopropyl and $R^3$ is hydrogen.

7. A compound according to claim 1 wherein $R^1$ and $R^2$ are simultaneously tert. butyl and $R^3$ is hydrogen.

8. A process for the preparation a compound of the general formula I as defined in claim 1, characterized by

a) the oxidation of an N-acylcysteine derivative of the formula

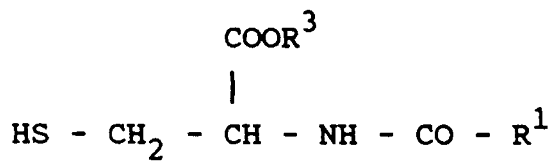

wherein $R^1$ and $R^3$ are as defined above or when $R^3$ is hydrogen optionally an alkali salt thereof, to the formation of a compound of the formula

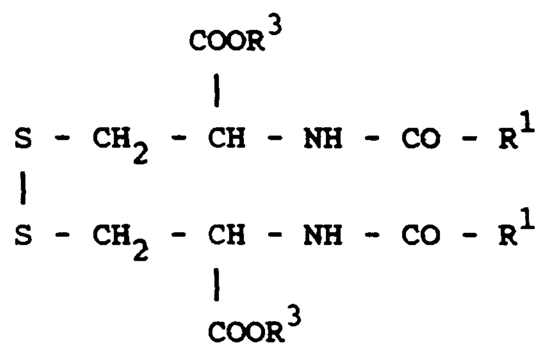

or

b) the reaction, in the presence of a suitable base, of a compound having the formula

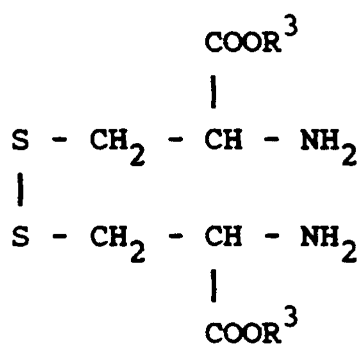

or a salt thereof wherein $R^3$ is as defined above, with a compound of the formula

$R^1$ - COX

wherein $R^1$ is defined as above and COX is a reactive group capable of reacting with an amino group under formation of an amide moiety, to the formation of a compound of the formula

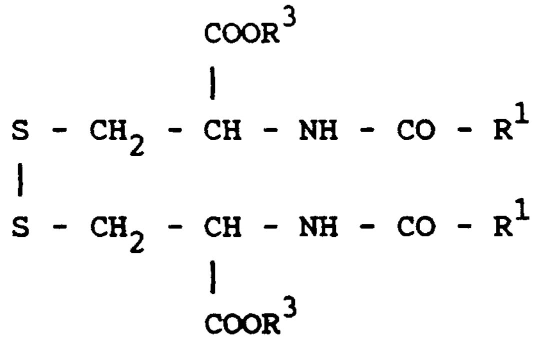

or

c) the reaction of 2-(N-acylamino)-3-halopropionic acid derivative of the formula

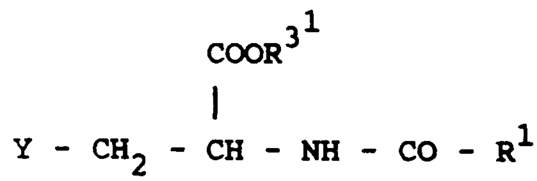

wherein $R^1$ is as defined above,

$R^{3^1}$

is $R^3$ as defined above or an acid or base labile organic group and Y is a halogen atom, with sulphur or disulphide dianion in the presence of a base, to the formation of a compound of the formula

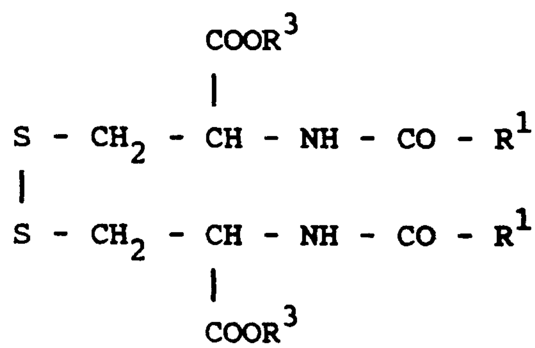

or

d) the oxidation of a mixture of N-acylcysteine ester derivatives of the formulas

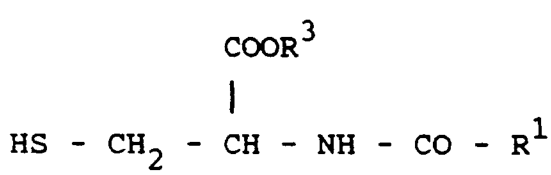

and

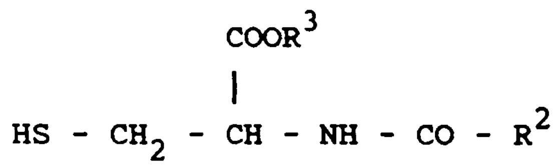

wherein $R^1$, $R^2$ and $R^3$ are as defined above or when $R^3$ is hydrogen optionally alkali salts thereof, to the formation of a compound of the formula

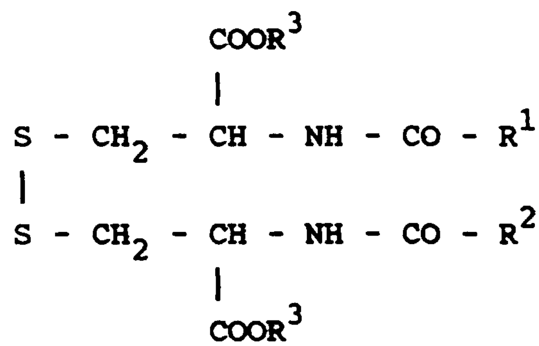

or

e) the oxidation of a mixture of cysteine or a cysteine ester and an N-acylcysteine derivative of the formulas

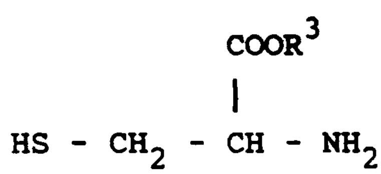

or a salt thereof and

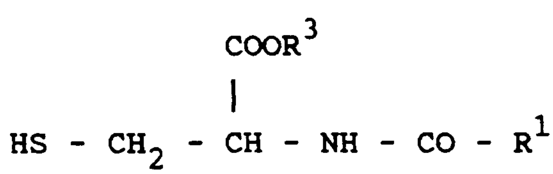

or an alkali or hydrochloride salt thereof,
wherein $R^1$ and $R^3$ are as defined above, to the formation of a compound of the formula

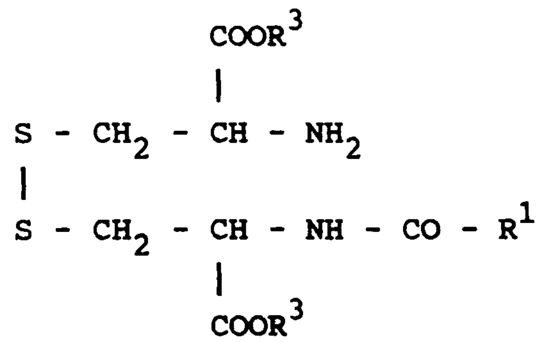

or a salt thereof,

or

f) the reaction, in the presence of a suitable base, of an excess of cysteine or a cystine diester having the formula

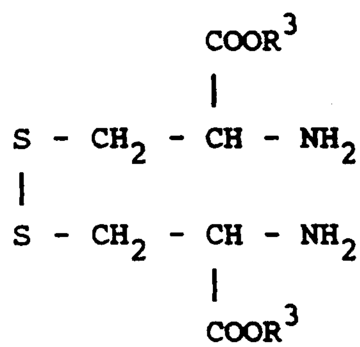

or a salt thereof wherein $R^3$ is as defined above, with a compound of the formula

$R^2$ - COX

wherein $R^2$ and COX are as defined above, to the formation of a compound of the formula

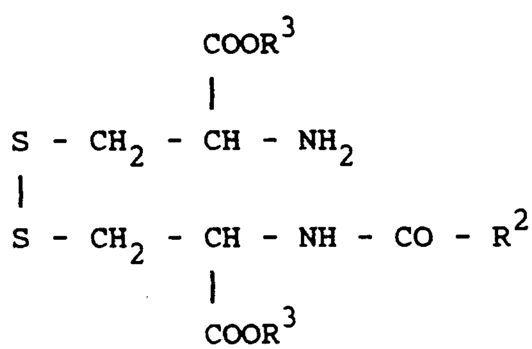

or

g) the reaction, in the presence of a suitable base, of a compound of the formula

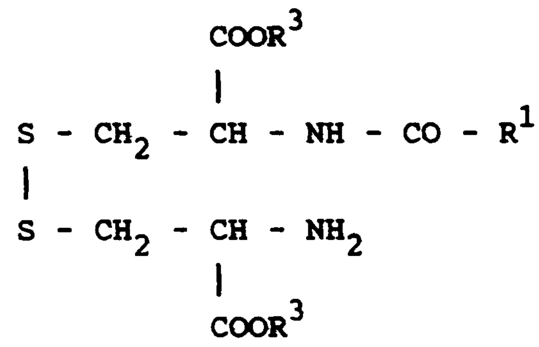

or a salt thereof wherein $R^1$ and $R^3$ are as defined above, with a compound of the formula

$R^2$ - COX

wherein $R^2$ and COX are as defined above, to the formation of a compound of the formula

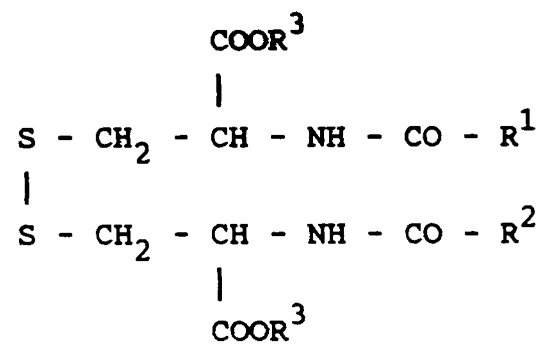

h) the reaction of an N-acylcysteine derivative of the formula

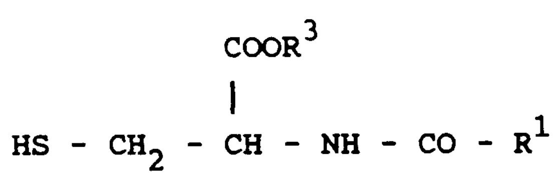

or a salt thereof,
wherein $R^1$ and $R^3$ are as defined above, with an activating reagent to the formation of an adduct of the formula

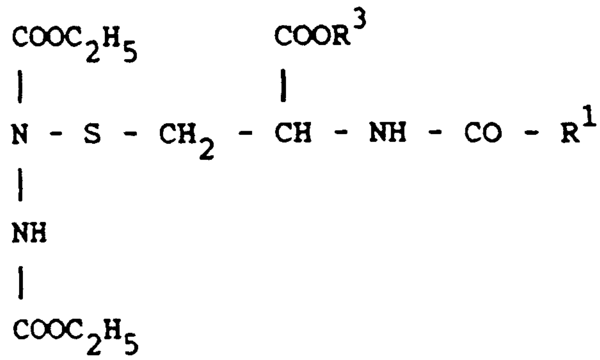

followed by reaction with a second, different or the same N-acylcysteine or cysteine ester derivative, to give a compound of the formula

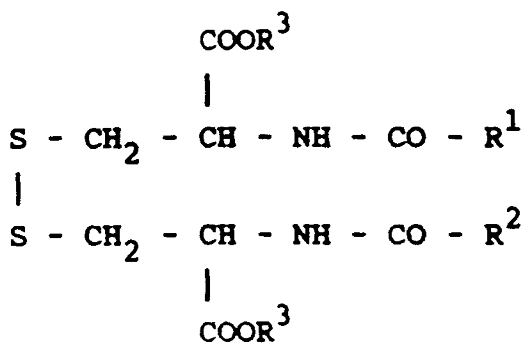

i) the reaction of a compound of the formula

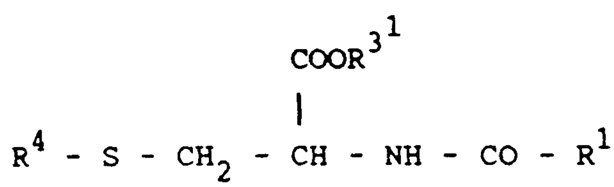

wherein $R^4$ is -Cl or

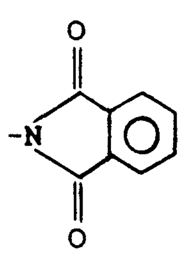

,

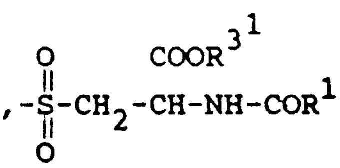

and $R^1$ and

$R^{3^1}$

are as defined above, with a compound of the formula

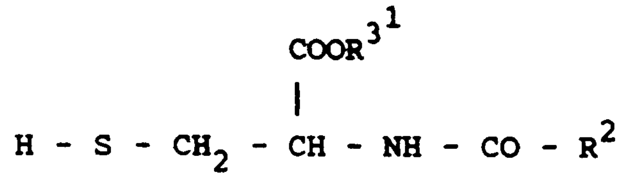

wherein $R^2$ and

$$R^{3^1}$$

are as defined above to the formation of a compound of the formula

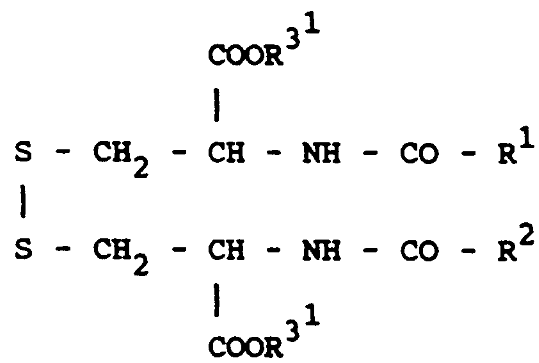

whereafter, if a compound wherein $R^3$ = H is desired,
removal of the protecting group

$$R^{3^1},$$

or
j) the esterification of a compound of the formula

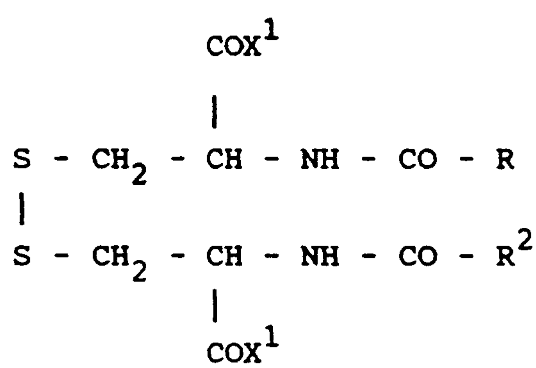

wherein R and $R^2$ are as defined above, and $X^1$ is OH or a halogen atom with a compound of the formula

$R^{3E}$-OH

wherein $R^{3E}$ is methyl, ethyl, propyl, isopropyl, butyl or isobutyl, to the formation of a compound of the formula

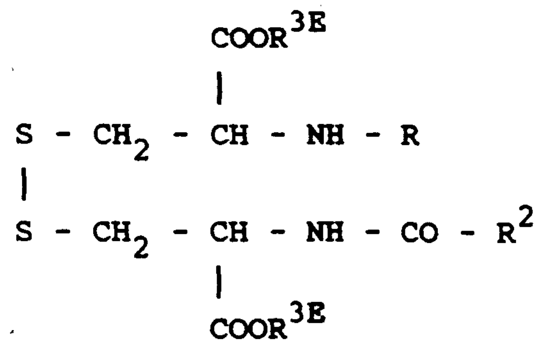

or

k) the alkylation of a compound of the formula

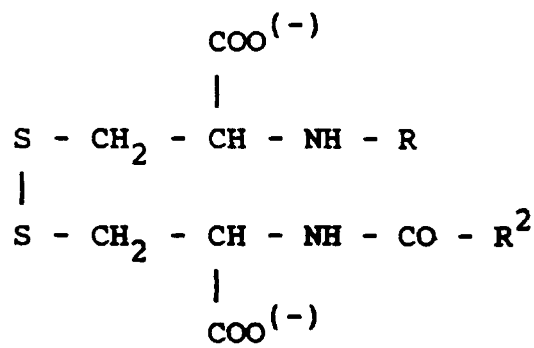

wherein R and $R^2$ are as defined above, with a compound of the formula

$R^{3E}$ - Z

Wherein $R^{3E}$ is as defined above and Z is halogen, alkylsulphate, tosylate or another nucleofuge to the formation of a compound of the formula

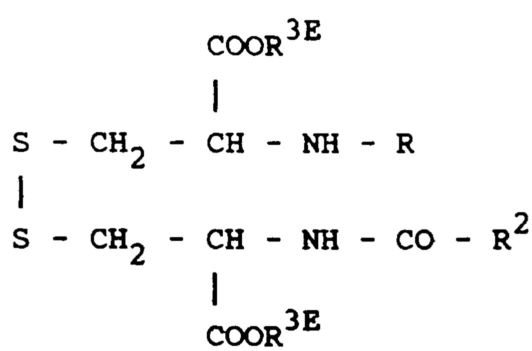

l) the reaction, in the presence of a suitable base, of a carboxyl protected cystine derivative of the formula

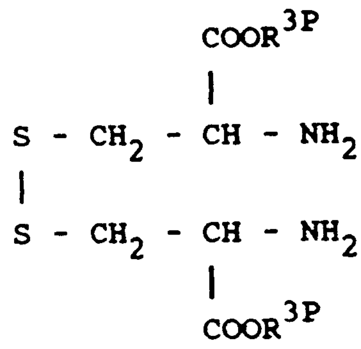

or a hydrochloride salt thereof, wherein $R^{3P}$ is an acid or base labile organic group, with a compound of the formula

$R^1$- COX

wherein $R^1$ is defined as above and COX is a reactive group capable of reacting with an amino group under formation of an amide moiety, and thereafter removal of the protecting group $R^{3P}$ to the formation of a compound of the formula

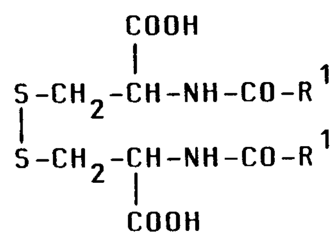

m) the reaction, under alkaline conditions, of a carboxyl protected cystine derivative of the formula

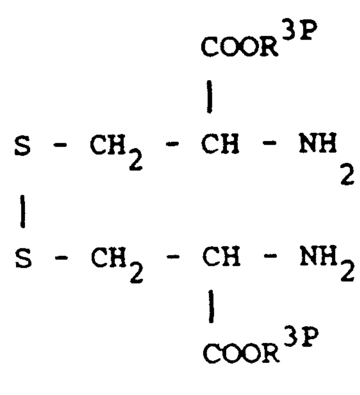

or a hydrochloride salt thereof, wherein $R^{3P}$ is as defined above, with a compound of the formula

$R^2$ - COX

wherein $R^2$ and COX are as defined above and thereafter removal of the protecting group $R^{3P}$ to the formation of a compound of the formula

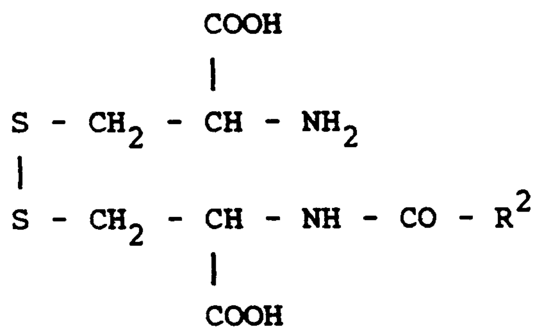

n) the reaction of a carboxyl protected cystine derivative of the formula

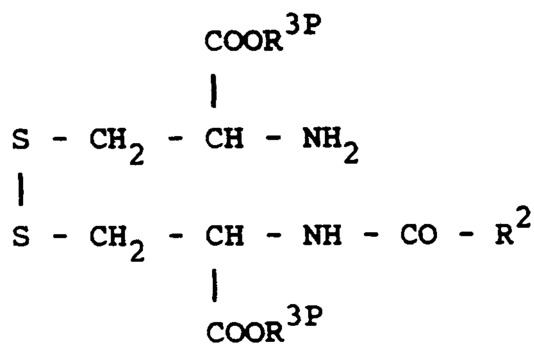

or a hydrochloride salt thereof, wherein $R^2$ and $R^{3P}$ are as defined above, with a compound of the formula

$R^1$ - COX

wherein $R^1$ and COX are as defined above and thereafter removal of the protecting group $R^{3P}$ to the formation of a compound of the formula

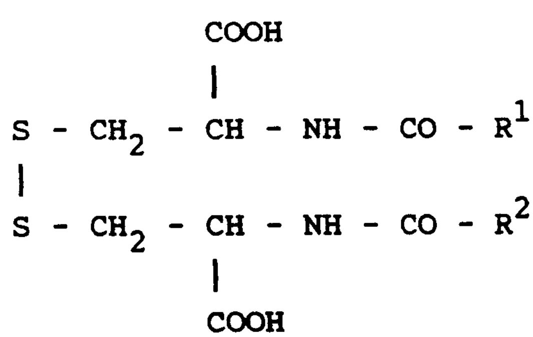

o) the equilibration, in alkaline aqueous solution of a mixture of a cystine derivative and a cysteine derivative having the formulas

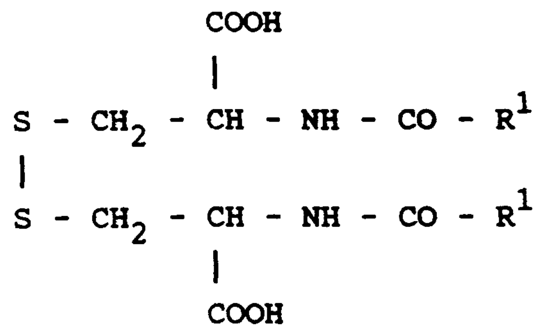

and

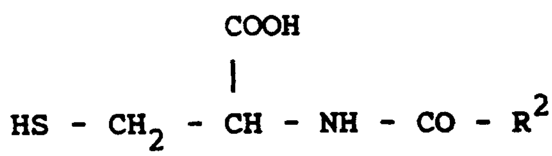

or alkali salts thereof, wherein $R^1$ and $R^2$ are as defined above, to the formation of a compound of the formula

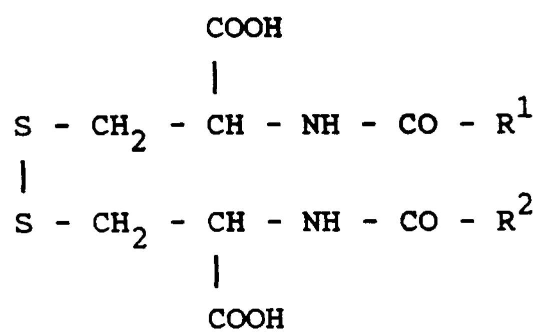

and finally, if desired, transferal of the compounds obtained by any of the methods a) - o) into a physiologically acceptable salt.

**9.** A pharmaceutical preparation comprising as active ingredient a compound according to any of claims 1-7.

**10.** A pharmaceutical preparation according to claim 9 in dosage unit form.

**11.** A pharmaceutical preparation according to claims 9-10 comprising the active ingredient in association with a pharmaceutically acceptable carrier.

**12.** A compound according to any of claims 1-7 for use as a therapeutically active substance.

**13.** Use of a compound according to any of claims 1-7 for the preparation of medicaments with immunomodulating action.

**14.** Compounds according to claims 1-7 for use in a method for the treatment of diseases due to defects in the immune system in mammals including man.

**Claims for the following Contracting States : ES, GR**

**1.** A process for the preparation of a compound of the general formula I

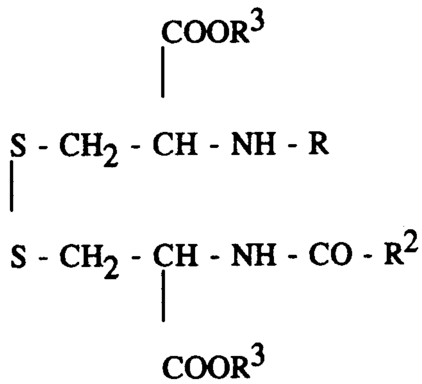

I

wherein R is hydrogen or a moiety -CO-$R^1$ wherein $R^1$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, $R^2$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl and $R^3$ is hydrogen, methyl, ethyl, propyl, isopropyl, butyl or isobutyl, provided that $R^1$ and $R^2$ are not simultaneously methyl and further provided that when $R^3$ is hydrogen $R^1$ and $R^2$ are not simultaneously n-propyl, n-heptyl, n-nonyl or n-undecyl and further provided that when R and $R^3$ are all hydrogen, then $R^2$ is not n-nonyl or a physiologically acceptable salt and/or a stereochemical isomer thereof, characterized by

a) the oxidation of an N-acylcysteine derivative of the formula

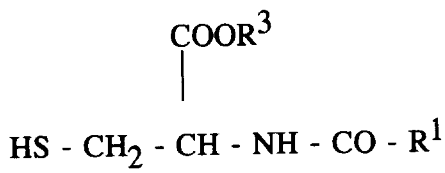

wherein $R^1$ and $R^3$ are as defined above or when $R^3$ is hydrogen optionally an alkali salt thereof, to the formation of a compound of the formula

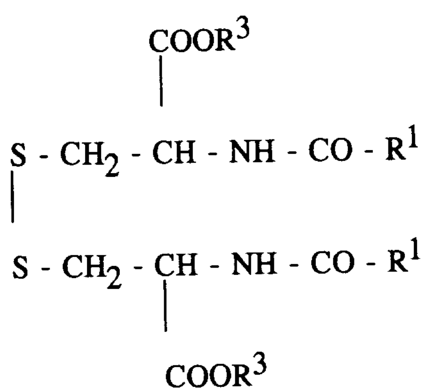

or

b) the reaction, in the presence of a suitable base, of a compound having the formula

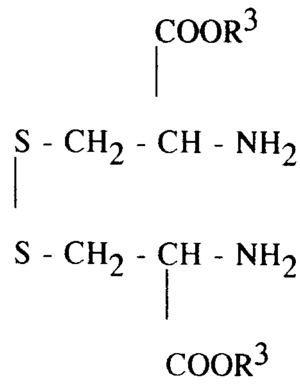

or a salt thereof wherein $R^3$ is as defined above, with a compound of the formula

$R^1$ - COX

wherein $R^1$ is defined as above and COX is a reactive group capable of reacting with an amino group under formation of an amide moiety, to the formation of a compound of the formula

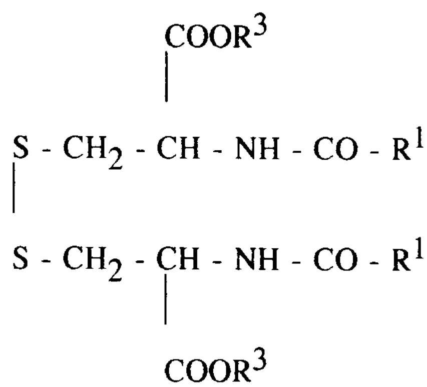

or

c) the reaction of 2-(N-acylamino)-3-halopropionic acid derivative of the formula

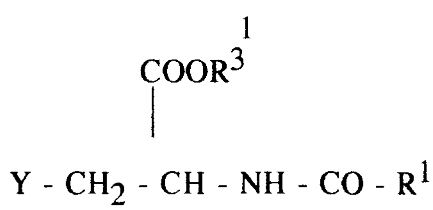

wherein $R^1$ is as defined above

$$R^{3^{1}}$$

is $R^3$ as defined above or an acid or base labile organic group and Y is a halogen atom, with sulphur

or disulphide dianion in the presence of a base, to the formation of a compound of the formula

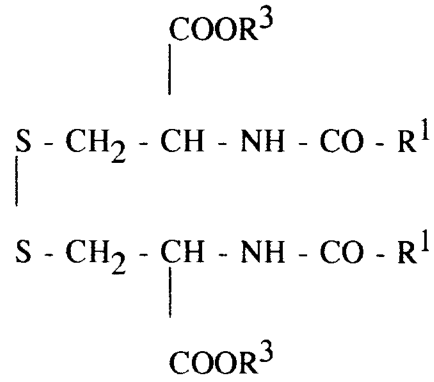

or

d) the oxidation of a mixture of N-acylcysteine ester derivatives of the formulas

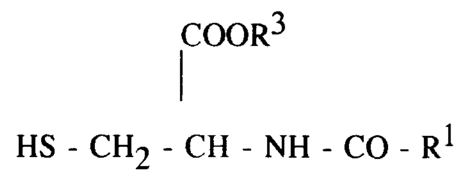

and

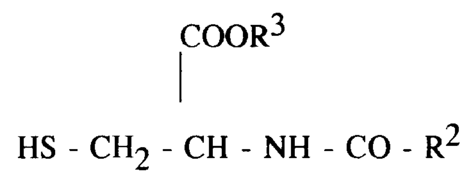

wherein $R^1$, $R^2$ and $R^3$ are as defined above or when $R^3$ is hydrogen optionally alkali salts thereof, to the formation of a compound of the formula

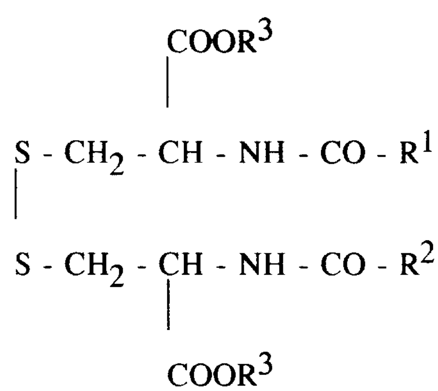

or

e) the oxidation of a mixture of cysteine or a cysteine ester and an N-acylcysteine derivative of the formulas

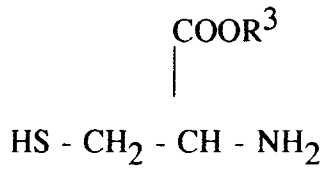

or a salt thereof and

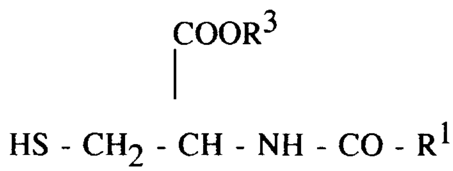

or an alkali or hydrochloride salts thereof, wherein $R^1$ and $R^3$ are as defined above, to the formation of a compound of the formula

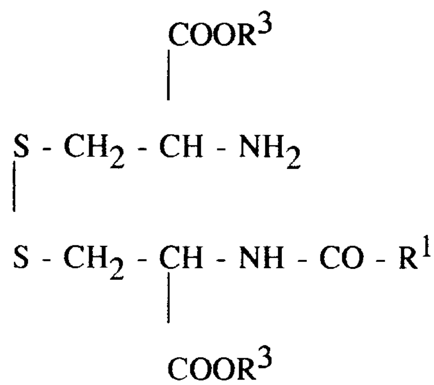

or a salt thereof,
or
f) the reaction, in the presence of a suitable base, of an excess of cysteine or a cystine diester having the formula

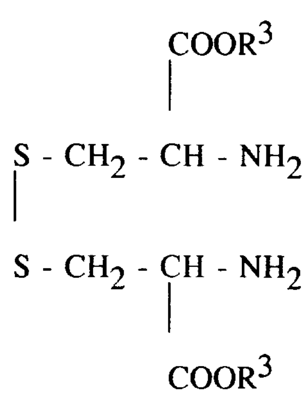

or a salt thereof wherein $R^3$ is as defined above, with a compound of the formula

$R^2$ - COX

wherein $R^2$ and COX are as defined above, to the formation of a compound of the formula

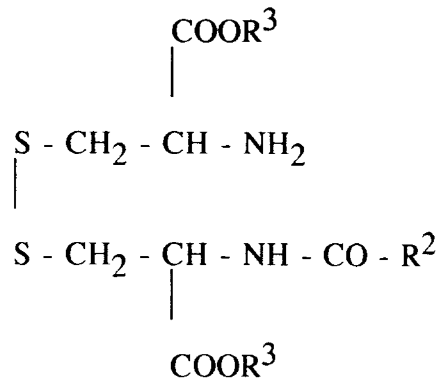

or

g) the reaction, in the presence of a suitable base, of a compound of the formula

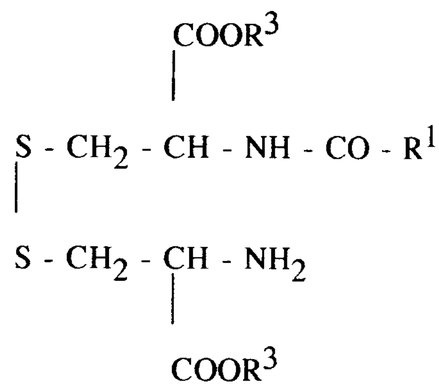

or a salt thereof wherein $R^1$ and $R^3$ are as defined above, with a compound of the formula

$R^2$ - COX

wherein $R^2$ and COX are as defined above, to the formation of a compound of the formula

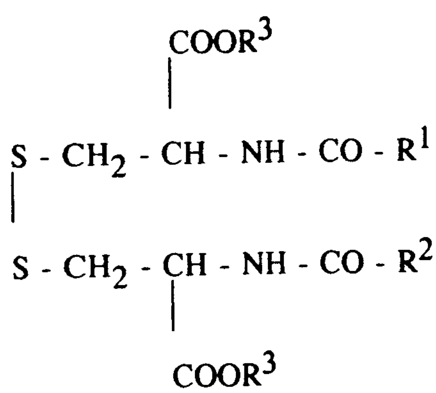

h) the reaction of an N-acylcysteine derivative of the formula

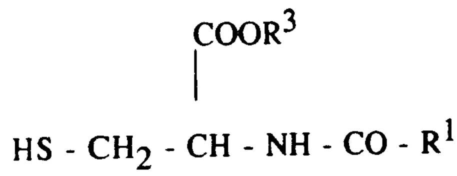

or a salt thereof,
wherein $R^1$ and $R^3$ are as defined above, with an activating reagent to the formation of an adduct of the formula

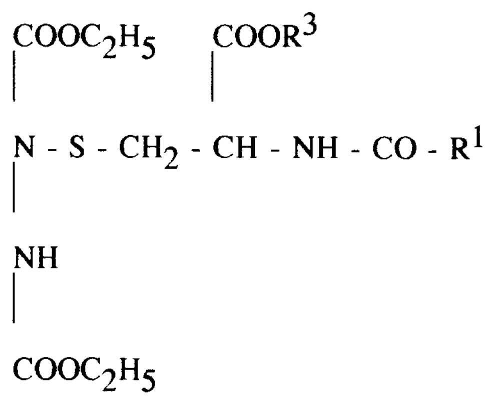

followed by reaction with a second, different or the same N-acylcysteine or cysteine ester derivative, to give a compound of the formula

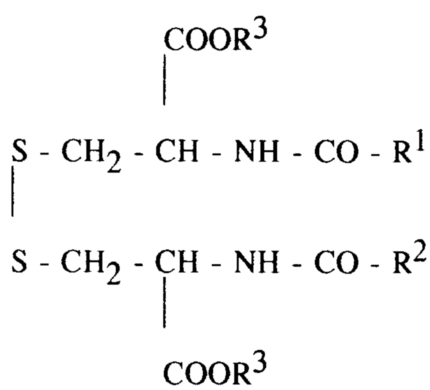

i) the reaction of a compound of the formula

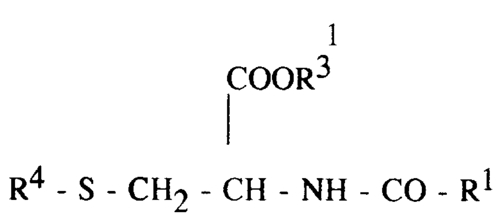

wherein $R^4$ is -Cl or

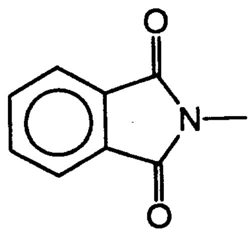

,

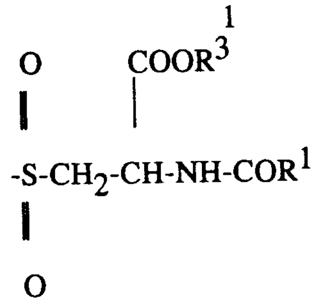

and $R^1$ and

$$R^{3^{1}}$$

are as defined above, with a compound of the formula

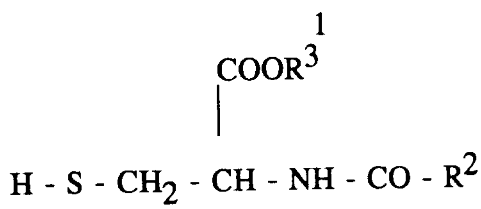

wherein $R^2$ and

$$R^{3^{1}}$$

are as defined above to the formation of a compound of the formula

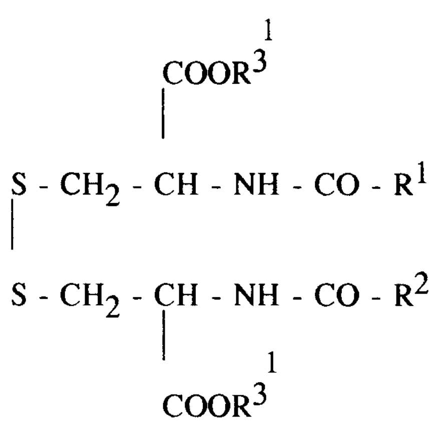

whereafter, if a compound wherein $R^3$ = H is desired, removal of the protecting group

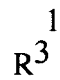 ,

or

j) the esterification of a compound of the formula

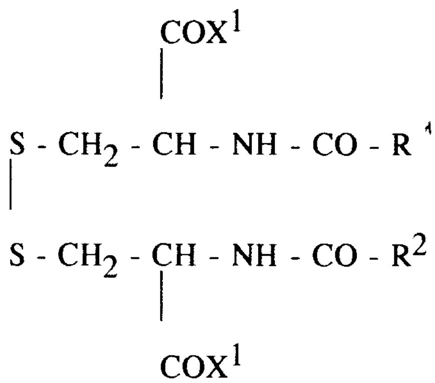

wherein $R^1$ and $R^2$ are as defined above, and $X^1$ is OH or a halogen atom with a compound of the formula

$R^{3E}$-OH

wherein $R^{3E}$ is methyl, ethyl, propyl, isopropyl, butyl or isobutyl, to the formation of a compound of the formula

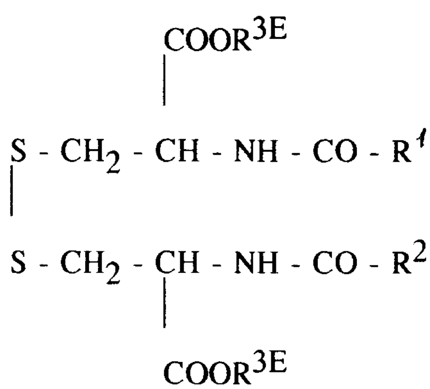

or

k) the alkylation of a compound of the formula

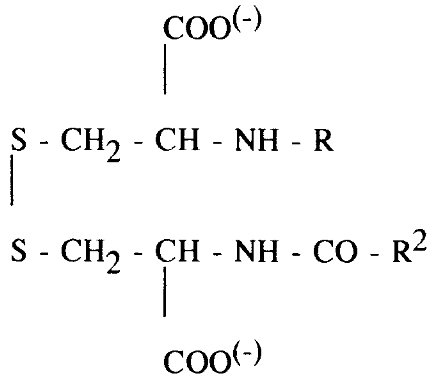

wherein R and $R^2$ are as defined above, with a compound of the formula

$R^{3E}$-Z

wherein $R^{3E}$ is as defined above and Z is halogen, alkylsulphate, tosylate or another nucleofuge to the formation of a compound of the formula

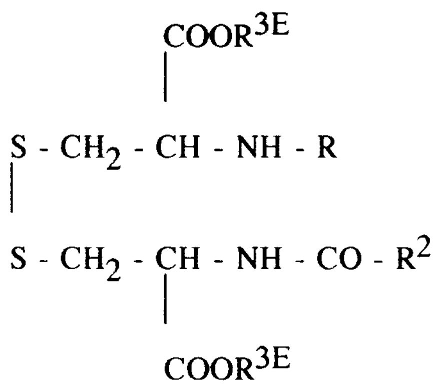

l) the reaction, in the presence of a suitable base, of a carboxyl protected cystine derivative of the formula

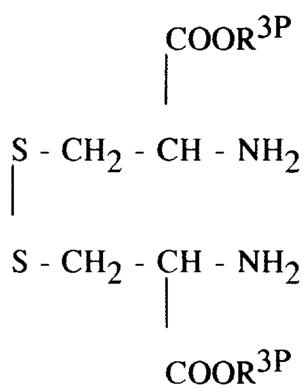

or a hydrochloride salt thereof, wherein $R^{3P}$ is an acid or base labile organic group, with a compound of the formula

$R^1$-COX

wherein $R^1$ is defined as above and COX is a reactive group capable of reacting with an amino group under formation of an amide moiety, and thereafter removal of the protecting group $R^{3P}$ to the formation of a compound of the formula

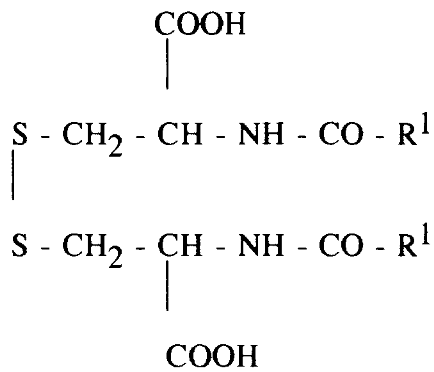

m) the reaction, under alkaline conditions, of a carboxyl protected cystine derivative of the formula

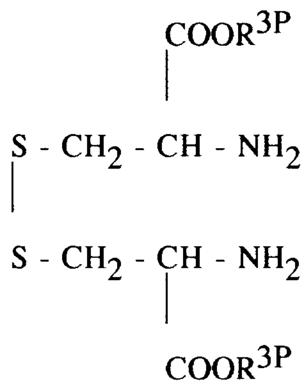

or a hydrochloride salt thereof, wherein $R^{3P}$ is as defined above, with a compound of the formula

$R^2$ - COX

wherein $R^2$ and COX are as defined above and thereafter removal of the protecting group $R^{3P}$ to the formation of a compound of the formula

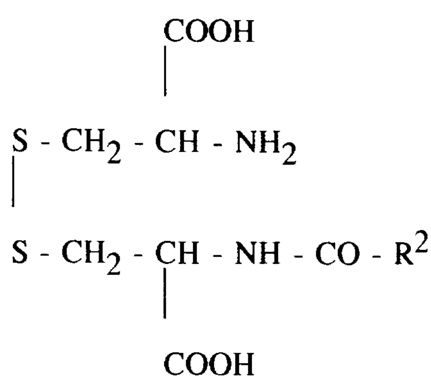

n) the reaction of a carboxyl protected cystine derivative of the formula

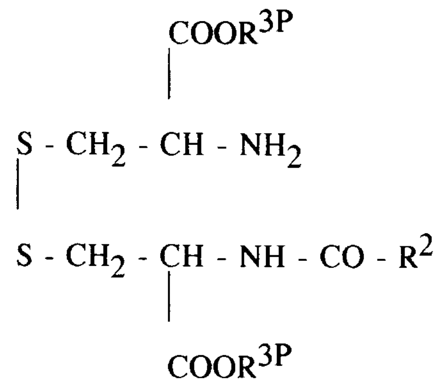

or a hydrochloride salt thereof, wherein $R^2$ and $R^{3P}$ are as defined above, with a compound of the formula

$R^1$ - COX

wherein $R^1$ and COX are as defined above and thereafter removal of the protecting group $R^{3P}$ to the formation of a compound of the formula

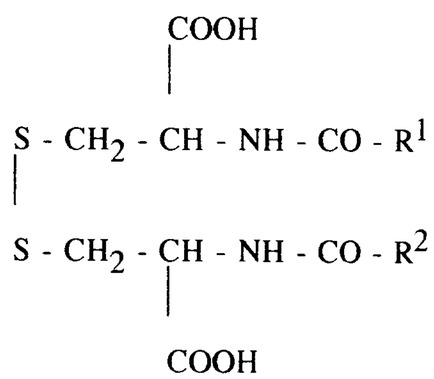

o) the equilibration, in alkaline aqueous solution of a mixture of a cystine derivative and a cysteine derivative having the formulas

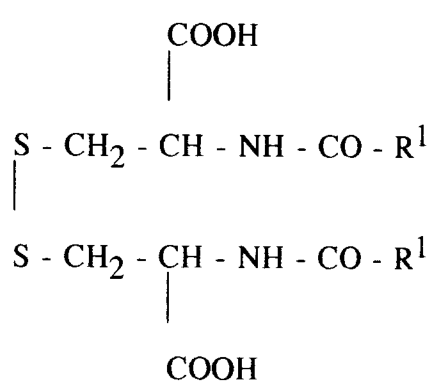

and

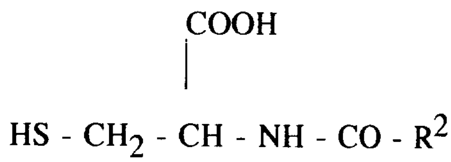

or alkali salts thereof, wherein $R^1$ and $R^2$ are as defined above, to the formation of a compound of the formula

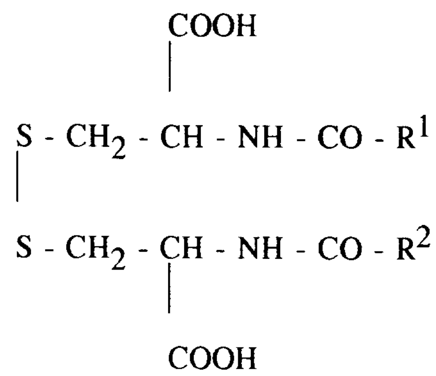

and finally, if desired, transferal of the compounds obtained by any of the methods a) - o) into a physiologically acceptable salt.

**2.** A process according to claim 1, characterized in the preparation of a compound of the formula I wherein R is hydrogen or a moiety -CO-$R^1$ wherein $R^1$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, $R^2$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl and $R^3$ is hydrogen, methyl or ethyl, provided that $R^1$ and $R^2$ are not simultaneously methyl and further provided that when $R^3$ is hydrogen $R^1$ and $R^2$ are not simultaneously n-propyl, n-heptyl, n-nonyl or n-undecyl and further provided that when R and $R^3$ are all hydrogen, then $R^2$ is not n-nonyl.

**3.** A process according to claim 1, characterized in the preparation of a compound of the formula I wherein $R^1$ and $R^2$ are isopropyl and $R^3$ is methyl.

**4.** A process according to claim 1, characterized in the preparation of a compound of the formula I wherein $R^1$ and $R^2$ are n-pentyl and $R^3$ is methyl.

**5.** A process according to claim 1, characterized in the preparation of a compound of the formula I wherein $R^1$ and $R^2$ are n-heptyl and $R^3$ is methyl or ethyl.

**6.** A process according to claim 1, characterized in the preparation of a compound of the formula I wherein $R^1$ and $R^2$ are simultaneously isopropyl and $R^3$ is hydrogen.

**7.** A process according to claim 1, characterized in the preparation of a compound of the formula I wherein $R^1$ and $R^2$ are simultaneously tert. butyl and $R^3$ is hydrogen.

8. Use of a compound of the general formula

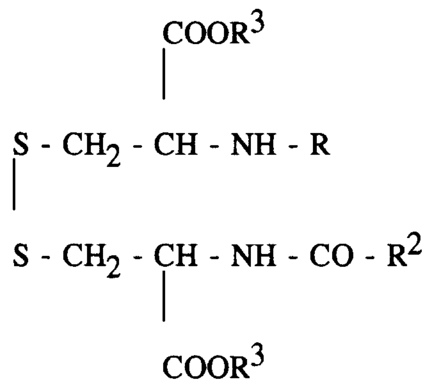

wherein R is hydrogen or a moiety -CO-$R^1$ wherein $R^1$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl, $R^2$ is methyl, ethyl, n-propyl, n-butyl, n-pentyl, n-hexyl, n-heptyl, n-octyl, n-nonyl, n-decyl, n-undecyl, iso-propyl, 1-methylpropyl, tert. butyl, 3-methylbutyl or 2-methylbutyl and $R^3$ is hydrogen, methyl, ethyl, propyl, isopropyl, butyl or isobutyl, provided that $R^1$ and $R^2$ are not simultaneously methyl and further provided that when $R^3$ is hydrogen $R^1$ and $R^2$ are not simultaneously n-propyl, n-heptyl, n-nonyl or n-undecyl and further provided that when R and $R^3$ are all hydrogen, then $R^2$ is not n-nonyl or a physiologically acceptable salt and/or a stereochemical isomer thereof, for the preparation of medicaments with immunomodulating action.

**Patentansprüche**
**Patentansprüche für folgende Vertragsstaaten : AT, BE, CH, DE, DK, FR, GB, IT, LI, LU, NL, SE**

1. Verbindungen der allgemeinen Formel

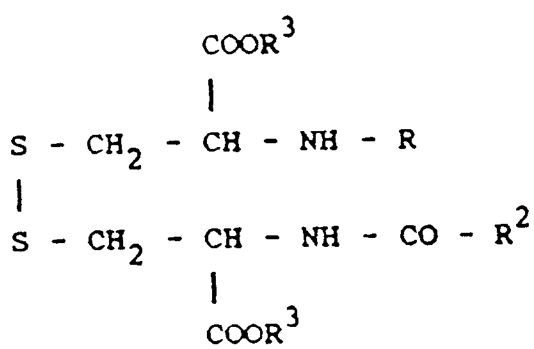

(I),

worin R Wasserstoff oder eine Gruppe -CO-$R^1$ bedeutet, wobei $R^1$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl darstellt; $R^2$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl ist; und $R^3$ Wasserstoff, Methyl, Ethyl, Propyl, Isopropyl, Butyl oder Isobutyl bedeutet; mit der Maßgabe, daß $R^1$ und $R^2$ nicht gleichzeitig Methyl darstellen, und ferner mit der Maßgabe, daß, wenn $R^3$ Wasserstoff ist, $R^1$ und $R^2$ nicht gleichzeitig n-Propyl, n-Heptyl, n-Nonyl oder n-Undecyl bedeuten, und ferner mit der Maßgabe, daß, wenn R und $R^3$ alle Wasserstoff darstellen, $R^2$ nicht n-Nonyl ist; oder ein physiologisch annehmbares Salz und/oder ein stereochemisches Isomer hievon.

2. Verbindung nach Anspruch 1, worin R Wasserstoff oder eine Gruppe -CO-$R^1$ bedeutet, wobei $R^1$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl darstellt; $R^2$ Methyl, Ethyl, n-

Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl ist; und $R^3$ Wasserstoff, Methyl oder Ethyl bedeutet; mit der Maßgabe, daß $R^1$ und $R^2$ nicht gleichzeitig Methyl darstellen, und ferner mit der Maßgabe, daß, wenn $R^3$ Wasserstoff ist, $R^1$ und $R^2$ nicht gleichzeitig n-Propyl, n-Heptyl, n-Nonyl oder n-Undecyl bedeuten, und ferner mit der Maßgabe, daß, wenn R und $R^3$ alle Wasserstoff darstellen, $R^2$ nicht n-Nonyl ist.

**3.** Verbindung nach Anspruch 1, worin $R^1$ und $R^2$ Isopropyl bedeuten, und $R^3$ Methyl darstellt.

**4.** Verbindung nach Anspruch 1, worin $R^1$ und $R^2$ n-Pentyl bedeuten, und $R^3$ Methyl darstellt.

**5.** Verbindung nach Anspruch 1, worin $R^1$ und $R^2$ n-Heptyl bedeuten, und $R^3$ Methyl oder Ethyl darstellt.

**6.** Verbindung nach Anspruch 1, worin $R^1$ und $R^2$ gleichzeitig Isopropyl bedeuten, und $R^3$ Wasserstoff ist.

**7.** Verbindung nach Anspruch 1, worin $R^1$ und $R^2$ gleichzeitig tert.Butyl bedeuten, und $R^3$ Wasserstoff ist.

**8.** Verfahren zur Herstellung einer Verbindung der allgemeinen Formel (I), wie in Anspruch 1 definiert, gekennzeichnet durch

a) die Oxidation eines N-Acylcystein-Derivats der Formel

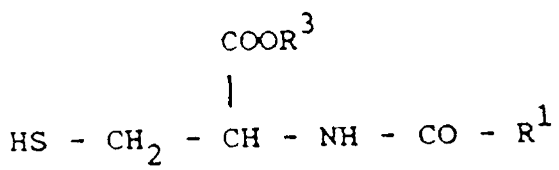

,

worin $R^1$ und $R^3$ wie oben definiert sind, oder, wenn $R^3$ Wasserstoff bedeutet, gegebenenfalls eines Alkalisalzes hievon, zur Bildung einer Verbindung der Formel

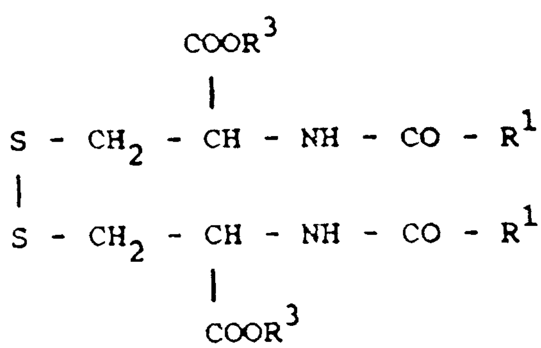

,

oder

b) das Umsetzen, in Anwesenheit einer geeigneten Base, einer Verbindung der Formel

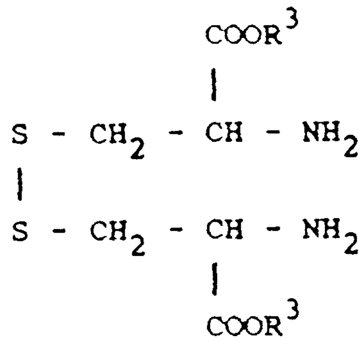

oder eines Salzes hievon, worin $R^3$ wie oben definiert ist, mit einer Verbindung der Formel

$R^1$ - COX,

worin $R^1$ wie oben definiert ist, und COX eine reaktive Gruppe, die mit einer Amino-Gruppe unter Bildung einer Amid-Gruppe reagieren kann, bedeutet, zur Bildung einer Verbindung der Formel

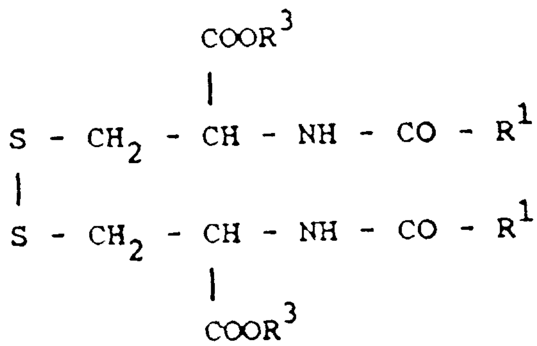

,

oder

c) das Umsetzen eines 2-(N-Acylamino)-3-halogenpropionsäure-Derivats der Formel

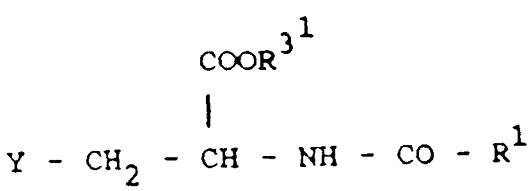

,

worin $R^1$ wie oben definiert ist,

$R^{3^1}$

die Bedeutung $R^3$, wie oben definiert, oder einer Säure- oder Basen-labilen organischen Gruppe hat, und Y ein Halogenatom darstellt, mit Schwefel oder einem Disulfiddianion in Anwesenheit einer Base, zur Bildung einer Verbindung der Formel

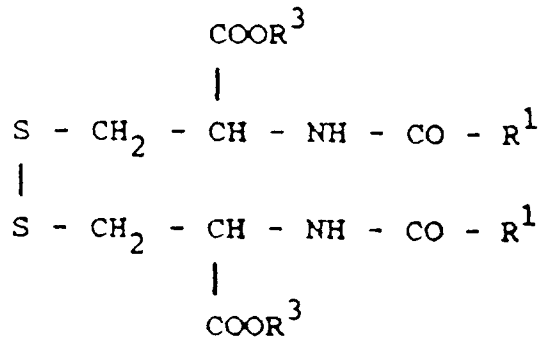

,

oder

d) die Oxidation einer Mischung von N-Acylcysteinester-Derivaten der Formeln

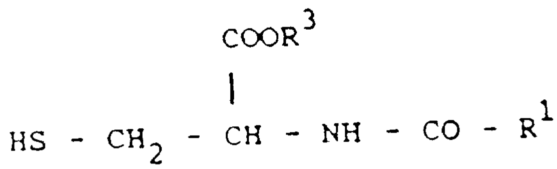

und

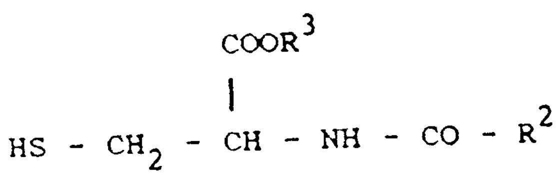

,

worin $R^1$, $R^2$ und $R^3$ wie oben definiert sind, oder, wenn $R^3$ Wasserstoff bedeutet, gegebenenfalls der Alkalisalze hievon, zur Bildung einer Verbindung der Formel

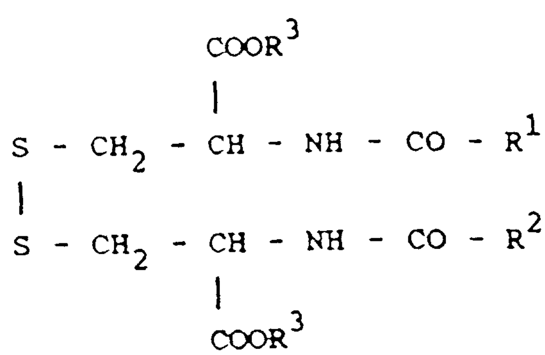

,

oder

e) die Oxidation einer Mischung von Cystein oder eines Cysteinesters und eines N-Acylcystein-Derivats der Formeln

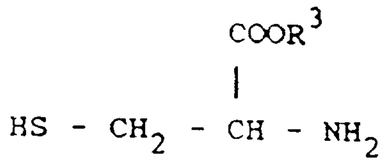

oder eines Salzes hievon und

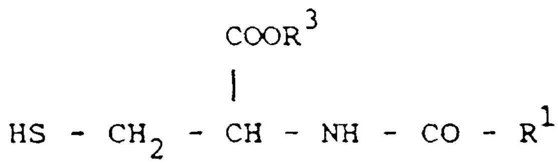

oder eines Alkali- oder Hydrochloridsalzes hievon, worin $R^1$ und $R^3$ wie oben definiert sind, zur Bildung einer Verbindung der Formel

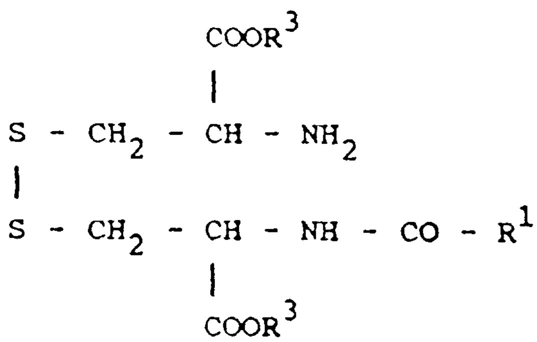

oder eines Salzes hievon, oder

f) das Umsetzen, in Anwesenheit einer geeigneten Base, eines Überschusses an Cystein oder eines Cystindiesters der Formel

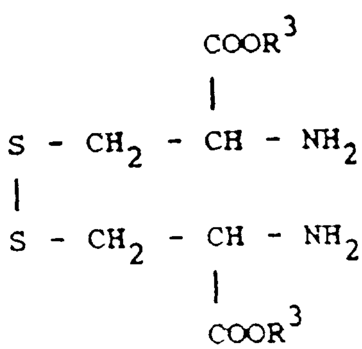

oder eines Salzes hievon, worin $R^3$ wie oben definiert ist, mit einer Verbindung der Formel

$R^2$ - COX,

worin $R^2$ und COX wie oben definiert sind, zur Bildung einer Verbindung der Formel

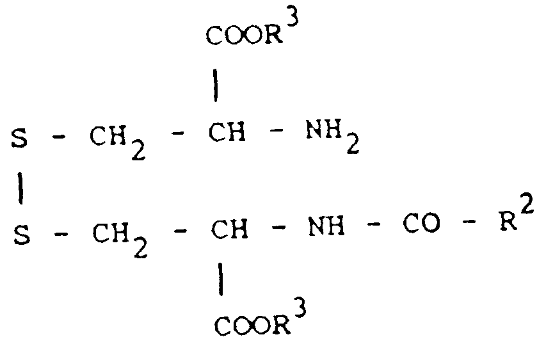

,

oder

g) das Umsetzen, in Anwesenheit einer geeigneten Base, einer Verbindung der Formel

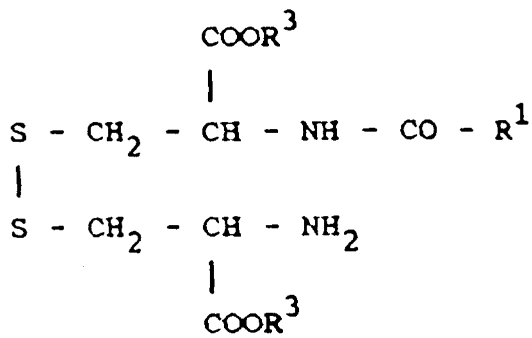

oder eines Salzes hievon, worin $R^1$ und $R^3$ wie oben definiert sind, mit einer Verbindung der Formel

$R^2$ - COX,

worin $R^2$ und COX wie oben definiert sind, zur Bildung einer Verbindung der Formel

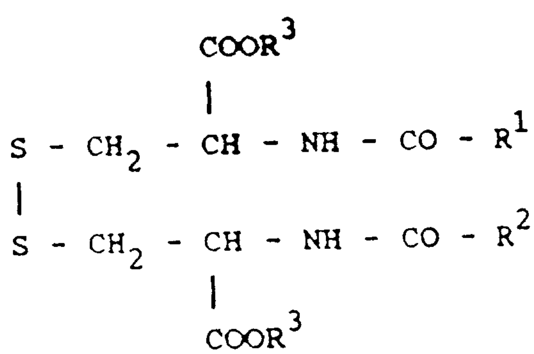

,

h) das Umsetzen eines N-Acylcystein-Derivats der Formel

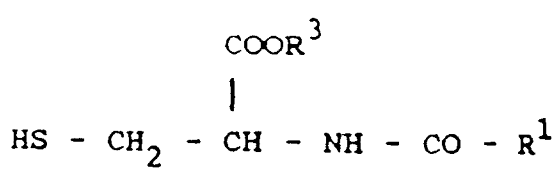

oder eines Salzes hievon, worin $R^1$ und $R^3$ wie oben definiert sind, mit einem Aktivierungsreagens,

zur Bildung eines Addukts der Formel

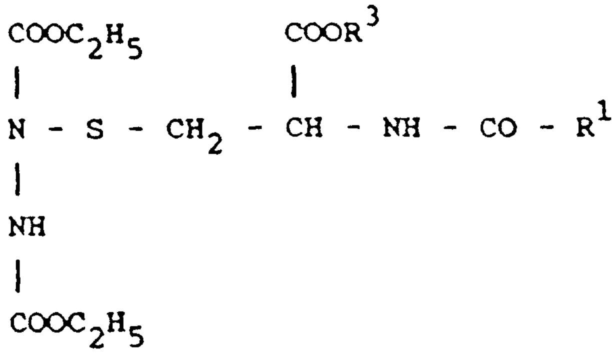

,

gefolgt vom Umsetzen mit einem zweiten, anderen oder gleichen N-Acylcystein oder Cysteinester-Derivat, wobei eine Verbindung der Formel

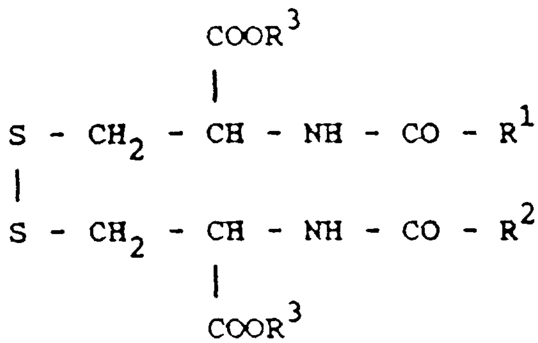

erhalten wird,
i) das Umsetzen einer Verbindung der Formel

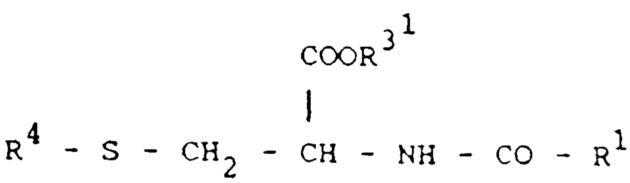

,

worin $R^4$ die Bedeutung -Cl oder

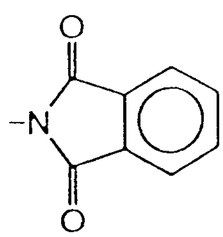

,

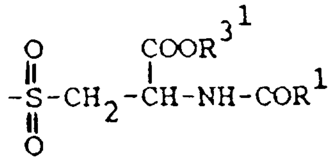

hat, und $R^1$ und

$R^{31}$

wie oben definiert sind, mit einer Verbindung der Formel

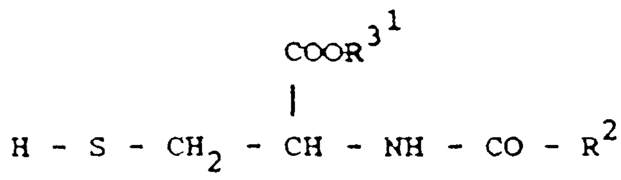

,

worin $R^2$ und

$R^{31}$

wie oben definiert sind, zur Bildung einer Verbindung der Formel

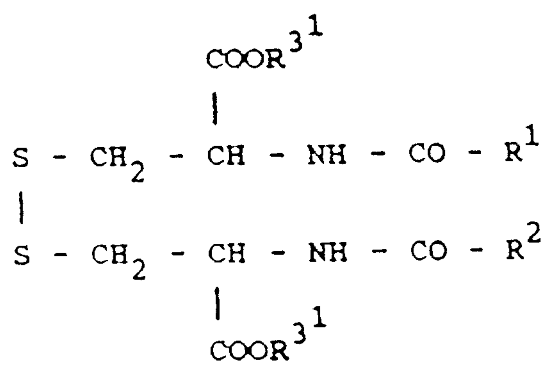

,

wonach, wenn eine Verbindung, worin $R^3$ = H, gewünscht wird, die Schutzgruppe

$R^{31}$

entfernt wird, oder

j) die Veresterung einer Verbindung der Formel

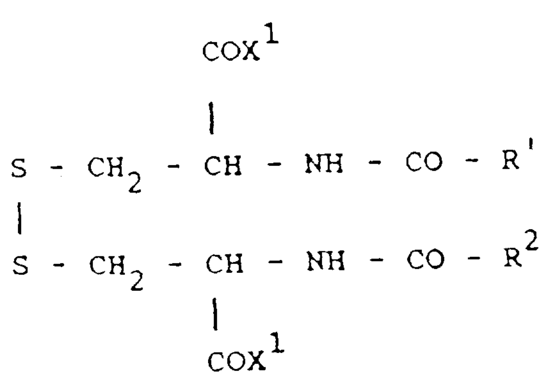

,

worin $R^1$ und $R^2$ wie oben definiert sind, und $X^1$ die Bedeutung OH oder eines Halogenatoms hat, mit einer Verbindung der Formel

$R^{3E}$-OH,

worin $R^{3E}$ Methyl, Ethyl, Propyl, Isopropyl, Butyl oder Isobutyl ist, zur Bildung einer Verbindung der Formel

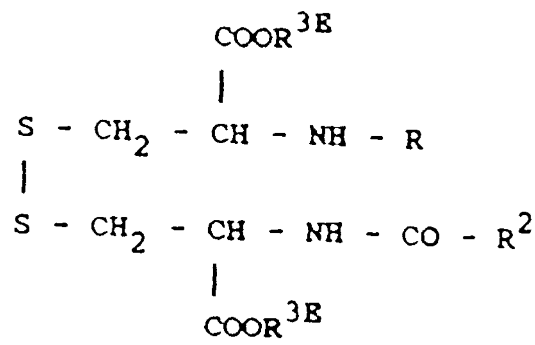

,

oder

k) die Alkylierung einer Verbindung der Formel

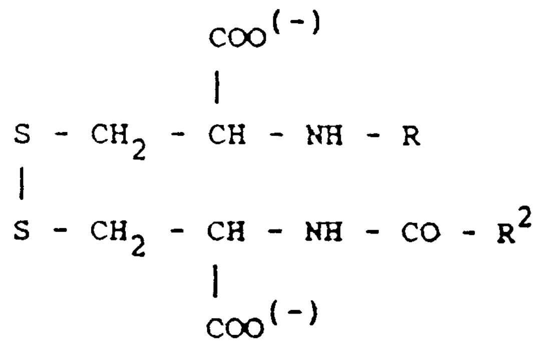

,

worin R und $R^2$ wie oben definiert sind, mit einer Verbindung der Formel

$R^{3E}$-Z,

worin $R^{3E}$ wie oben definiert ist, und Z Halogen, Alkylsulfat, Tosylat oder einen anderen Nucelofug darstellt, zur Bildung einer Verbindung der Formel

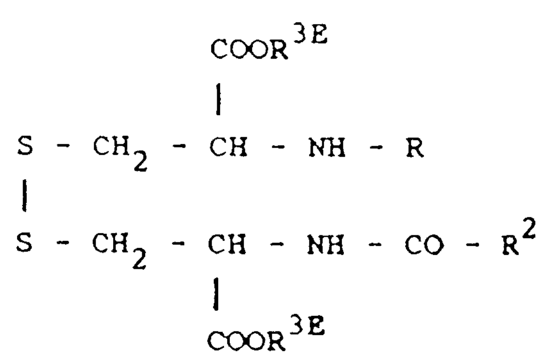

,

l) das Umsetzen, in Anwesenheit einer geeigneten Base, eines Carboxyl-geschützten Cystin-Derivats der Formel

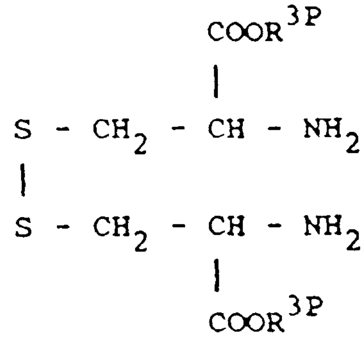

oder eines Hydrochloridsalzes hievon, worin $R^{3P}$ eine Säure- oder Basen-labile organische Gruppe bedeutet, mit einer Verbindung der Formel

$R^1$ - COX,

worin $R^1$ wie oben definiert ist, und COX eine reaktive Gruppe, die mit einer Amino-Gruppe unter Bildung einer Amid-Gruppe reagieren kann, darstellt, und danach Entfernung der Schutzgruppe $R^{3P}$, zur Bildung einer Verbindung der Formel

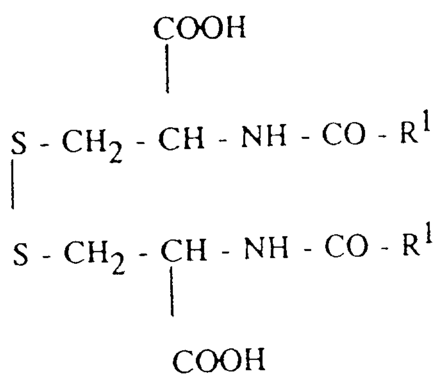

,

m) das Umsetzen, unter alkalischen Bedingungen, eines Carboxyl-geschützten Cystin-Derivats der Formel

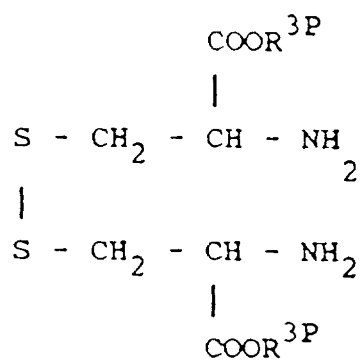

oder eines Hydrochloridsalzes hievon, worin $R^{3P}$ wie oben definiert ist, mit einer Verbindung der Formel

$R^2$ - COX,

worin $R^2$ und COX wie oben definiert sind, und danach Entfernung der Schutzgruppe $R^{3P}$, zur Bildung einer Verbindung der Formel

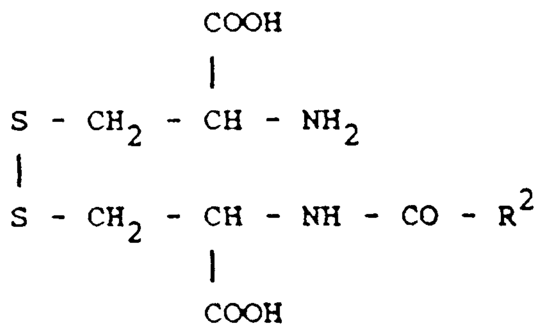

,

n) das Umsetzen eines Carboxyl-geschützten Cystin-Derivats der Formel

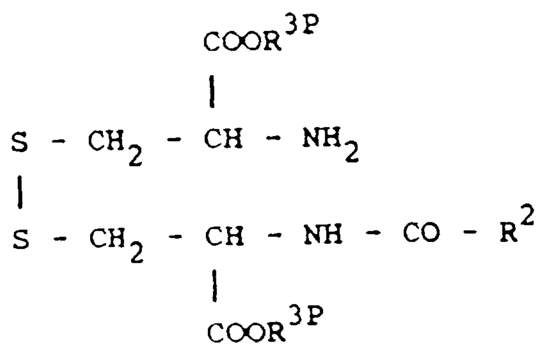

oder eines Hydrochloridsalzes hievon, worin $R^2$ und $R^{3P}$ wie oben definiert sind, mit einer Verbindung der Formel

$R^1$ - COX,

worin $R^1$ und COX wie oben definiert sind, und danach Entfernung der Schutzgruppe $R^{3P}$, zur Bildung einer Verbindung der Formel

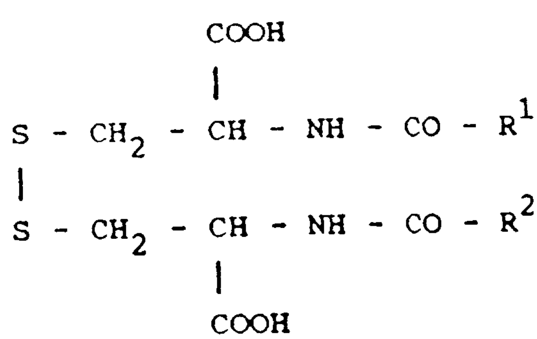

,

o) die Äquilibrierung, in alkalischer wässeriger Lösung, einer Mischung eines Cystin-Derivats und eines Cystein-Derivats der Formeln

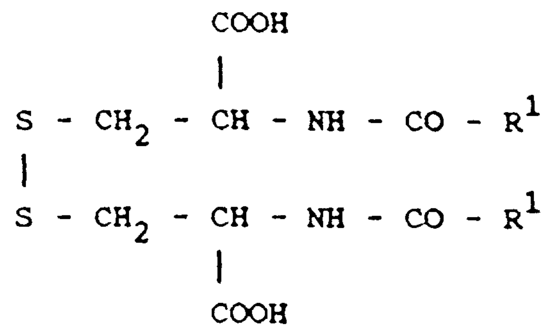

und

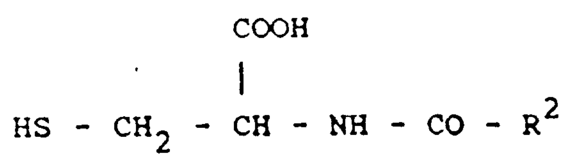

oder von Alkalisalzen hievon, worin $R^1$ und $R^2$ wie oben definiert sind, zur Bildung einer Verbindung der Formel

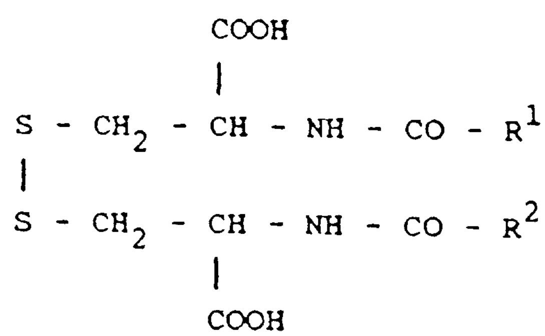

,

und schließlich, wenn gewünscht, Transfer der durch eine der Methoden a) bis o) erhaltenen Verbindungen in ein physiologisch annehmbares Salz.

**9.** Pharmazeutische Zubereitung, welche als aktiven Bestandteil eine Verbindung nach einem der Ansprüche 1 bis 7 umfaßt.

**10.** Pharmazeutische Zubereitung nach Anspruch 9 in Einheitsdosierungsform.

**11.** Pharmazeutische Zubereitung nach Anspruch 9 oder 10, welche den aktiven Bestandteil in Vereinigung mit einem pharmazeutisch annehmbaren Träger umfaßt.

**12.** Verbindung nach einem der Ansprüche 1 bis 7 zur Verwendung als therapeutisch wirksame Substanz.

**13.** Verwendung einer Verbindung nach einem der Ansprüche 1 bis 7 zur Herstellung von Medikamenten mit Immunmodulationswirkung.

**14.** Verbindungen nach einem der Ansprüche 1 bis 7 zur Verwendung in einem Verfahren zur Behandlung von Erkrankungen aufgrund von Defekten im Immunsystem von Säugern, einschließlich Menschen.

**Patentansprüche für folgende Vertragsstaaten : ES, GR**

**1.** Verfahren zur Herstellung einer Verbindung der allgemeinen Formel (I)

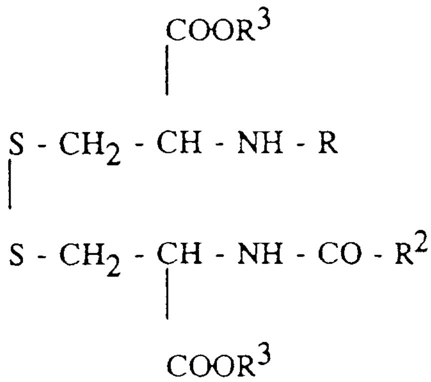

(I),

worin R Wasserstoff oder eine Gruppe -CO-$R^1$ bedeutet, wobei $R^1$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl darstellt; $R^2$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl ist; und $R^3$ Wasserstoff, Methyl, Ethyl, Propyl, Isopropyl, Butyl oder Isobutyl bedeutet; mit der Maßgabe, daß $R^1$ und $R^2$ nicht gleichzeitig Methyl darstellen, und ferner mit der Maßgabe, daß, wenn $R^3$ Wasserstoff ist, $R^1$ und $R^2$ nicht gleichzeitig n-Propyl, n-Heptyl, n-Nonyl oder n-Undecyl bedeuten, und ferner mit der Maßgabe, daß, wenn R und $R^3$ alle Wasserstoff darstellen, $R^2$ nicht n-Nonyl ist; oder eines physiologisch annehmbaren Salzes und/oder eines stereochemischen Isomers hievon, gekennzeichnet durch

a) die Oxidation eines N-Acylcystein-Derivats der Formel

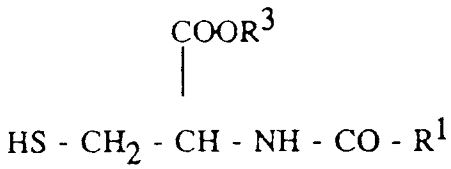

,

worin $R^1$ und $R^3$ wie oben definiert sind, oder, wenn $R^3$ Wasserstoff bedeutet, gegebenenfalls eines Alkalisalzes hievon, zur Bildung einer Verbindung der Formel

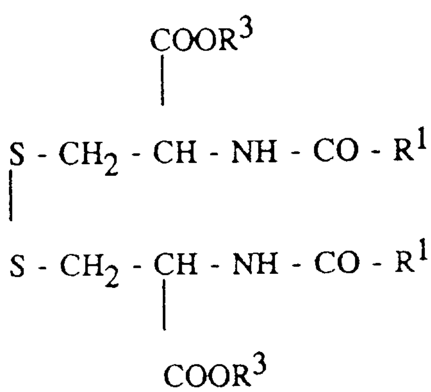

,

oder

b) das Umsetzen, in Anwesenheit einer geeigneten Base, einer Verbindung der Formel

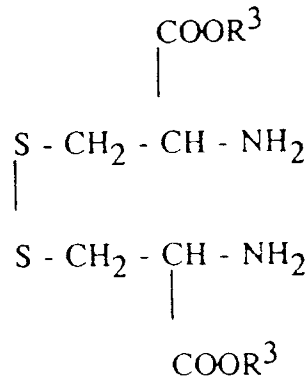

oder eines Salzes hievon, worin $R^3$ wie oben definiert ist, mit einer Verbindung der Formel

$R^1$ - COX,

worin $R^1$ wie oben definiert ist, und COX eine reaktive Gruppe, die mit einer Amino-Gruppe unter Bildung einer Amid-Gruppe reagieren kann, bedeutet, zur Bildung einer Verbindung der Formel

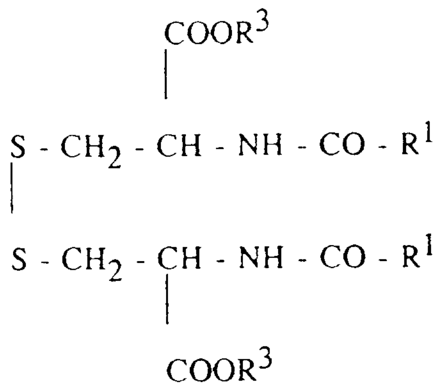

,

oder

c) das Umsetzen eines 2-(N-Acylamino)-3-halogenpropionsäure-Derivats der Formel

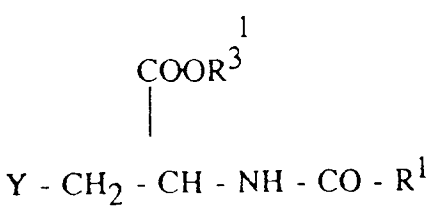

,

worin $R^1$ wie oben definiert ist,

$R^{3 1}$

die Bedeutung $R^3$, wie oben definiert, oder einer Säure- oder Basen-labilen organischen Gruppe hat, und Y ein Halogenatom darstellt, mit Schwefel oder einem Disulfiddianion in Anwesenheit einer Base, zur Bildung einer Verbindung der Formel

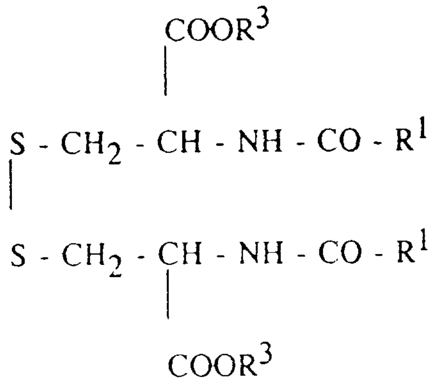

,

oder

d) die Oxidation einer Mischung von N-Acylcysteinester-Derivaten der Formeln

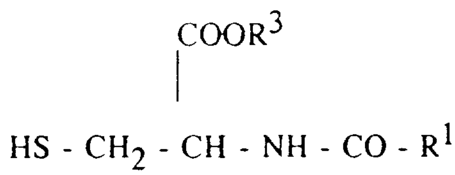

und

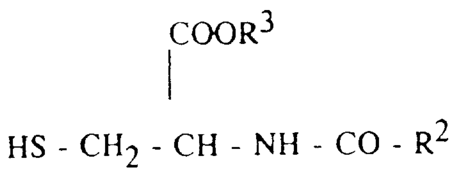

,

worin $R^1$, $R^2$ und $R^3$ wie oben definiert sind, oder, wenn $R^3$ Wasserstoff bedeutet, gegebenenfalls der Alkalisalze hievon, zur Bildung einer Verbindung der Formel

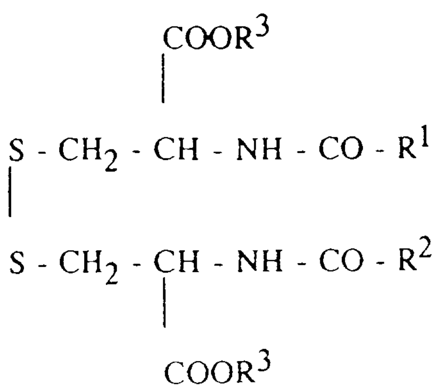

,

oder

e) die Oxidation einer Mischung von Cystein oder eines Cysteinesters und eines N-Acylcystein-Derivats der Formeln

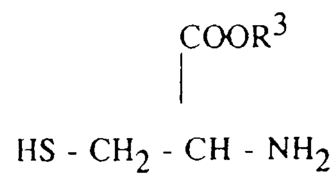

oder eines Salzes hievon und

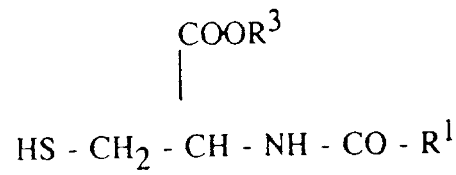

oder eines Alkali- oder Hydrochloridsalzes hievon, worin $R^1$ und $R^3$ wie oben definiert sind, zur Bildung einer Verbindung der Formel

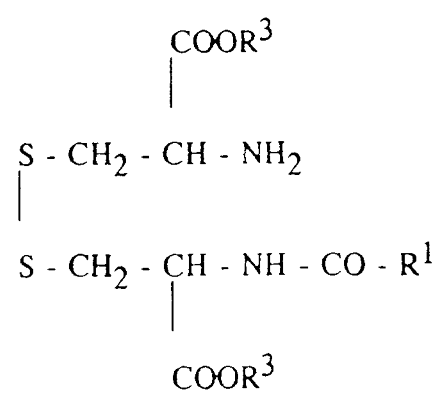

oder eines Salzes hievon, oder
f) das Umsetzen, in Anwesenheit einer geeigneten Base, eines Überschusses an Cystein oder eines Cystindiesters der Formel

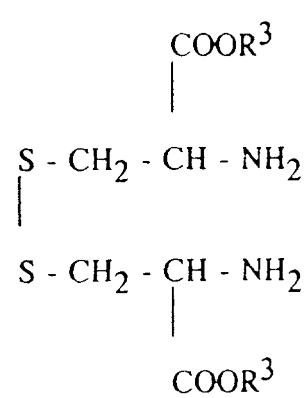

oder eines Salzes hievon, worin $R^3$ wie oben definiert ist, mit einer Verbindung der Formel

$R^2$ - COX,

worin $R^2$ und COX wie oben definiert sind, zur Bildung einer Verbindung der Formel

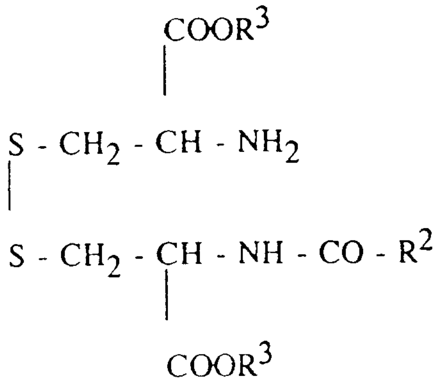

,

oder

g) das Umsetzen, in Anwesenheit einer geeigneten Base, einer Verbindung der Formel

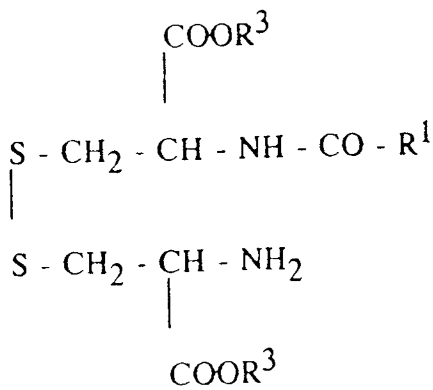

oder eines Salzes hievon, worin $R^1$ und $R^3$ wie oben definiert sind, mit einer Verbindung der Formel

$R^2$ - COX,

worin $R^2$ und COX wie oben definiert sind, zur Bildung einer Verbindung der Formel

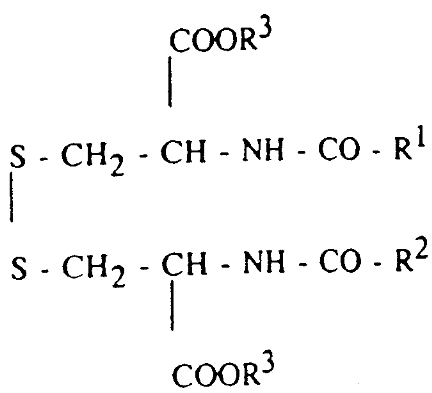

,

h) das Umsetzen eines N-Acylcystein-Derivats der Formel

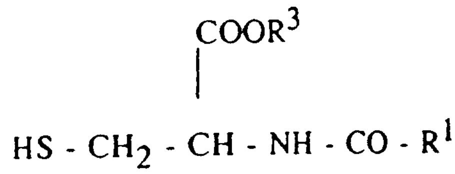

oder eines Salzes hievon, worin $R^1$ und $R^3$ wie oben definiert sind, mit einem Aktivierungsreagens, zur Bildung eines Addukts der Formel

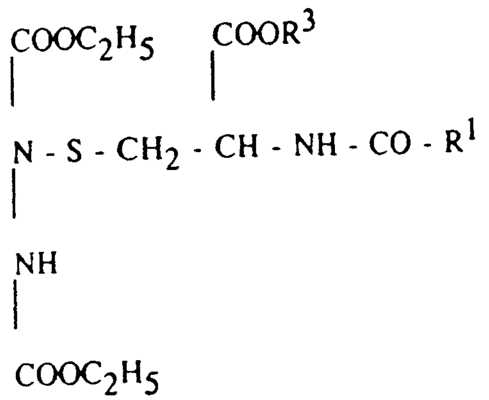

,

gefolgt vom Umsetzen mit einem zweiten, anderen oder gleichen N-Acylcystein oder Cysteinester-Derivat, wobei eine Verbindung der Formel

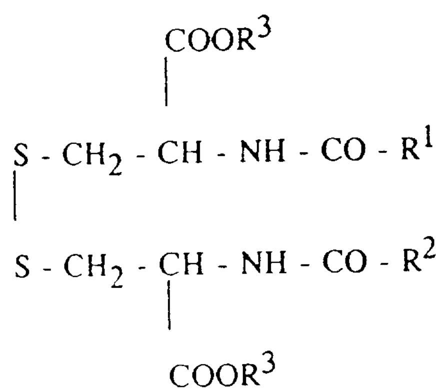

erhalten wird,

i) das Umsetzen einer Verbindung der Formel

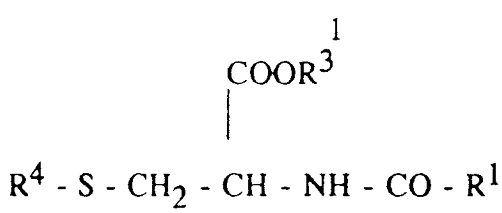

worin $R^4$ die Bedeutung -Cl oder

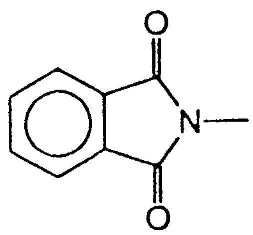 , 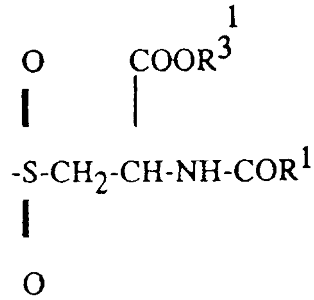

hat, und $R^1$ und

$R^{3^1}$

wie oben definiert sind, mit einer Verbindung der Formel

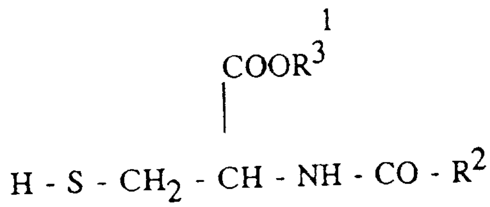 ,

worin $R^2$ und

$R^{3^1}$

wie oben definiert sind, zur Bildung einer Verbindung der Formel

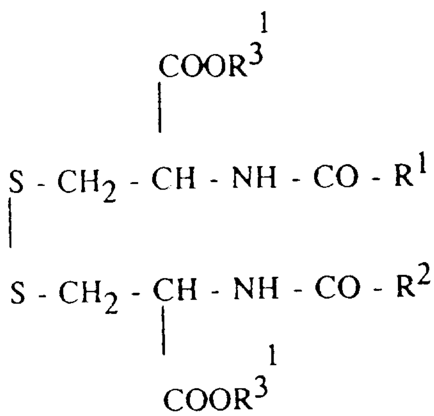 ,

wonach, wenn eine Verbindung, worin $R^3$ = H, gewünscht wird, die Schutzgruppe

$R^{3^1}$

entfernt wird, oder

j) die Veresterung einer Verbindung der Formel

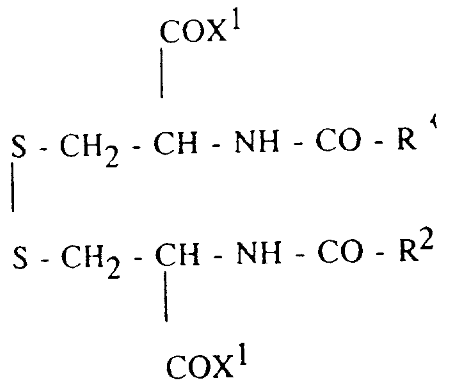

,

worin $R^1$ und $R^2$ wie oben definiert sind, und $X^1$ die Bedeutung OH oder eines Halogenatoms hat, mit einer Verbindung der Formel

$R^{3E}$-OH,

worin $R^{3E}$ Methyl, Ethyl, Propyl, Isopropyl, Butyl oder Isobutyl ist, zur Bildung einer Verbindung der Formel

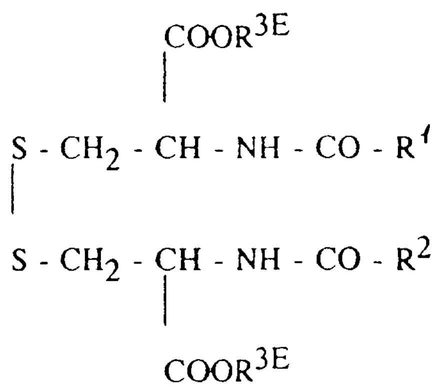

,

oder

k) die Alkylierung einer Verbindung der Formel

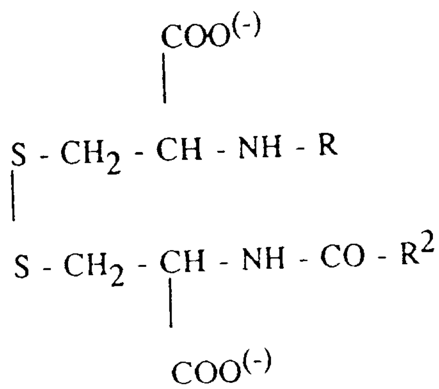

,

worin R und $R^2$ wie oben definiert sind, mit einer Verbindung der Formel

$R^{3E}$-Z,

worin $R^{3E}$ wie oben definiert ist, und Z Halogen, Alkylsulfat, Tosylat oder einen anderen Nucelofug darstellt, zur Bildung einer Verbindung der Formel

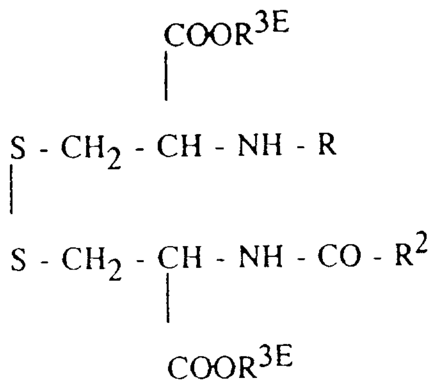

,

I) das Umsetzen, in Anwesenheit einer geeigneten Base, eines Carboxyl-geschützten Cystin-Derivats der Formel

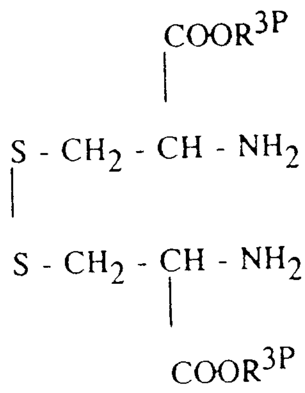

oder eines Hydrochloridsalzes hievon, worin $R^{3P}$ eine Säure- oder Basen-labile organische Gruppe bedeutet, mit einer Verbindung der Formel

$R^1$ - COX,

worin $R^1$ wie oben definiert ist, und COX eine reaktive Gruppe, die mit einer Amino-Gruppe unter Bildung einer Amid-Gruppe reagieren kann, darstellt, und danach Entfernung der Schutzgruppe $R^{3P}$, zur Bildung einer Verbindung der Formel

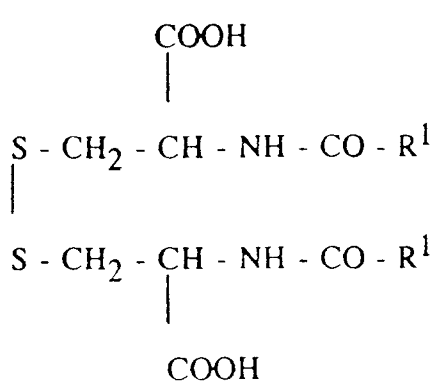

,

m) das Umsetzen, unter alkalischen Bedingungen, eines Carboxyl-geschützten Cystin-Derivats der Formel

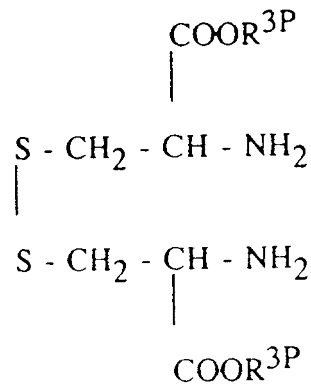

oder eines Hydrochloridsalzes hievon, worin $R^{3P}$ wie oben definiert ist, mit einer Verbindung der Formel

$R^2$ - COX,

worin $R^2$ und COX wie oben definiert sind, und danach Entfernung der Schutzgruppe $R^{3P}$, zur Bildung einer Verbindung der Formel

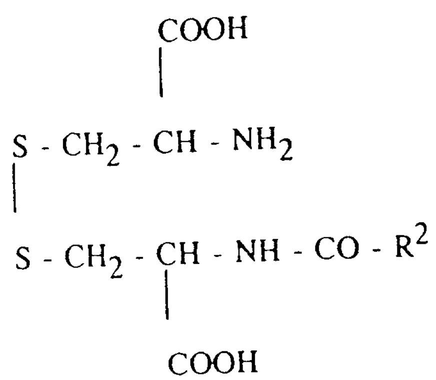

,

n) das Umsetzen eines Carboxyl-geschützten Cystin-Derivats der Formel

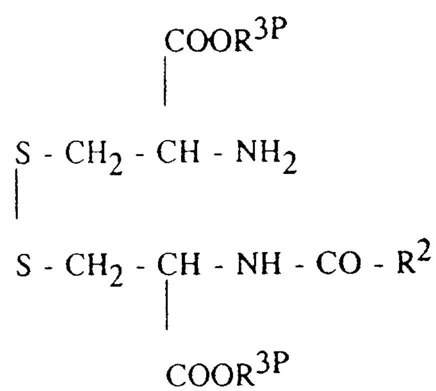

oder eines Hydrochloridsalzes hievon, worin $R^2$ und $R^{3P}$ wie oben definiert sind, mit einer Verbindung der Formel

$R^1$ - COX,

worin $R^1$ und COX wie oben definiert sind, und danach Entfernung der Schutzgruppe $R^{3P}$, zur Bildung einer Verbindung der Formel

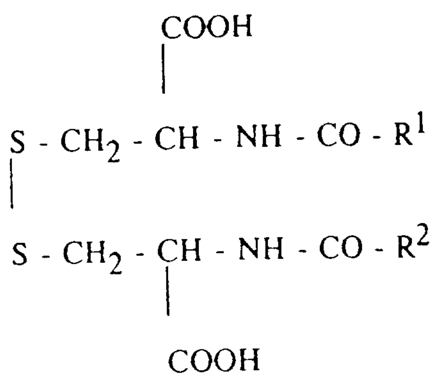

,

o) die Äquilibrierung, in alkalischer wässeriger Lösung, einer Mischung eines Cystin-Derivats und eines Cystein-Derivats der Formeln

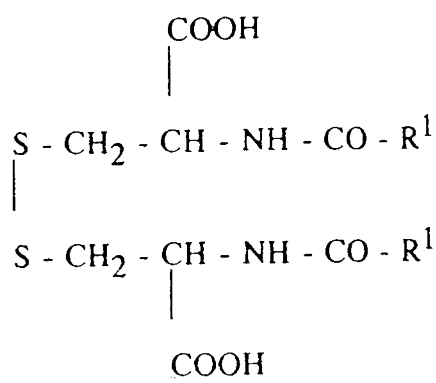

und

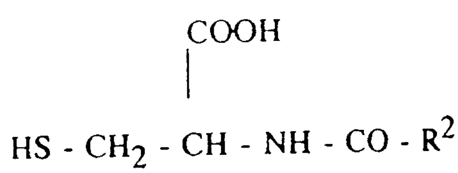

oder von Alkalisalzen hievon, worin $R^1$ und $R^2$ wie oben definiert sind, zur Bildung einer Verbindung der Formel

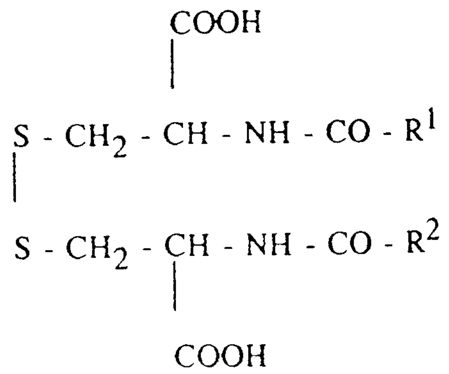

,

und schließlich, wenn gewünscht, Transfer der durch eine der Methoden a) bis o) erhaltenen Verbindungen in ein physiologisch annehmbares Salz.

2. Verfahren nach Anspruch 1, gekennzeichnet durch die Herstellung einer Verbindung der Formel (I), worin R Wasserstoff oder eine Gruppe -CO-$R^1$ bedeutet, wobei $R^1$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl darstellt; $R^2$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl ist; und $R^3$ Wasserstoff, Methyl oder Ethyl bedeutet; mit der Maßgabe, daß $R^1$ und $R^2$ nicht gleichzeitig Methyl darstellen, und ferner mit der Maßgabe, daß, wenn $R^3$ Wasserstoff ist, $R^1$ und $R^2$ nicht gleichzeitig n-Propyl, n-Heptyl, n-Nonyl oder n-Undecyl bedeuten, und ferner mit der Maßgabe, daß, wenn R und $R^3$ alle Wasserstoff darstellen, $R^2$ nicht n-Nonyl ist.

3. Verfahren nach Anspruch 1, gekennzeichnet durch die Herstellung einer Verbindung der Formel (I), worin $R^1$ und $R^2$ Isopropyl bedeuten, und $R^3$ Methyl darstellt.

4. Verfahren nach Anspruch 1, gekennzeichnet durch die Herstellung einer Verbindung der Formel (I), worin $R^1$ und $R^2$ n-Pentyl bedeuten, und $R^3$ Methyl darstellt.

5. Verfahren nach Anspruch 1, gekennzeichnet durch die Herstellung einer Verbindung der Formel (I), worin $R^1$ und $R^2$ n-Heptyl bedeuten, und $R^3$ Methyl oder Ethyl darstellt.

6. Verfahren nach Anspruch 1, gekennzeichnet durch die Herstellung einer Verbindung der Formel (I), worin $R^1$ und $R^2$ gleichzeitig Isopropyl bedeuten, und $R^3$ Wasserstoff ist.

7. Verfahren nach Anspruch 1, gekennzeichnet durch die Herstellung einer Verbindung der Formel (I), worin $R^1$ und $R^2$ gleichzeitig tert.Butyl bedeuten, und $R^3$ Wasserstoff ist.

8. Verwendung einer Verbindung der allgemeinen Formel

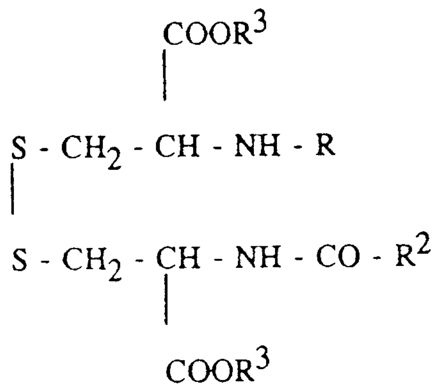

,

worin R Wasserstoff oder eine Gruppe -CO-$R^1$ bedeutet, wobei $R^1$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl darstellt; $R^2$ Methyl, Ethyl, n-Propyl, n-Butyl, n-Pentyl, n-Hexyl, n-Heptyl, n-Octyl, n-Nonyl, n-Decyl, n-Undecyl, Isopropyl, 1-Methylpropyl, tert.Butyl, 3-Methylbutyl oder 2-Methylbutyl ist; und $R^3$ Wasserstoff, Methyl, Ethyl, Propyl, Isopropyl, Butyl oder Isobutyl bedeutet; mit der Maßgabe, daß $R^1$ und $R^2$ nicht gleichzeitig Methyl darstellen, und ferner mit der Maßgabe, daß, wenn $R^3$ Wasserstoff ist, $R^1$ und $R^2$ nicht gleichzeitig n-Propyl, n-Heptyl, n-Nonyl oder n-Undecyl bedeuten, und ferner mit der Maßgabe, daß, wenn R und $R^3$ alle Wasserstoff darstellen, $R^2$ nicht n-Nonyl ist; oder eines physiologisch annehmbaren Salzes und/oder eines stereochemischen Isomers hievon, zur Herstellung von Medikamenten mit Immunmodulationswirkung.

## Revendications

**Revendications pour les Etats contractants suivants : AT, BE, CH, DE, DK, FR, GB, IT, LI, LU, NL, SE**

**1.** Composés de formule générale

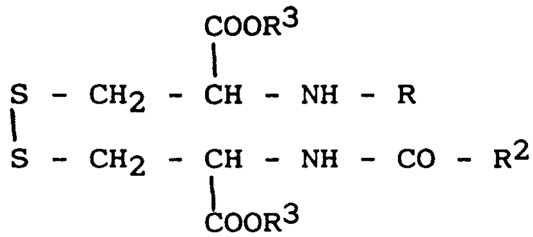

I

dans laquelle R est un hydrogène ou un reste -CO-$R^1$, dans lequel $R^1$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, $R^2$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, et $R^3$ est un hydrogène ou un radical méthyle, éthyle, propyle, isopropyle, butyle ou isobutyle, du moment que $R^1$ et $R^2$ ne sont pas simultanément des radicaux méthyle, et en outre du moment que, quand $R^3$ est un hydrogène, $R^1$ et $R^2$ ne sont pas simultanément des radicaux n-propyle, n-heptyle, n-nonyle ou n-undécyle, et en outre du moment que, quand R et $R^3$ sont tous les deux des hydrogènes, alors $R^2$ n'est pas le radical n-nonyle, ou un de ses sels acceptables d'un point de vue physiologique et/ou un de leurs isomères stéréochimiques.

**2.** Composé selon la revendication 1, dans lequel R est un hydrogène ou un reste -CO-$R^1$, dans lequel $R^1$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, $R^2$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, et $R^3$ est un hydrogène ou le radical méthyle ou éthyle, du moment que $R^1$ et $R^2$ ne sont pas simultanément des radicaux méthyle, et en outre du moment que, quand $R^3$ est un hydrogène, $R^1$ et $R^2$ ne sont pas simultanément des radicaux n-propyle, n-heptyle, n-nonyle ou n-undécyle, et en outre du moment que, quand R et $R^3$ sont tous les deux des hydrogènes, alors $R^2$ n'est pas le radical n-nonyle.

**3.** Composé selon la revendication 1, dans lequel $R^1$ et $R^2$ sont chacun le radical isopropyle et $R^3$ est le radical méthyle.

**4.** Composé selon la revendication 1, dans lequel $R^1$ et $R^2$ sont chacun le radical n-pentyle et $R^3$ est le radical méthyle.

**5.** Composé selon la revendication 1, dans lequel $R^1$ et $R^2$ sont chacun le radical n-heptyle et $R^3$ est le radical méthyle ou éthyle.

**6.** Composé selon la revendication 1, dans lequel $R^1$ et $R^2$ sont simultanément des radicaux isopropyle, et $R^3$ est un hydrogène.

**7.** Composé selon la revendication 1, dans lequel $R^1$ et $R^2$ sont simultanément des radicaux tert-butyle, et $R^3$ est un hydrogène.

**8.** Procédé pour préparer un composé de formule générale I selon la revendication 1, caractérisé en ce qu'il comprend :

a) l'oxydation d'un dérivé de N-acylcystéine de formule

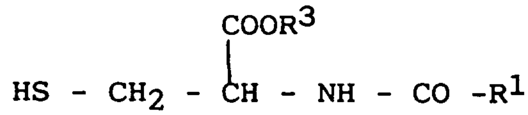

dans laquelle $R^1$ et $R^3$ sont tels que définis ci-dessus, ou encore, quand $R^3$ est un hydrogène, éventuellement de l'un de ses sels de métal alcalin, avec formation d'un composé de formule

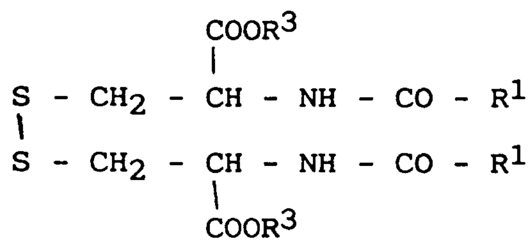

ou bien

b) la réaction, en présence d'une base appropriée, d'un composé ayant la formule suivante :

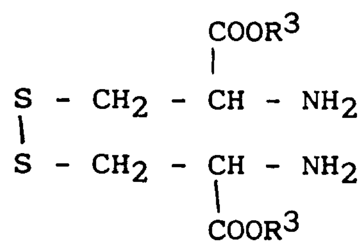

ou de l'un de ses sels, où $R^3$ est tel que défini ci-dessus, avec un composé de formule

$R^1$ -COX

où $R^1$ est tel que défini ci-dessus, et COX est un groupe réactif à même de réagir avec un groupe amino, avec formation d'une liaison amide, pour former un composé de formule

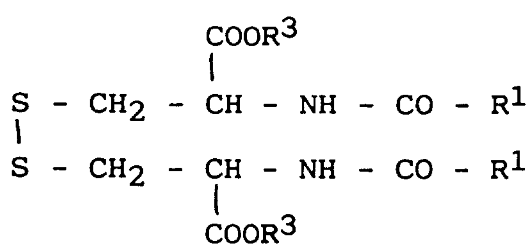

ou bien

c) la réaction d'un dérivé d'un acide 2-(N-acylamino)-3-halogénopropionique de formule

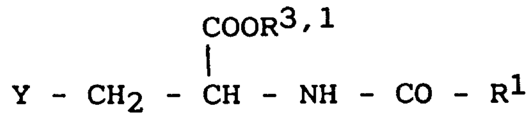

dans laquelle $R^1$ est tel que défini ci-dessus, $R^{3,1}$ est $R^3$ tel que défini ci-dessus ou un groupe organique labile acide ou basique, et Y est un atome d'halogéne, avec du soufre ou un dianion disulfure, en présence d'une base, pour former un composé de formule

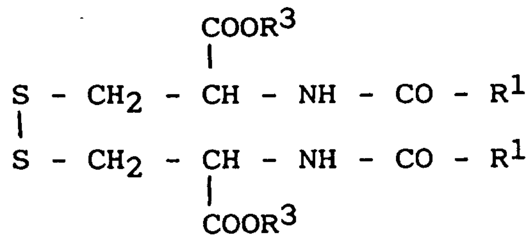

ou bien

d) l'oxydation d'un mélange de dérivés d'ester de N-acylcystéine ayant les formules suivantes :

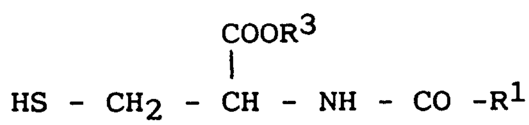

et

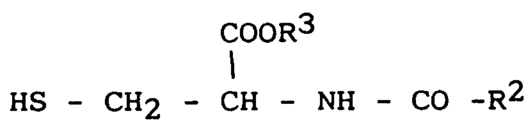

où $R^1$, $R^2$ et $R^3$ sont tels que définis ci-dessus, ou bien, quand $R^3$ est un hydrogène, éventuellement de leurs sels de métaux alcalins, pour former un composé de formule

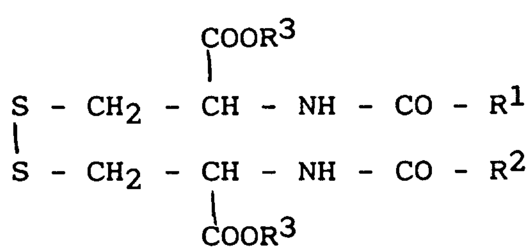

ou bien

e) l'oxydation d'un mélange de cystéine ou d'un ester de cystéine et d'un dérivé de N-acylcystéine, ayant les formules suivantes :

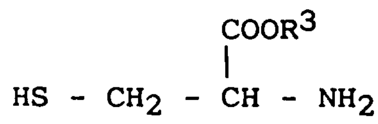

ou de l'un de leurs sels, et

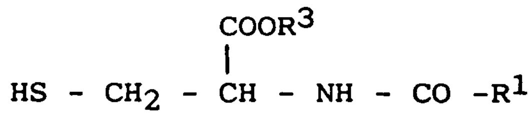

ou de l'un de ses sels avec un métal alcalin ou un de ses chlorhydrates,

où $R^1$ et $R^3$ sont tels que définis ci-dessus, pour former un composé ayant la formule suivante :

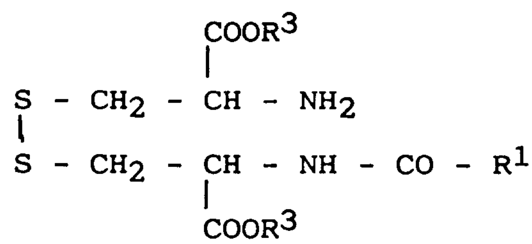

ou l'un de ses sels,

ou bien

f) la réaction, en présence d'une base appropriée, d'un excès de cystéine ou d'un diester de cystine ayant la formule suivante :

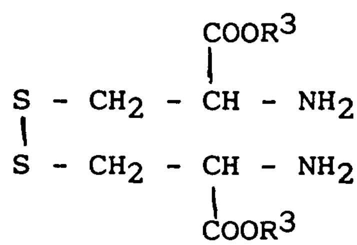

ou de l'un de ses sels, où $R^3$ est tel que défini ci-dessus, avec un composé de formule

$R^2$ - COX

où $R^2$ et COX sont tels que définis ci-dessus, pour former un composé de formule

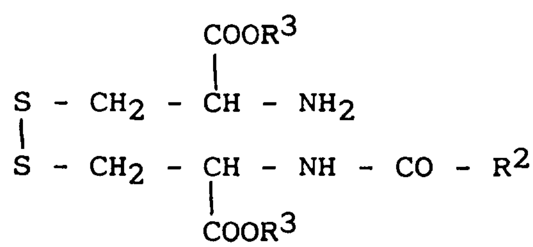

ou bien

g) la réaction, en présence d'une base appropriée, d'un composé de formule

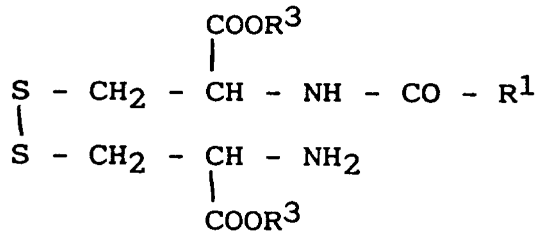

ou de l'un de ses sels, où $R^1$ et $R^3$ sont tels que définis ci-dessus, avec un composé de formule

$R^2$ - COX

où $R^2$ et COX sont tels que définis ci-dessus, pour former un composé de formule

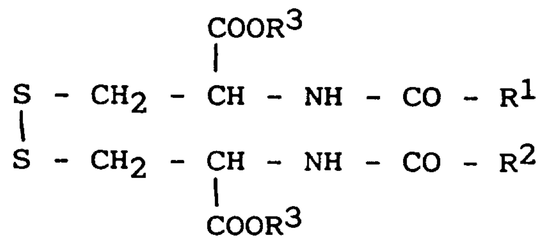

h) la réaction d'un dérivé de N-acylcystéine de formule

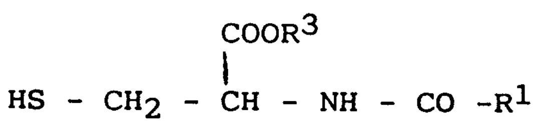

ou de l'un de ses sels,

où $R^1$ et $R^3$ sont tels que définis ci-dessus, avec un réactif d'activation pour former un produit d'addition de formule

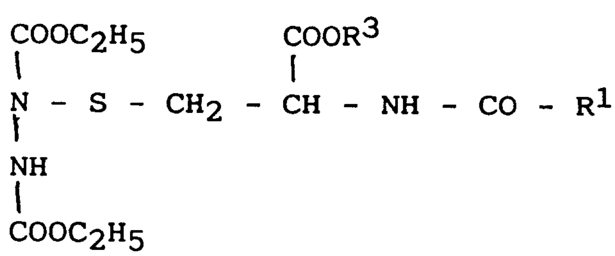

suivie de la réaction avec un deuxième dérivé de N-acylcystéine ou d'ester de cystéine, identique ou différent, pour donner un composé de formule

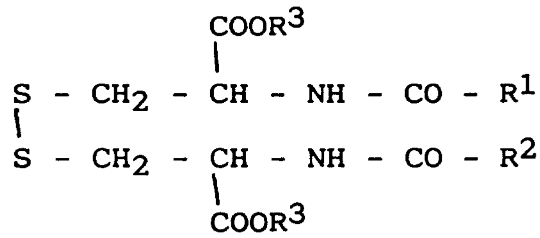

i) la réaction d'un composé de formule

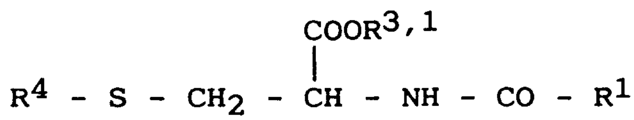

dans laquelle $R^4$ est Cl ou

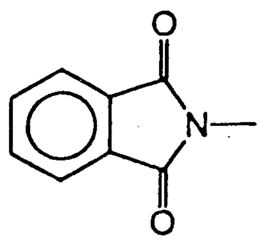

,

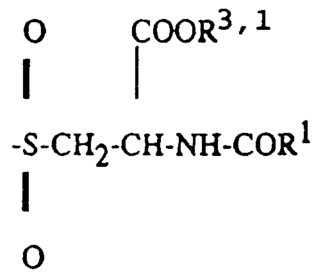

et $R^1$ et $R^{3,1}$ sont tels que définis ci-dessus, avec un composé de formule

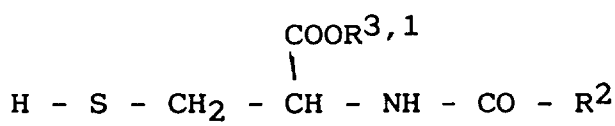

dans laquelle $R^2$ et $R^{3,1}$ sont tels que définis ci-dessus, pour former un composé de formule

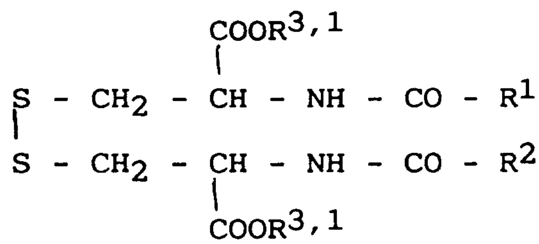

ce après quoi, si l'on souhaite un composé dans lequel $R^3$ = H, l'élimination du groupe protecteur $R^{3,1}$, ou bien

j) l'estérification d'un composé de formule

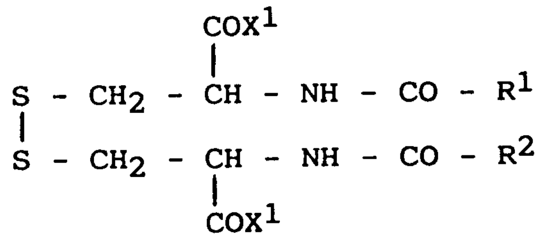

dans laquelle $R^1$ et $R^2$ sont tels que définis ci-dessus, et $X^1$ est OH ou un atome d'halogène, avec un composé de formule

$R^{3E}$-OH

où $R^{3E}$ est le radical méthyle, éthyle, propyle, isopropyle, butyle ou isobutyle, pour former un composé de formule

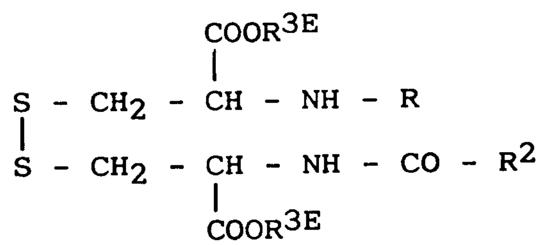

ou bien

k) l'alkylation d'un composé de formule

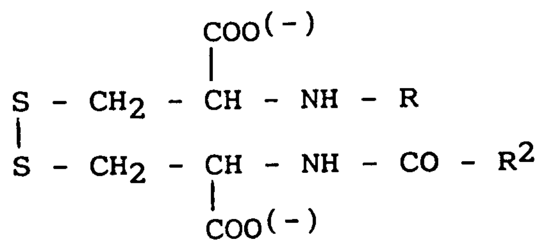

dans laquelle R et $R^2$ sont tels que définis ci-dessus, avec un composé de formule

$R^{3E}$ - Z

où $R^{3E}$ est tel que défini ci-dessus, et Z est un halogène, un alkylsulfate, un tosylate ou un autre nucléofuge, pour former un composé de formule

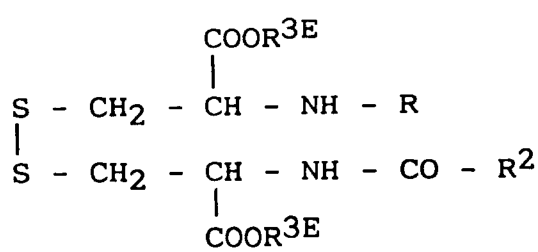

l) la réaction, en présence d'une base appropriée, d'un dérivé de cystine carboxyl-protégé de formule

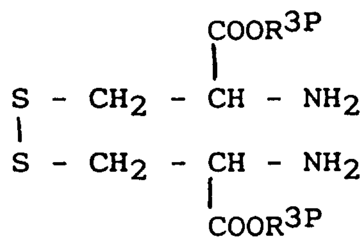

ou de l'un de ses chlorhydrates, où $R^{3P}$ est un groupe organique labile acide ou basique, avec un composé de formule

$R^1$ - COX

où $R^1$ est tel que défini ci-dessus et COX est un groupe réactif à même de réagir avec un groupe amino avec formation d'une liaison amide, puis l'élimination du groupe protecteur $R^{3P}$ pour former un composé de formule

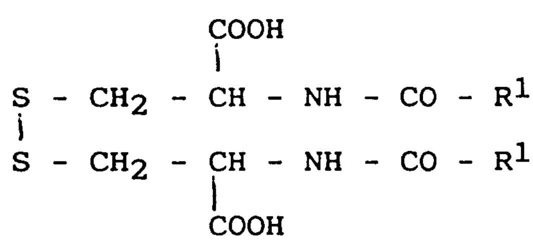

m) la réaction, dans des conditions alcalines, d'un dérivé de cystine carboxyl-protégé, ayant la formule suivante

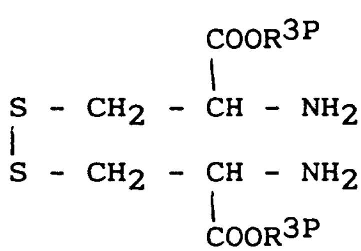

ou de l'un de ses chlorhydrates, où $R^{3P}$ est tel que défini ci-dessus, avec un composé de formule

$R^2$ - COX

où $R^2$ et COX sont tels que définis ci-dessus, puis l'élimination du groupe protecteur $R^{3P}$ pour former un composé de formule

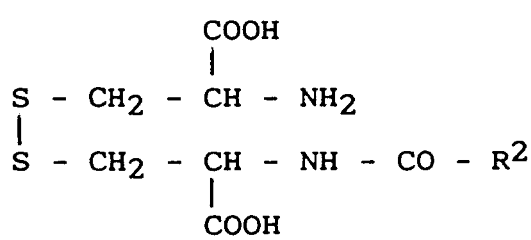

n) la réaction d'un dérivé de cystine carboxyl-protégé de formule

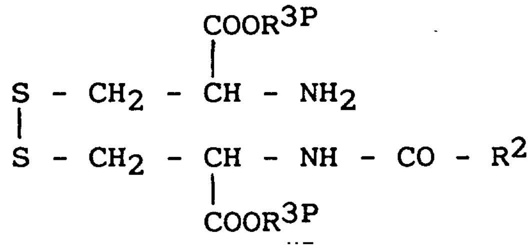

ou d'un de ses chlorhydrates, où $R^2$ et $R^{3P}$ sont tels que définis ci-dessus, avec un composé de formule

$R^1$ - COX

où $R^1$ et COX sont tels que définis ci-dessus, puis l'élimination du groupe protecteur $R^{3P}$ pour former un composé de formule

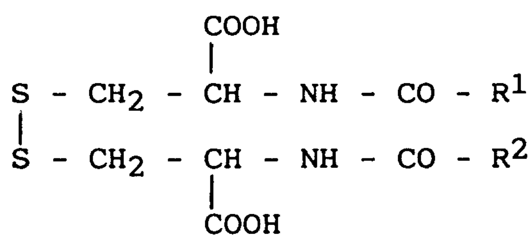

o) l'équilibrage, en solution aqueuse alcaline, d'un mélange d'un dérivé de cystine et d'un dérivé de cystéine, ayant les formules suivantes

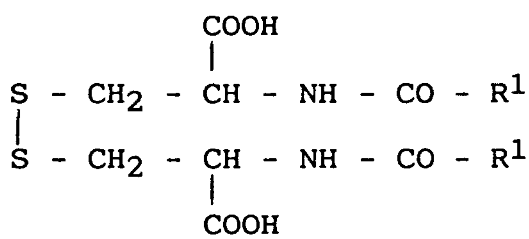

et

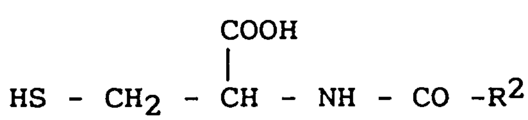

ou de leurs sels de métaux alcalins, où $R^1$ et $R^2$ sont tels que définis ci-dessus, pour former un composé de formule

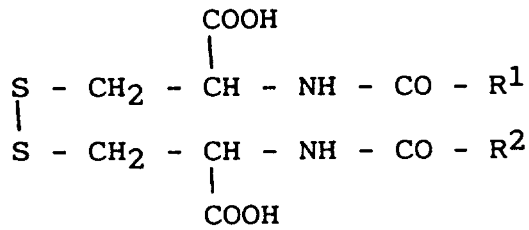

et finalement, si on le souhaite, le transfert, en un sel acceptable d'un point de vue physiologique, des composés obtenus par l'un quelconque des procédés a) à o).

**9.** Préparation pharmaceutique comprenant comme principe actif un composé selon l'une quelconque des revendications 1 à 7.

**10.** Préparation pharmaceutique selon la revendication 9, sous une forme posologique unitaire.

**11.** Préparation pharmaceutique selon les revendications 9-10, qui comprend le principe actif en association avec un excipient pharmaceutiquement acceptable.

**12.** Composé selon l'une quelconque des revendications 1 à 7, pour utilisation comme substance à activité thérapeutique.

**13.** Utilisation d'un composé selon l'une quelconque des revendications 1 à 7 pour préparer des médicaments présentant une action d'immunomodulation.

**14.** Composés selon les revendications 1 à 7, pour utilisation dans un procédé pour le traitement de maladies dues à des défauts du système immunitaire chez les mammifères, parmi lesquels l'homme.

**Revendications pour les Etats contractants suivants : ES, GR**

**1.** Procédé pour préparer un composé de formule générale

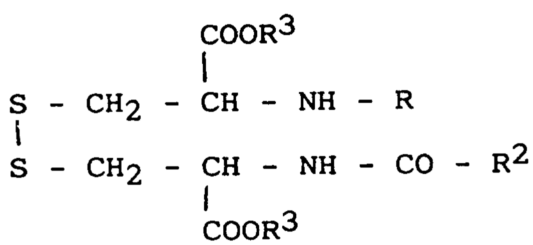

I

dans laquelle R est un hydrogène ou un reste -CO-$R^1$, dans lequel $R^1$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, $R^2$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, et $R^3$ est un hydrogène ou un radical méthyle, éthyle, propyle, isopropyle, butyle ou isobutyle, du moment que $R^1$ et $R^2$ ne sont pas simultanément des radicaux méthyle, et en outre du moment que, quand $R^3$ est un hydrogène, $R^1$ et $R^2$ ne sont pas simultanément des radicaux n-propyle, n-heptyle, n-nonyle ou n-undécyle, et en outre du moment que, quand R et $R^3$ sont tous les deux des hydrogènes, alors $R^2$ n'est pas le radical n-nonyle, ou un de ses sels acceptables d'un point de vue physiologique et/ou un de ses isomères stéréochimiques, caractérisé en ce qu'il comprend

a) l'oxydation d'un dérivé de N-acylcystéine de formule

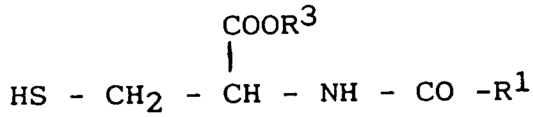

dans laquelle $R^1$ et $R^3$ sont tels que définis ci-dessus, ou encore, quand $R^3$ est un hydrogène, éventuellement de l'un de ses sels de métal alcalin, avec formation d'un composé de formule

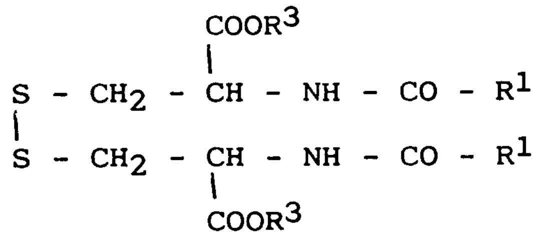

ou bien

b) la réaction, en présence d'une base appropriée, d'un composé ayant la formule suivante :

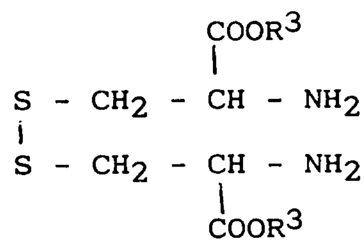

ou de l'un de ses sels, où $R^3$ est tel que défini ci-dessus, avec un composé de formule

$R^1$ -COX

où $R^1$ est tel que défini ci-dessus, et COX est un groupe réactif à même de réagir avec un groupe amino, avec formation d'une liaison amide, pour former un composé de formule

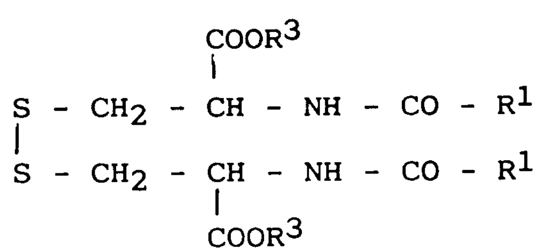

ou bien

c) la réaction d'un dérivé d'un acide 2-(N-acylamino)-3-halogénopropionique de formule

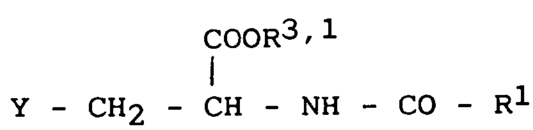

dans laquelle $R^1$ est tel que défini ci-dessus, $R^{3,1}$ est $R^3$ tel que défini ci-dessus ou un groupe organique labile acide ou basique, et Y est un atome d'halogène, avec du soufre ou un dianion disulfure, en présence d'une base, pour former un composé de formule

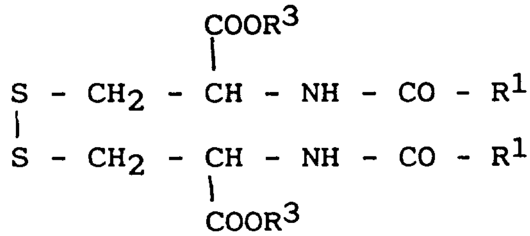

ou bien

d) l'oxydation d'un mélange de dérivés d'ester de N-acylcystéine ayant les formules suivantes :

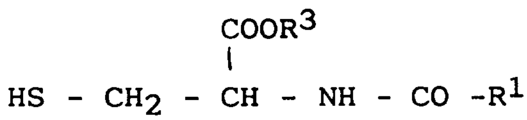

et

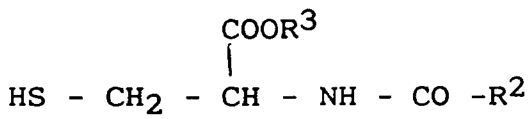

où $R^1$, $R^2$ et $R^3$ sont tels que définis ci-dessus, ou bien, quand $R^3$ est un hydrogène, éventuellement de leurs sels de métaux alcalins, pour former un composé de formule

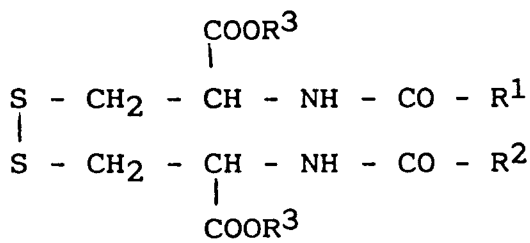

ou bien

e) l'oxydation d'un mélange de cystéine ou d'un ester de cystéine et d'un dérivé de N-acylcystéine, ayant les formules suivantes :

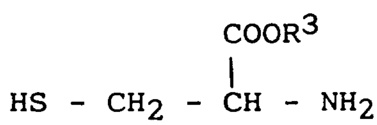

ou l'un de leurs sels, et

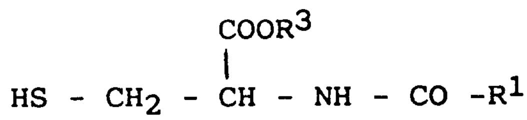

ou de l'un de ses sels avec un métal alcalin ou un de ses chlorhydrates,

où $R^1$ et $R^3$ sont tels que définis ci-dessus, pour former un composé ayant la formule suivante :

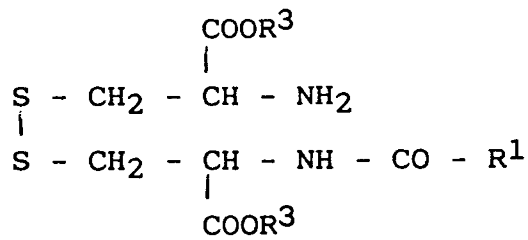

ou de l'un de ses sels,

ou bien

f) la réaction, en présence d'une base appropriée, d'un excès de cystéine ou d'un diester de cystine ayant la formule suivante :

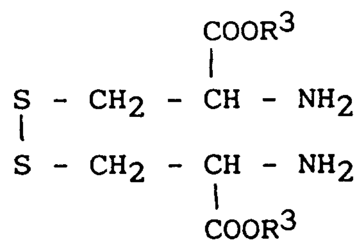

ou de l'un de ses sels, où $R^3$ est tel que défini ci-dessus, avec un composé de formule

$R^2$ - COX

où $R^2$ et COX sont tels que définis ci-dessus, pour former un composé de formule

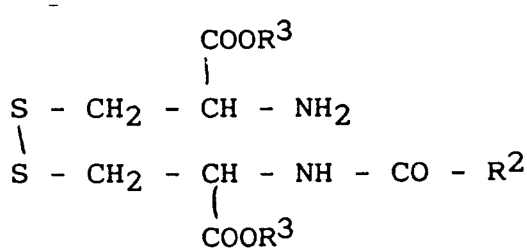

ou bien

g) la réaction, en présence d'une base appropriée, d'un composé de formule

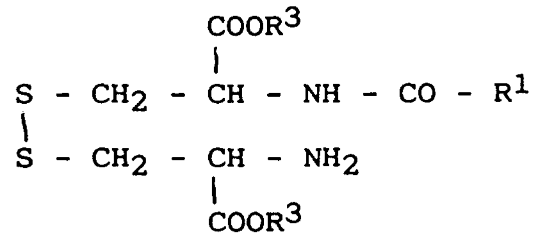

ou de l'un de ses sels, où $R^1$ et $R^3$ sont tels que définis ci-dessus, avec un composé de formule

$R^2$ - COX

où $R^2$ et COX sont tels que définis ci-dessus, pour former un composé de formule

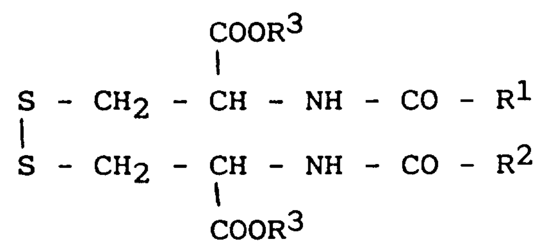

h) la réaction d'un dérivé de N-acylcystéine de formule

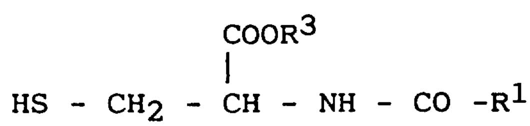

ou de l'un de ses sels,

où $R^1$ et $R^3$ sont tels que définis ci-dessus, avec un réactif d'activation pour former un produit d'addition de formule

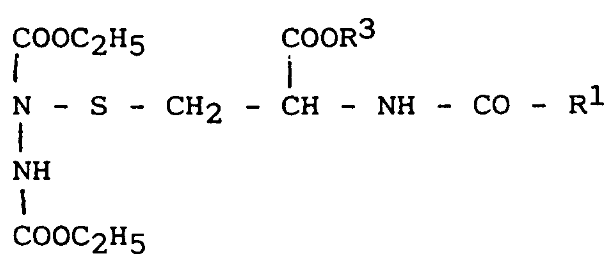

suivie de la réaction avec un deuxième dérivé de N-acylcystéine ou d'ester de cystéine, identique ou différent, pour donner un composé de formule

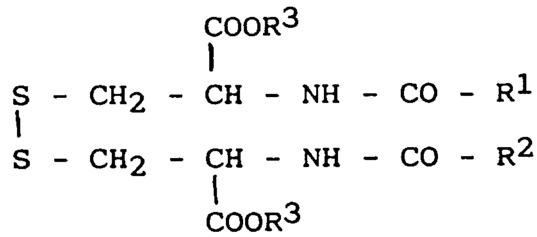

i) la réaction d'un composé de formule

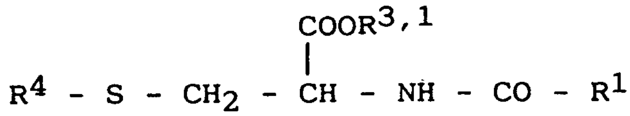

dans laquelle $R^4$ est Cl ou

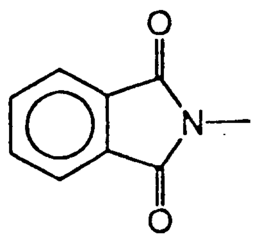
,
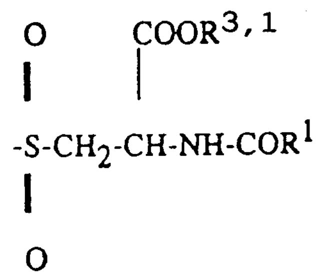

et $R^1$ et $R^{3,1}$ sont tels que définis ci-dessus, avec un composé de formule

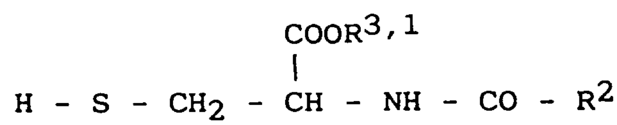

dans laquelle $R^2$ et $R^{3,1}$ sont tels que définis ci-dessus, pour former un composé de formule

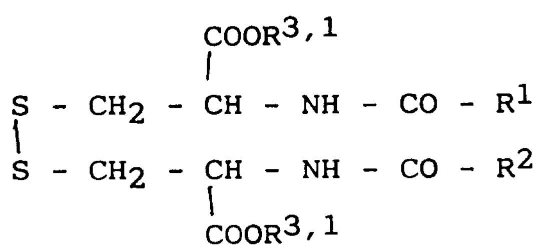

ce après quoi, si l'on souhaite un composé dans lequel $R^3$ = H, l'élimination du groupe protecteur $R^{3,1}$, ou bien

j) l'estérification d'un composé de formule

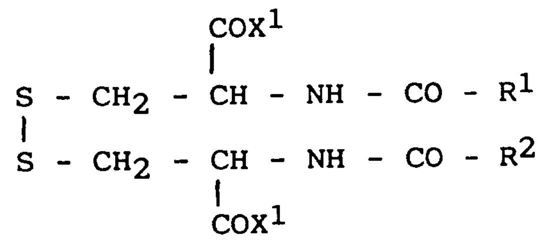

dans laquelle $R^1$ et $R^2$ sont tels que définis ci-dessus, et $X^1$ est OH ou un atome d'halogène, avec un composé de formule

$R^{3E}$-OH

où $R^{3E}$ est le radical méthyle, éthyle, propyle, isopropyle, butyle ou isobutyle, pour former un composé de formule

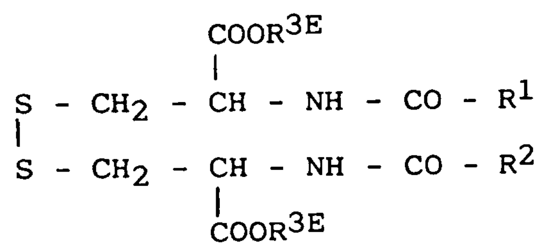

ou bien

k) l'alkylation d'un composé de formule

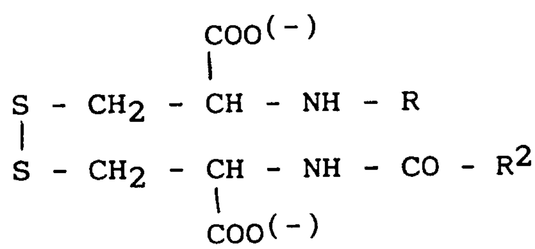

dans laquelle R et $R^2$ sont tels que définis ci-dessus, avec un composé de formule

$R^{3E}$ - Z

où $R^{3E}$ est tel que défini ci-dessus, et Z est un halogène, un alkylsulfate, un tosylate ou un autre nucléofuge, pour former un composé de formule

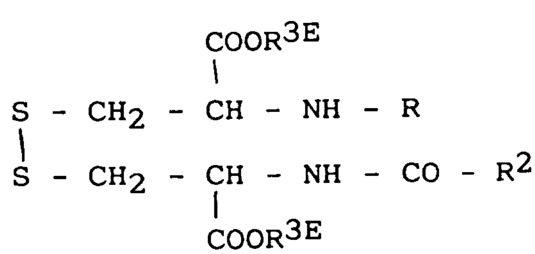

l) la réaction, en présence d'une base appropriée, d'un dérivé de cystine carboxyl-protégé de formule

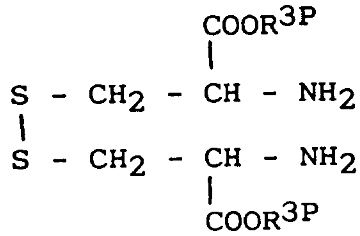

ou de l'un de ses chlorhydrates, où $R^{3P}$ est un groupe organique labile acide ou basique, avec un composé de formule

$R^1$ - COX

où $R^1$ est tel que défini ci-dessus et COX est un groupe réactif à même de réagir avec un groupe amino avec formation d'une liaison amide, puis l'élimination du groupe protecteur $R^{3P}$ pour former un composé de formule

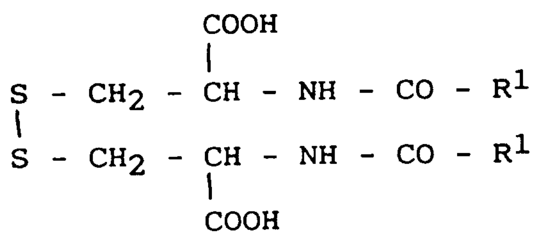

m) la réaction, dans des conditions alcalines, d'un dérivé de cystine carboxyl-protégé, ayant la formule suivante

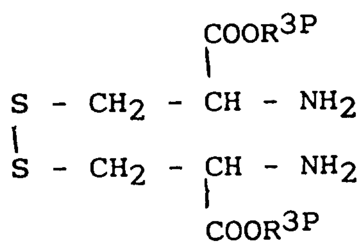

ou de l'un de ses chlorhydrates, où $R^{3P}$ est tel que défini ci-dessus, avec un composé de formule

$R^2$ - COX

où $R^2$ et COX sont tels que définis ci-dessus, puis l'élimination du groupe protecteur $R^{3P}$ pour former un composé de formule

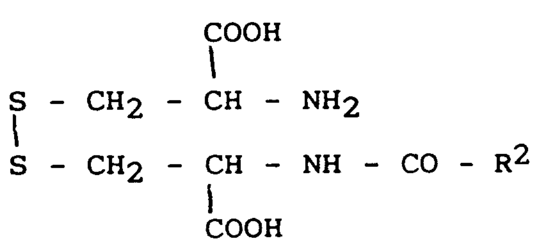

n) la réaction d'un dérivé de cystine carboxyl-protégé de formule

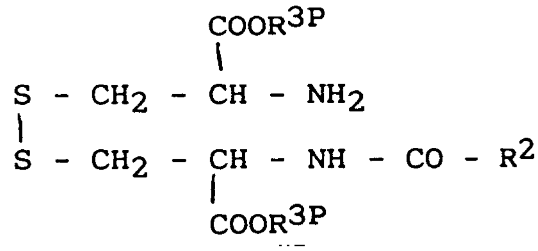

ou d'un de ses chlorhydrates, où $R^2$ et $R^{3P}$ sont tels que définis ci-dessus, avec un composé de formule

$R^1$ - COX

où $R^1$ et COX sont tels que définis ci-dessus, puis l'élimination du groupe protecteur $R^{3P}$ pour former un composé de formule

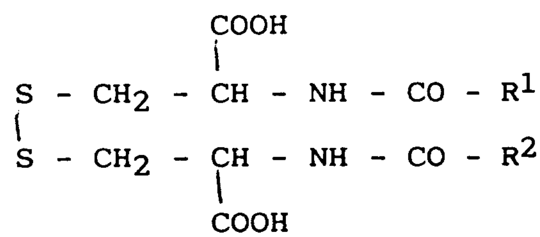

o) l'équilibrage, en solution aqueuse alcaline, d'un mélange d'un dérivé de cystine et d'un dérivé de cystéine, ayant les formules suivantes

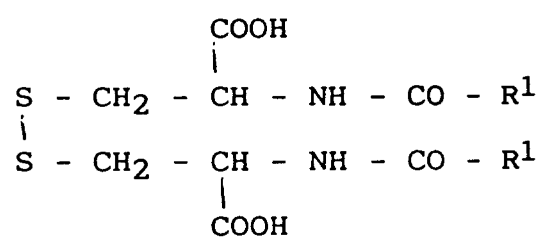

et

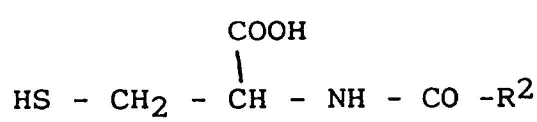

ou de leurs sels de métaux alcalins, où $R^1$ et $R^2$ sont tels que définis ci-dessus, pour former un composé de formule

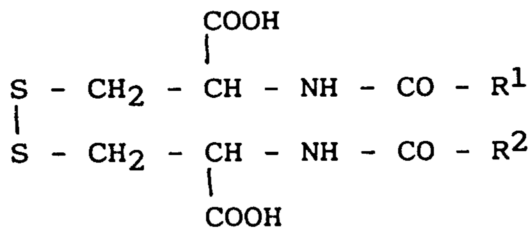

et finalement, si on le souhaite, le transfert, en un sel acceptable d'un point de vue physiologique, des composés obtenus par l'un quelconque des procédés a) à o).

**2.** Procédé selon la revendication 1, caractérisé en ce qu'il consiste à préparer un composé de formule I, dans lequel R est un hydrogène ou un reste -CO-$R^1$, dans lequel $R^1$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, $R^2$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, et $R^3$ est un hydrogène ou le radical méthyle ou éthyle, du moment que $R^1$ et $R^2$ ne sont pas simultanément des radicaux méthyle, et en outre du moment que, quand $R^3$ est un hydrogène, $R^1$ et $R^2$ ne sont pas simultanément des radicaux n-propyle, n-heptyle, n-nonyle ou n-undécyle, et en outre du moment que, quand R et $R^3$ sont tous les deux des hydrogènes, alors $R^2$ n'est pas le radical n-nonyle.

**3.** Procédé selon la revendication 1, caractérisé en ce qu'il consiste à préparer un composé de formule I dans laquelle $R^1$ et $R^2$ sont chacun le radical isopropyle et $R^3$ est le radical méthyle.

**4.** Procédé selon la revendication 1, caractérisé en ce qu'il consiste à préparer un composé de formule I dans laquelle $R^1$ et $R^2$ sont chacun le radical n-pentyle et $R^3$ est le radical méthyle.

**5.** Procédé selon la revendication 1, caractérisé en ce qu'il consiste à préparer un composé de formule I dans laquelle $R^1$ et $R^2$ sont chacun le radical n-heptyle et $R^3$ est le radical méthyle ou éthyle.

**6.** Procédé selon la revendication 1, caractérisé en ce qu'il consiste à préparer un composé de formule I dans laquelle $R^1$ et $R^2$ sont simultanément des radicaux isopropyle, et $R^3$ est un hydrogène.

**7.** Procédé selon la revendication 1, caractérisé en ce qu'il consiste à préparer un composé de formule I dans laquelle $R^1$ et $R^2$ sont simultanément des radicaux ter-butyle, et $R^3$ est un hydrogène.

**8.** Utilisation d'un composé de formule générale

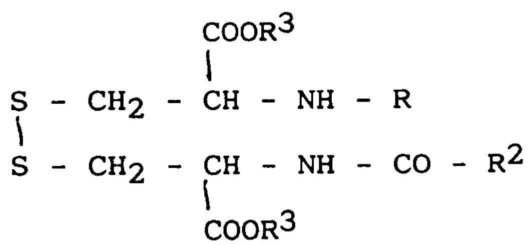

I

dans laquelle R est un hydrogène ou un reste -CO-$R^1$, dans lequel $R^1$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, $R^2$ est un radical méthyle, éthyle, n-propyle, n-butyle, n-pentyle, n-hexyle, n-heptyle, n-octyle, n-nonyle, n-décyle, n-undécyle, isopropyle, 1-méthylpropyle, tert-butyle, 3-méthylbutyle ou 2-méthylbutyle, et $R^3$ est un hydrogène ou un radical méthyle, éthyle, propyle, isopropyle, butyle ou isobutyle, du moment que $R^1$ et $R^2$ ne sont pas simultanément des radicaux méthyle, et en outre du moment que, quand $R^3$ est un hydrogène, $R^1$ et $R^2$ ne sont pas simultanément des radicaux n-propyle, n-heptyle, n-nonyle ou n-undécyle, et en outre du moment que, quand R et $R^3$ sont tous les deux des hydrogènes, alors $R^2$ n'est pas le radical n-nonyle,

ou d'un de ses sels acceptables d'un point de vue physiologique et/ou d'un de ses isomères stéréochimiques, pour préparer des médicaments ayant une action d'immunomodulation.